# EUROPEAN PATENT SPECIFICATION

(11) **EP 0 988 304 B1**
(45) Date of publication and mention of the grant of the patent: **02.05.2001**
(21) Application number: 98939041.4
(22) Date of filing: 14.08.1998
(51) Int. Cl.: C07D 473/00, C07D 473/18, C07D 473/32, C07H 19/16, C07H 19/167, C07H 19/173, A61K 31/52, A61K 31/70

(54) **NUCLEOSIDES ANALOGUES, SUCH AS ANTIVIRALS INCLUDING INHIBITORS OF RETROVIRAL REVERSE TRANSCRIPTASE AND THE DNA POLYMERASE OF HEPATITIS B VIRUS (HBV)**
ANALOGE NUKLEOSIDE, WIE ANTIVIRALE EINSCHLIESSLICH INHIBITOREN DER RETROVIRALEN REVERSTRANSKRIPTASE UND DER DNA POLYMERASE DES HEPATITIS B VIRUS
ANALOGUES DE NUCLEOSIDES TELS QUE DES ANTIVIRAUX Y COMPRIS DES INHIBITEURS DE TRANSCRIPTASE INVERSE RETROVIRALE ET L'ADN POLYMERASE DU VIRUS DE L'HEPATITE B(HBV)

(30) Priority: 15.08.1997 SE 9702957; 12.11.1997 SE 9704147; 13.02.1998 SE 9800452
(43) Date of publication of application: 29.03.2000
(60) Divisional application: 01103370.1
(73) Proprietor: MEDIVIR AB, S-141 44 Huddinge (SE)
(72) Inventor: ZHOU, Xiao-Xiong, S-141 41 Huddinge (SE); JOHANSSON, Nils-Gunnar, S-150 23 Enhörna (SE); WÄHLING, Horst, S-127 61 Skärholmen (SE)
(74) Representative: Dehmel, Albrecht, Dr.
(86) International application number: SE9801467
(87) International publication number: WO9909031

(56) References cited:
- EP-A2- 0 375 329
- EP-A2- 0 694 547
- WO-A1-89/03837
- WO-A1-92/13561
- WO-A1-94/24134
- WO-A1-97/27194
- WO-A1-97/30051
- WO-A1-98/21223
- US-A- 4 957 924

## Description

### Technical Field

This invention relates to the field of nucleoside analogues, such as antivirals including inhibitors of retroviral reverse transcriptase and the DNA polymerase of Hepatitis B Virus (HBV). The invention provides novel compounds with favourable pharmaceutical parameters, methods for their preparation, pharmaceutical compositions comprising these compounds and methods employing then for the inhibition of viral and neoplastic diseases including HBV and HIV.

### Background to the invention

International patent application no.WO 88/00050 describes the antiretroviral and anti-HBV activity of a series of 3'-fluorinated nucleosides, including the compounds 2',3'-dideoxy, 3'-fluoroguanosine (FLG) and 3'-deoxy-3'-fluorothymidine (FLT). The latter compound underwent clinical evaluation as an anti-HIV agent and although its antiviral activity and pharmacokinetics were good, it showed unexpected toxicity (Flexner et al, J Inf Dis 170(6) 1394-403 (1994)). The former compound FLG is very active in vitro however the present inventors have detected that its bioavailability is so poor - around 4% - that the in vivo utility of the compound has thus far been limited to intraperitoneally or subcutaneously administered animal models.

US patent 4,963,662 discloses generically a series of 3'-fluorinated nucleosides and corresponding triphosphates and specifically describes the preparation of the 5'-O-palmitoyl derivative of FLT, without reporting any improvement in bioavailability. International patent application WO 93/13778 describes FLG derivatives modified at the 6-position of the base, in particular with n-propoxy, cyclobutoxy, cyclopropanylamino, piperidino or pyrrolidino. International patent application no. 93 14103 describes FLG derivatives where the oxygen at the guanine 6-position is replaced with amino, ether, halo or sulphonate.

EP-A 0 694 547, published in early 1996, discloses the diastereomers of the L-monovaline ester of 2-(2-amino-1,6-dihydro-6-oxo-purin-9-yl)-methoxy-1,3-propanediol (and of its pharmaceutically acceptable salts). The compounds are described to exhibit an antiviral activity.

WO 97/27194 describes a two-step process for preparing these compounds, i.e., the purine derivatives which are the L-monovaline ester of 2-(2-amino-1,6-dihydro-6-oxo-purin-9-yl)-methoxy-1,3-propanediol and its pharmaceutically acceptable salts.

US 4,957,924 finally reveals specific amino acid esters of acyclovir and pharmaceutically acceptable salts thereof. The compounds are described to be useful in the treatment of herpes virus infections.

### Brief Description of the Invention

In order to comprehend the present invention it appears to be useful to introduce the skilled reader into the field of prodrugs of nucleoside analogues. Generally, such compounds may have the formula I: 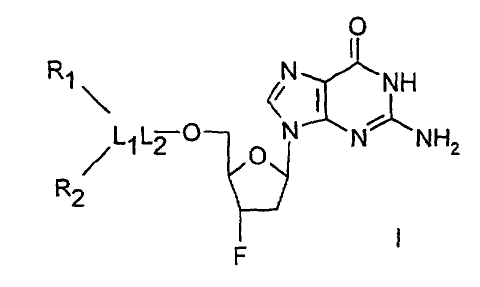 wherein:
R₁ is selected from
   hydroxy, amino or carboxy; optionally having esterified/amide bonded thereon a C₄-C₂₂ saturated or unsaturated, optionally substituted fatty acid or alcohol, or an aliphatic L-amino acid;
R₂ is the residue of an aliphatic L-amino acid;
L₁ is a trifunctional linker group;
L₂ is absent or a difunctional linker group;
including pharmaceutically acceptable salts thereof.

Pharmaceutical compositions comprising the compounds and salts of formula I and pharmaceutically acceptable carriers or diluents therefor may be easily prepared. Thus, methods are provided for the inhibition of HBV and retroviruses such as HIV, comprising bringing a compound or salt of the formula I into contact with a retrovirus or HBV, for example by administering an effective amount of the compound or salt to an individual afflicted with a retrovirus or HBV. The compounds or salts of formula I may thus be useful in therapy, for example in the preparation of a medicament for the treatment of retroviral or HBV infections.

In treating conditions caused by retroviruses such as HIV, or HBV, the compounds or salts of formula I are preferably administered in an amount of 50 to 1 500 mg once, twice or three times per day, especially 100 to 700 mg twice or thrice daily. It is desirable to achieve serum levels of the active metabolite of 0.01 to 100 µg/ml, especially 0.1 to 5 µg/ml.

Where R₁ is a fatty acid residue, it preferably has in total an even number of carbon atoms, advantageously decanoyl (C₁₀), lauroyl (C₁₂), myristoyl (C₁₄), palmitoyl (C₁₆), stearoyl (C₁₈), eicosanoyl (C₂₀) or behenoyl (C₂₂). The fatty acid preferably has in total 10 to 22, and more preferably 16 to 20 carbon atoms, especially 18. The fatty acid may be unsaturated and have one to three double bonds, especially one double bond. Unsaturated fatty acids preferably belong to the n-3 or n-6 series. Convenient unsaturated R₁ groups include those derived from the monounsaturated acids myristoleic, myristelaidic, palmitoleic, palmitelaidic, n6-octadecenoic, oleic, elaidic, gandoic, erucic, brassidic acids or multiply unsaturated fatty acids such as linoleic, γ-linolenic, arachidonic acid and α-linolenic acid. Preferably, however, R1 as a fatty acid is saturated as these compounds tend to have superior stability and shelf life.

R₁ as fatty alcohol residue preferably corresponds to one of the above described fatty acids. Alternatively the fatty alcohol may comprise residues of shorter alcohols, such as methanol, ethanol or propanol.

R₁ as a saturated or unsaturated fatty acid or alcohol may optionally be substituted with up to five similar or different substituents independently selected from the group consisting of hydroxy, C₁-C₆ alkyl, C₁-C₆ alkoxy, C₁-C₆ alkoxy C₁-C₆ alkyl, C₁-C₆ alkanoyl, amino, halo, cyano, azido, oxo, mercapto and nitro, and the like.

Suitable aliphatic amino acids for R₂ and, if present R₁, include L-alanine, L-leucine, L-isoleucine and most preferably L-valine. For ease of synthesis it is preferred that both R₂ and R₁ are residues of aliphatic amino acids, preferably the same residue.

The expression trifunctional in the context of the first linker group L₁ means that the linker has at least three functional groups, including at least two functional groups derived from respective hydroxy, amine or carboxyl groups, the amine and hydroxy function(s) being available for esterification/amide bonding with the carboxy functions of R₁ and R₂ whereas a carboxy function(s) on the linker is available for amide bonding with the free α-amine function of R₂ , or R₁ as the case may be, or esterification with R₁ as a fatty alcohol. Where R₁ itself defines an hydroxy, amine or carboxy group, the hydroxy group being presently favoured of the three, one of said functions on the trifunctional linker simply comprises this hydroxy, amine or carboxy group.

The trifunctional linker farther comprises a third functional group for linkage with either the optional second linker group L₂ illustrated in more detail below, or the hydroxy group at the 5' position of the mother nucleoside, such as 2',3'-dideoxy-3'-fluoroguanosine. Appropriate third functional groups will depend on the nature of the cooperating function on optional linker group L₂, if present, and may include amino, hydoxy, carbonyl, sulfonyl, phosphoryl, phosphonyl, carbamoyl and the like. If L₂ is absent, this third functional group on first linker L₁ will typically comprise a carboxyl function which can esterify with the 5'-O group of the nucleoside analogue.

Preferably the functional groups on the trifunctional linker which cooperate with R₁ and R₂ are hydroxyl functions and the linkage is an ester linkage with the carboxyl functions of an R₁ fatty acid, if present, and R₂. A further preferred embodiment comprises a free hydroxy group as R₁ and an hydroxyl function on the linker esterified to the carboxy function of R₂. An alternative embodiment comprises an (optionally protected) carboxyl group as R₁ and an hydroxyl function on the linker esterifed to a carboxy function on R₂.

Useful trifunctional L₁ group, especially for esterifying directly to the nucleoside include linkers of the formula IIa or IIb: 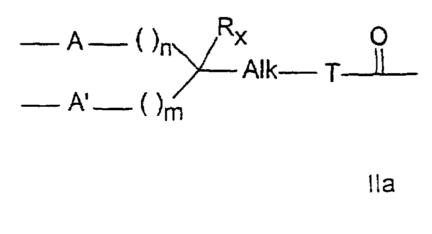 where A and A' define a respective ester linkage between an hydroxy on the linker and the carboxy on R₁ or R₂ or an ester linkage between a carboxy on the linker and the hydroxy on R₁ as a fatty alcohol, or an amide linkage between an amine on the linker and a carboxy on R₁ or R₂, or an amide linkage between a carboxy on the linker and an amine on R₁ or R₂, or one of A and A' is as defined and the other is hydroxy, amino or carboxy in the event that R₁ itself is a free hydroxy, amino or carboxy group.
Rx is H or C₁-C₃ alkyl,
T is a bond, -O- or -NH-;
Alk is absent, C₁-C₄ alkyl or C₂ - C₄ alkenyl, optionally substituted as
described above; and
m and n are independently 0, 1 or 2.

It is preferred that the R₁ or R₂ groups are each esterified to a respective one of the leftmost functional hydroxy groups (viz A and A') of Formula IIa, while the carbonyl moiety to the right is esterified, optionally via a second linker group L₂, to the 5'-O-group of the nucleoside.

Alternatively the L₁ group may comprise a linker of the formula IIb: 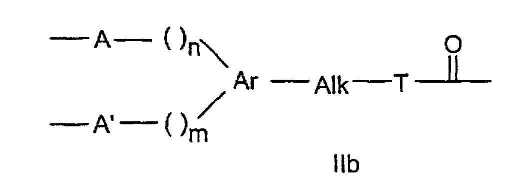 where Ar is a saturated or unsaturated, preferably monocyclic carbo- or heterocycle with 5 or 6 ring atoms; and
A, A', T, Alk, m and n are as defined above.

In Formula IIb, Ar is preferably an aromatic group such as pyridine or especially phenyl, such as aromatic moieties wherein the arms bearing the R₁ and R₂ groups are respectively para and ortho, meta and ortho, both ortho, or preferably para and meta, both para or both meta to the remainder of the linker.

In formulae IIa and IIb, the following combinations of m, n and Alk are presently favoured:

| | | |
|---|---|---|
| m | n | Alk |
| 1 | 0 | methylene |
| 1 | 0 | ethylene |
| 1 | 1 | absent |
| 1 | 1 | methylene |
| 1 | I | ethylene |
| 1 | 1 | propylene |
| 1 | 2 | absent |
| 1 | 2 | methylene |
| 1 | 1 | ethenylene |
| 1 | 1 | propenylene |

As R₁ and R₂ may have different structures, it will be apparent that many L₁ groups, particularly those of formula IIa, will define chiral structures.

A particularly preferred group of trifunctional linkers comprise glycerol derivatives of the formula IIc 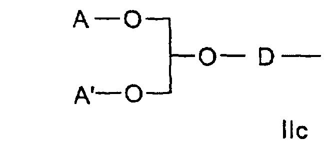 where A is hydrogen, the acyl residue of an aliphatic L-amino acid ester or the acyl residue of a fatty acid ester, A' is the acyl residue of an aliphatic amino acid residue and D is a C₂-C₆ saturated or unsaturated dicarboxylic acid residue. Trifunctional linkers of the formula IIc are hydrolysed or otherwise break down in vivo to release the nature identical compounds glycerol, the L-amino acid, the fatty acid (if present) and the dicarboxylic acid, each of which are generally safely metabolised and/or excreted by the body. Preferably A and A' are both residues of an aliphatic amino acid, most preferably the same residue, particularly residues of L-valine or L-isoleucine.

In the event that the dicarboxylic acid moiety in the derivative of formula IIc is esterified directly to the 5' hydroxy function (or equivalent) on the nucleoside, an alternative analysis would be to define the glycerol moiety as trifunctional linker L₁ and the dicarboxylic acid moiety as difunctional linker L₂.

Particularly preferred dicarboxylic acid residues include those derived from oxalic, malonic, tartronic, succinic, maleic, fumaric, malic, tartaric, glutaric, glutaconic, citraconic, itaconic, ethidine-malonic, mesaconic, adipic, allylmalonic, propylidenemalonic, hydromuconic, pyrocinchonic and muconic acids and the like. The dicarboxylic acid residue may be optionally substituted, for example with the substituents listed above in respect of R₁ as a fatty acid. Hydroxy substituents can in turn be esterified with a further L-amino acid or fatty acid residue.

Several of the abovementioned dicarboxylic acids can themselves define a trifunctional linker. For instance hydroxy-substituted dicarboxylic acids such as tartaric acid or malic acid offer a number of configurations. Taking tartaric acid as an example a carboxyl function is available for esterification with the 5'-hydroxyl function of a nucleoside (optionally via difunctional linker L₂). The hydroxy functions are available for esterification with the respective carboxyl functions of R₂ and an R₁ fatty acid or amino acid while the remaining carboxy group can be free, or optionally protected, for instance with a conventional pharmaceutically acceptable ester such as the methyl or ethyl ester. Alternatively the optional protection of the free carboxy function can itself comprise an ester with an R₁ fatty alcohol, with one or both hydroxyl functions being esterified to R₂:

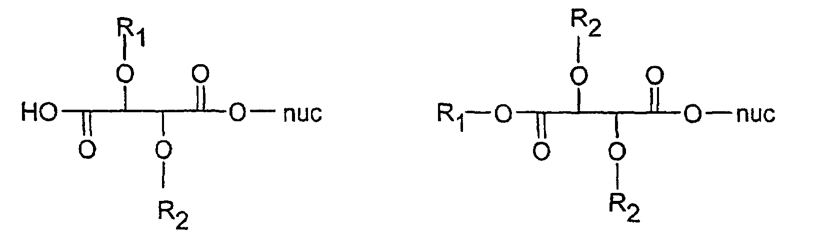

Favoured linkers of the tartaric acid series above can be generically depicted as Formula IIe: 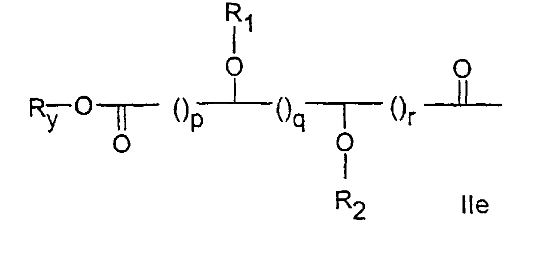 and isomers where R₁ and R₂ are reversed, where R₁ and R₂ are as shown above, p, q and r are each independently 0 to 5, preferably 0 or 1 and Ry is the free acid, an R₁ ester or a conventional pharmaceutically acceptable carboxy protecting group, such as the methyl, benzyl or especially the ethyl ester.

Favoured linkers of the malic series have the formula IIf: 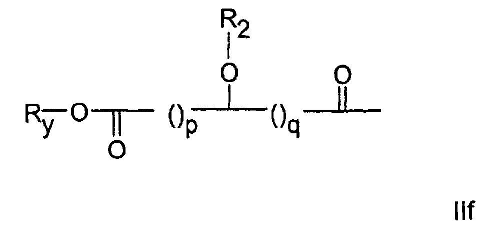 where Ry, p,q and R₂ are as defined above, preferably those where p and q are zero.

Preferred compounds of this type thus include:
5'-O-[3-methoxycarbonyl-2-valyloxy-propionyl]-2',3'-dideoxy-3'-fluoroguanosine,
5'-O-[3-benzyloxycarbonyl-2-valyloxy-propionyl]-2',3'-dideoxy-3'-fluoroguanosine,
5'-O-[3-methoxycarbonyl-2-isoleucoxy-propionyl]-2',3'-dideoxy-3'-fluoroguanosine,
5'-O-[3-benzyloxycarbonyl-2-isoleucyloxy-propionyl]-2',3'-dideoxy-3'-fluoroguanosine,
5'-O-[4-methoxycarbonyl-2,3-bis-valyloxy-butyryl]-2',3'-dideoxy-3'-fluoroguanosine,
5'-O-[4-benzyloxycarbonyl-2,3-bis-valyloxy-butyryl]-2',3'-dideoxy-3'-fluoroguanosine,
5'-O-[4-methoxycarbonyl-2,3-bis-isoleucyloxy-butyryl]-2',3'-dideoxy-3'-fluoroguanosine,
5'-O-[4-benzyloxycarbonyl-2,3-bis-isoleucyloxy-butyryl]-2',3'-dideoxy-3'-fluoroguanosine;
particularly those derived from L-malic acid and L-tartaric acid; and corresponding derivatives employing conventional pharmaceutically acceptable esters on the terminal carboxy function.

Particularly favoured compounds include:
5'-O-[3-ethoxycarbonyl-2-valyloxy-propionyl]-2',3'-dideoxy-3'-fluoroguanosine,
5'-O-[3-ethoxycarbonyl-2-isoleucyloxy-propionyl]-2',3'-dideoxy-3'-fluoroguanosine,
5'-O-[4-ethoxycarbonyl-2,3-bis-valyloxy-butyryl]-2',3'-dideoxy-3'-fluoroguanosine,
5'-O-[4-ethoxycarbonyl-2,3-bis-isoleucyloxy-butyryl]-2',3'-dideoxy-3'-fluoroguanosine,
expecially the isomers derived from L-malic and L-tartaric acid.

Related alternative compounds are those wherein one of R₁ and R₂ is omitted. Representative compounds include those of the formual Ia: 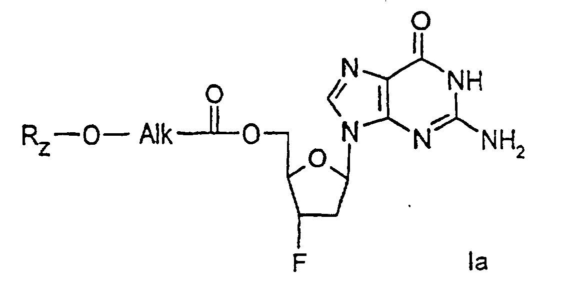 where Alk is optionally substituted C₁-C₄ alkyl or C₂-C₄ alkenyl and R_{z} is the ester residue of an aliphatic L-amino acid or a fatty acid as defined for R₁ and R₂ above. Such linkers are conveniently prepared from α-hydroxy ω-carboxylic acids such as carbonic acid, glycollic acid, hydroxypropanoic acid, hydroxybutyric acid, hydroxyvaleric acid or hydroxycaproic acid.

Representative compounds of Formula Ia include:
2',3'-dideoxy-3'-fluoro-5'-O-[3-(L-valyloxy)-propionyl] guanosine
2',3'-dideoxy-3'-fluoro-5'-O-[5-(L-valyloxy)-pentanoyl] guanosine,
2',3'-dideoxy-3'-fluoro-5'-O-[6-(L-valyloxy)-hexanoyl] guanosine,
2',3'-dideoxy-3'-fluoro-5-O-[3-(L-isoleuclyoxy)-propionyl] guanosine
2',3'-dideoxy-3'-fluoro-5'-O-[5-(L-isoleucyloxy)-pentanoyl] guanosine,
2',3'-dideoxy-3'-fluoro-5'-O-[6-(L-isoleucyloxy)-hexanoyl] guanosine,
and pharmaceutically acceptable salts thereof.

Particularly favoured compounds of formula Ia include:
2',3'-dideoxy-3'-fluoro-5'-O-[4-(L-valyloxy)-butyryl] guanosine; and
2',3'-dideoxy-3'-fluoro-5'-O-[4-(L-isoleucyloxy)-butyryl] guanosine and pharmaceutically acceptable salts thereof. In these compounds hydrolysis and removal of the R₂ group in vivo leaves a reactive terminal radical which will tend to cyclize and prompt the effective release of the mother nucleoside.

The present invention provides compounds of formula I, wherein both linkers L₁ and L₂ are absent and R₁ as a fatty acid residue is itself used as the linker, with the aliphatic L-amino acid residue of R₂ being esterified to a hydroxy function on the fatty acid alkyl chain, for example on the β-carbon. In one embodiment the fatty acid of R₁ is esterified directly on the 5'-hydroxy (or equivalent) function of the nucleoside, generally with the R₂ group already esterifiedon the fatty acid of R₁. Alternatively, the functionalised fatty acid (the hydroxy function being appropriately protected) can be first esterified to the nucleoside and deprotected prior to coupling with R₂. Compounds in accordance with the present invention have the formula Ig: 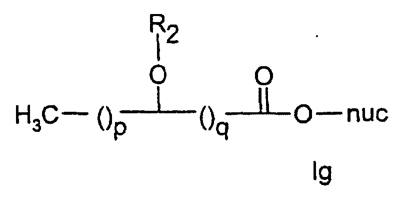 where O-nuc is the residue of a monohydroxyl bearing D- or L- nucleoside analogue; R₂ is the residue of an aliphatic L-amino acid,
p is 0, 1 or 2-20 (optionally including a double bond) and q is 0-5.

Representative and preferred compounds include the compounds as defined in claims 2 to 4. Particularly preferred compounds are:
2',3'-dideoxy-3'-fluoro-5-O-[2-(L-valyloxy)-butyryl] guanosine,
2',3'-dideoxy-3'-fluoro-5-O-[2-(L-valyloxy)-hexanoyl] guanosine,
2',3'-dideoxy-3'-fluoro-5-O-[2-(Irvalyloxy)-octanoyl] guanosine,
2',3'-dideoxy-3'-fluoro-5-O-[2-(L-valyloxy)-decanoyl] guanosine,
2',3'-dideoxy-3'-fluoro-5-O-[2-(L-valyloxy)-dodecanoyl] guanosine,
2',3'-dideoxy-3'-fluoro-5-O-[2-(L-valyloxy)-myristoyl] guanosine,
2',3'-dideoxy-3'-fluoro-5-O-[2-(L-valyloxy)-palmitoyl] guanosine,
2',3'-dideoxy-3'-fluoro-5-O-[2-(L-valyloxy)-stearoyl] guanosine,
2',3'-dideoxy-3'-fluoro-5-O-[2-(L-valyloxy)-docosanoyl] guanosine,
2',3'-dideoxy-3'-fluoro-5-O-[2-(L-valyloxy)-eicosanoyl] guanosine
2',3'-dideoxy-3'-fluoro-5-O-[2-(L-isoleucyloxy)-butyryl] guanosine,
2',3'-dideoxy-3'-fluoro-5-O-(2-(L-isoleucyloxy)-hexanoyl] guanosine,
2',3'-dideoxy-3'-fluoro-5-O-[2-(L-isoleucyloxy)-octanoyl] guanosine,
2',3'-dideoxy-3'-fluoro-5-O-[2-(L-isoleucyloxy)-decanoyl] guanosine,
2',3'-dideoxy-3'-fluoro-5-O-[2-(L-isoleucyloxy)-dodecanoyl] guanosine,
2',3'-dideoxy-3'-fluoro-5-O-[2-(L-isoleucyloxy)-myristoyl] guanosine,
2',3'-dideoxy-3'-fluoro-5-O-[2-(L-isoleucyloxy)-palmitoyl] guanosine,
2',3'-dideoxy-3'-fluoro-5-O-[2-(L-isoleucyloxy)-stearoyl] guanosine,
2',3'-dideoxy-3'-fluoro-5-O-[2-(L-isoleucyloxy)-docosanoyl] guanosine,
2',3'-dideoxy-3'-fluoro-5-O-[2-(L-isoleucyloxy)-eicosanoyl] guanosine,
and the corresponding n-3 and n-6 monounsaturated analogues, such as 6 or 9 octadecenoyl derivatives.

In formula Ig, p and q are preferably 0, thus defining lactic acid derivatives, preferably L-lactic acid derivatives, such as
2',3'-dideoxy-3'-fluoro-5-O-[2-(L-valyloxy)-propionyl] guanosine; and
2',3'-dideoxy-3'-fluoro-5-O-[2-(L-isoleucyloxy)-propionyl] guanosine and pharmaceutically acceptable salts thereof, as the breakdown products, lactic acid and the amino acid are both well accepted physiologically.

The expression bifunctional in the context of second linker group L₂ means that the the linker has two functions enabling it to act as a spacer or bridge between the first linker group L₁ and the 5'-O group of the nucleoside. For instance the optional group L₂ may comprise a linker of the formula IIIa: 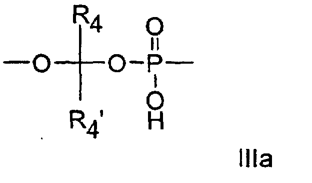 where R₄ and R₄' are hydrogen or C₁-C₄ alkyl. In formula IIIa, R₄ is preferably hydrogen, methyl, ethyl or isopropyl and R₄' is hydrogen. Linkers of formula IIIa are convenient as many nucleosides such as the FLG mother compound must first be phosphorylated by cellular enzymes before it can inhibit the viral polymerase. An initial or sequential hydrolysis of such compounds can release a monophosphorylated nucleoside in vivo which is available for immediate conversion to the di- and triphosphate.

Alternatively the optional bifunctional linker group L₂ may comprise a structure of the formula IIIb: 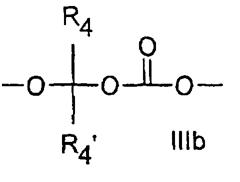 where R₄ and R₄' are independently H or C₁-C₄ alkyl.

A still further group of bifunctional linkers have the formula IIIc:

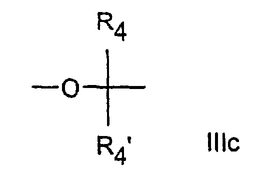

As described above, a preferred group of bifunctional linkers comprises α,ω-dicarboxylic C₂-C₆ alkyl derivatives, such as succinic acid, which are optionally substituted (for instance with the substituents defined above for R₁ as a fatty acid) and/or optionally mono or polyunsaturated, such as n-3 or n-6 monounsaturated. Preferred moieties within this class are listed above.

Although the disclosure above has concentrated on glycerol L₁ groups in conjunction with dicarboxylic L₂ groups, it will be appreciated that a wide variety of trifunctional linkers are appropriate with dicarboxylic L₂ groups, for instance structures of the formula IIa and IIb above lacking the rightmost carbonyl.

Other suitable compounds are double prodrugs comprising R₁(R₂) L₁L₂-derivatives of conventional FLG prodrugs, which conventional prodrugs release FLG in vivo, such as prodrug derivatives at the 2 and 6 positions of the FLG guanine base. Examples of such conventional FLG-prodrugs include compounds of the formula IV: 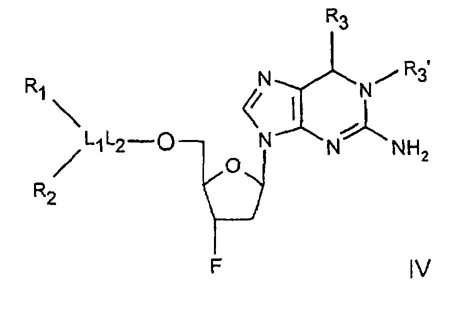 where R₁, R2, L₁ and L₂ are as defined above; and
R₃ is H, N₃, NH₂, or OH or a pharmaceutically acceptable ether or ester thereof; and
R₃' is an aromatic bond or hydrogen;

Potential pharmaceutically acceptable esters for R₃ include the fatty acids described in relation to R₁ above, such as stearoyl, oleoyl etc or shorter esters such as acetyl or butyryl. Other potential esters include the amino acid derivatives of R₂ or esters of phosphoric acid, such as monophosphate. Alternative esters include the corresponding fatty acid or alkylaryl carbonate, carbamate or sulphonic esters.

Suitable pharmaceutically acceptable ethers for R₃ include C₁-C₆ alkyl, cycloalkyl, C₆-C₁₂ alkaryl such as benzyl or methylpyridyl, any of which may be optionally substituted as for R₁ above. Convenient ethers include those described in the abovementioned WO 93/13778 such as n-propoxy, cyclobutoxy, cyclopropanylamino, piperidino or pyrrolidino and the like.

Representative nucleosides in accordance with the present invention include acyclic nucleoside analogues such as acyclovir and cyclic nucleoside analogues such as ddI (didanosine), ddC (zalcitabine), d4T (stavudine), FTC, lamivudine (3TC), 1592U89 (4-[2-amino-6-(cyclopropylamino)-9H-purin-9-yl],2-cyclopentene-1-methanol), AZT (zidovudine), DAPD (D-2,6-diaminopurine dioxolane), F-ddA and the like, each of which are well known in the nucleoside art. A number of monohydric L-nucleosides are under development and the invention will also find utility on this compounds. Compounds within this aspect of the invention will find utility in the corresponding indications to the mother compounds, for instance herpesvirus infections for acyclovir derivatives, HIV for ddI, stavudine, ddC, lamivudine, AZT & 1592U89, HBV for lamivudine, FTC etc.

A favoured subgroup of nucleoside analogues comprises derivatives of monohydric nucleosides of the formula Ic': 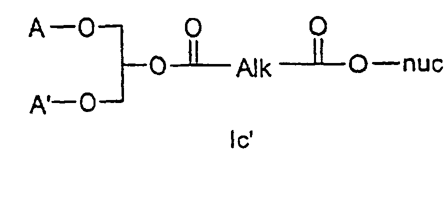 where A, A', Alk and O-nuc are as defined above. Formula Ic' above depicts compounds wherein A and A' depend from the 1 and 3 positions of the glycereol moiety and L₂ depends from the glycerol 2 position. In alternative isomers A and A' depend 1 and 2 or 2 and 3 and L₂ from 3 or 2 respectively.

Representative compounds within this subgroup include:
4'-O-[3-((2,3-bis-L-valyloxy)-1-propyloxycarbonyl)propionyl] acyclovir,
4'-O-[3-((2-hydroxy-3-L-valyloxy)-1-propyloxycarbonyl)propionyl] acyclovir,
4'-O-[3-((2,3-bis-L-isoleucyloxy)-1-propyloxycarbonyl)propionyl] acyclovir,
4'-O-[3-((2-hydroxy-3-L-isoleucyloxy)-1-propyloxycarbonyl)propionyl] acyclovir,
4'-O-[3-((1,3-bis-L-valyloxy)-2-propyloxycarbonyl)propionyl] acyclovir,
4'-O-[3-((1-hydroxy-3-L-valyloxy)-2-propyloxycarbonyl)propionyl] acyclovir,
4'-O-[3-((1,3-bis-L-isoleucyloxy)-2-propyloxycarbonyl)propionyl] acyclovir,
4'-O-[3-((1-hydroxy-3-L-isoleucyloxy)-2-propyloxycarbonyl)propionyl] acyclovir,
5'-O-[3-((2,3-bis-L-valyloxy)-1-propyloxycarbonyl)propionyl] lamivudine,
5'-O-[3-((2-hydroxy-3-L-valyioxy)-1-propyloxycarbonyl)propionyl] lamivudine,
5'-O-[3-((2,3-bis-L-isoleucyloxy)-1-propyloxycarbonyl)propionyl] lamivudine,
5'-O-[3-((2-hydroxy-3-L-isoleucyloxy)-1-propyloxycarbonyl)propionyl] lamivudine,
5'-O-[3-((1,3-bis-L-valyloxy)-2-propyloxycarbonyl)propionyl] lamivudine,
5'-O-[3-((1-hydroxy-3-L-valyloxy)-2-propyloxycarbonyl)propionyl] lamivudine,
5'-O-[3-((1,3-bis-L-isoleucyloxy)-2-propyloxycarbonyl)propionyl] lamivudine,
5'-O-[3-((1-hydroxy-3-L-isoleucyloxy)-2-propyloxycarbonyl)propionyl] lamivudine,
5'-O-[3-((2,3-bis-L-valyloxy)-1-propyloxycarbonyl)propionyl] DAPD,
5'-O-[3-((2-hydroxy-3-L-valyloxy)-1-propyloxycarbonyl)propionyl]DAPD,
5'-O-[3-((2,3-bis-L-isoleucyloxy)-1-propyloxycarbonyl)propionyl] DAPD,
5'-O-[3-((2-hydroxy-3-L-isoleucyloxy)-1-propyloxycarbonyl)propionyl] DAPD,
5'-O-[3-((1,3-bis-L-valyioxy)-2-propyloxycarbonyl)propionyl] DAPD,
5'-O-[3-((1-hydroxy-3-L-valyloxy)-2-propyloxycarbonyl)propionyl] DAPD,
5'-O-[3-((1,3-bis-L-isoleucyloxy)-2-propyloxycarbonyl)propionyl] DAPD,
5'-O-[3-((1-hydroxy-3-L-isoleucyloxy)-2-propyloxycarbonyl)propionyl] DAPD,
5'-O-[3-((2,3-bis-L-valyloxy)-1-propyloxycarbonyl)propionyl]-2',3'-dideoxyinosine
5'-O-[3-((2-hydroxy-3-L-valyloxy)-1-propyloxycarbonyl)propionyl]-2',3'-dideoxyinosine,
5'-O-[3-((2,3-bis-L-isoleucyloxy)-1-propyloxycarbonyl)propionyl]-2',3'-dideoxyinosine,
5'-O-[3-((2-hydroxy-3-L-isoleucyloxy)-1-propyloxycarbonyl)propionyl]-2',3'-dideoxyinosine,
5'-O-[3-((1,3-bis-L-valyloxy)-2-propyloxycarbonyl)propionyl]-2',3'-dideoxyinosine,
5'-O-[3-((1-hydroxy-3-L-valyloxy)-2-propyloxycarbonyl)propionyl]-2',3'-dideoxyinosine,
5'-O-[3-((1,3-bis-L-isoleucyloxy)-2-propyloxycarbonyl)propionyl]-2',3'-dideoxyinosine,
5'-O-[3-((1-hydroxy-3-L-isoleucyloxy)-2-propyloxycarbonyl)propionyl]-2',3'-dideoxyinosine,
5'-O-[3-((2,3 -bis-L-valyloxy)-1-propyloxycarbonyl)propionyl]stavudine,
5'-O-[3-((2-hydroxy-3-L-valyloxy)-1-propyloxycarbonyl)propionyl]stavudine,
5'-O-[3-((2,3-bis-L-isoleucyloxy)-1-propyloxycarbonyl)propionyl] stavudine,
5'-O-[3-((2-hydroxy-3-L-isoleucyloxy)-1-propyloxycarbonyl)propionyl] stavudine,
5'-O-[3-((1,3-bis-L-valyloxy)-2-propyloxycarbonyl)propionyl] stavudine,
5'-O-[3-((1-hydroxy-3-L-valyloxy)-2-propyloxycarbonyl)propionyl] stavudine,
5'-O-[3-((1,3-bis-L-isoleucyloxy)-2-propyloxycarbonyl)propionyl] stavudine,
5'-O-[3-((1-hydroxy-3-L-isoleucyloxy)-2-propyloxycarbonyl)propionyl] stavudine,
the corresponding derivatives of 4-[2-amino-6(cyclopropylamino)-9*H*-purin-9-yl]-2-cyclopentene-1-methanol, and pharmaceutically acceptable salts thereof.

An alternative subset of compounds comprises those of the formula Id: 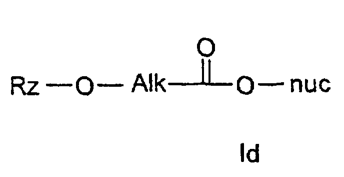 where Rz and Alk are as defined for formula Ia and O-nuc is as defined above.

Representative compounds of formula Id include
4'-O-[4-(L-valyloxy)-propionyl] acyclovir,
4'-O-[5-(L-valyloxy)-pentanoyl] acyclovir,
4'-O-[6-(L-valyloxy)-hexanoyl] acyclovir,
4'-O-[4-(L-isoleucyloxy)-propionyl] acyclovir,
4'-O-[5-(L-isoleucyloxy)-pentanoyl] acyclovir,
4'-O-[6-(L-isoleucyloxy)-hexanoyl] acyclovir,
5'-O-[4-(L-valyloxy)-propionyl] ddI,
5'-O-[5-(L-valyloxy)-pentanoyl] ddI,
5'-O-[6-(L-valyloxy)-hexanoyl] ddI,
5'-O-[4-(L-isoleucyloxy)-propionyl] ddI,
5'-O-[5-(L-isoleucyloxy)-pentanoyl] ddI,
5'-O-[6-(L-isoleucyloxy)-hexanoyl] ddI,
5'-O-[4-(L-valyloxy)-propionyl] stavudine,
5'-O-[5-(L-valyloxy)-pentanoyl] stavudine,
5'-O-[6-(L-valyloxy)-hexanoyl] stavudine,
5'-O-[4-(L-isoleucyloxy)-propionyl] stavudine,
5'-O-[5-(L-isoleucyloxy)-pentanoyl] stavudine,
5'-O-[6-(L-isoleucyloxy)-hexanoyl] stavudine,
5'-O-[4-(L-valyloxy)-propionyl] DAPD,
5'-O-[5-(L-valyloxy)-pentanoyl] DAPD,
5'-O-[6-(L-valyloxy)-hexanoyl] DAPD,
5'-O-[4-(L-isoleucyloxy)-propionyl] DAPD,
5'-O-[5-(L-isoleucyloxy)-pentanoyl] DAPD,
5'-O-[6-(L-isoleucyloxy)-hexanoyl] DAPD,
5'-O-[4-(L-valyloxy)-propionyl] lamivudine,
5'-O-[5-(L-valyloxy)-pentanoyl]lamivudine,
5'-O-[6-(L-valyloxy)-hexanoyl] lamivudine,
5'-O-[4-(L-isoleucyloxy)-propionyl] lamivudine,
5'-O-[5-(L-isoleucyloxy)-pentanoyl] lamivudine,
5'-O-[6-(L-isoleucyloxy)-hexanoyl] lamivudine,
and the corresponding derivatives of 4-[2-amino-6(cyclopropylamino)-9*H*-purin-9-yl]-2-cyclo-pentene-1-methanol.

Particularly preferred compounds within Formula Id include:
4'-O-[4-(L-valyloxy)-butyryl]acyclovir,
4'-O-[3-(L-isoleucyloxy)-butyryl]acyclovir,
5'-O-[4-(L-valyloxy)-butyryl]ddI,
5'-O-[3-(L-isoleucyloxy)-butyryl] ddI,
5'-O-[4-(L-valyloxy)-butyryl]stavudine,
5'-O-[3-(L-isoleucyloxy)-butyryl] stavudine,
5'-O-[4-(L-valyloxy)-butyryl] DAPD,
5'-O-[3-(L-isoleucyloxy)-butyryl] DAPD,
5'-O-[4-(L-valyloxy)-butyryl]lamivudine,
5'-O-[3-(L-isoleucyloxy)-butyryl]lamivudine,
and the corresponding derivatives of 4-[2-amino-6(cyclopropylamino)-9*H*-purin-9-yl]-2-cyclopentene-1-methanol; and pharmaceutically acceptable salts thereof.

In these compounds hydrolysis and removal of the R₂ group in vivo leaves a reactive terminal radical which will tend to cyclize and prompt the effective release of the mother nucleoside.

Similarly, other nucleosides are compounds of formula If: 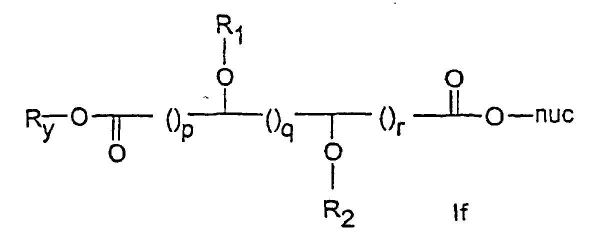 where R₁, R₂, R_{y}, p, q, r and O-nuc are as defined above.

Favoured compounds of formula If include:
5'-O-[3-ethoxycarbonyl-2-valyloxy-propionyl]-ddl,
5'-O-[3-ethoxycarbonyl-2-isoleucyloxy-propionyl]-ddI
5'-O-[4-ethoxycarbonyl-2,3-bis-valyloxy-butyryl]-ddl,
5'-O-[4-ethoxycarbonyl-2,3-bis-isoleucyloxy-butyryl]-ddI,
4'-O-[3-ethoxycarbonyl-2-valyloxy-propionyl]-acyclovir,
4'-O-[3-ethoxycarbonyl-2-isoleucyloxy-propionyl]-acyclovir
4'-O-[4-ethoxycarbonyl-2,3-bis-valyloxy-butyryl]-aciclovir,
4'-O-[4-ethoxycarbonyl-2,3-bis-isoleucyloxy-butyryl]-aciclovir,
5'-O-[3-ethoxycarbonyl-2-valyloxy-propionyl]-DAPD,
5'-O-[3-ethoxycarbonyl-2-isoleucyloxy-propionyl)-DAPD
5'-O-[4-ethoxycarbonyl-2,3-bis-valyloxy-butyryl]-DAPD,
5'-O-[4-ethoxycarbonyl-2,3-bis-isoleucyloxy-butyryl]-DAPD,
5'-O-[3-ethoxycarbonyl-2-valyloxy-propionyl]-stavudine,
5'-O-[3-ethoxycarbonyl-2-isoleucyloxy-propionyl]-stavudine
5'-O-[4-ethoxycarbonyl-2,3-bis-valyloxy-butyryl]-stavudine,
5'-O-[3-ethoxycarbonyl-2-valyloxy-propionyl]-lamivudine,
5'-O-[3-ethoxycarbonyl-2-isoleucyloxy-propionyl]-lamivudine
5',O-[4-ethoxycarbonyl-2,3-bis-valyloxy-butyryl]-lamivudine,
5'-O-[4-ethoxycarbonyl-2,3-bis-isoleucyloxy-butyryl]-lamivudine,
and the corrresponding malic and tartric derivatives of 4-[2-amino-6(cyclopropyl-amino)-9*H*-purin-9-yl]-2-cyclopentene-1-methanol and pharmaceutically acceptable salts thereof; in each case the isomers derived from L-tartrate and L-malate derivatives being preferred.

The present invention extends to compounds of the formula Ig 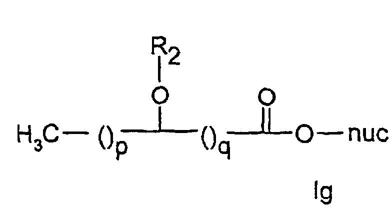 where R₂, p, q and O-nuc are as defined above.

Preferred compounds of formula Ig include:
4'-O-[2-(L-valyloxy)-propionyl] acyclovir,
4'-O-[2-(L-isoleucyloxy)-propionyl] acyclovir
5'-O-[2-(L-valyloxy)-propionyl] ddI,
5'-O-[2-(L-isoleucyloxy)-propionyl] ddI,
5'-O-[2-(L-valyloxy)-propionyl] stavudine,
5',O-[2-(L-isoleucyloxy)-propionyl] stavudine
5'-O-[2-(L-valyloxy)-propionyl] lamivudine,
5'-O-[2-(L-isoleucyloxy)-propionyl] lamivudine,
5'-O-[2-(L-valyloxy)-propionyl] DAPD,
5'-O-[2-(L-isoleucyloxy)-propionyl] DAPD
and the corresponding derivatives of 4-[2-amino-6(cyclopropylamino)-9*H*-purin-9-yl]-2-cyclopentene-1-methanol;and pharmaceutically acceptable salts thereof.

The breakdown products of such compounds, lactic acid and the amino acid, are both well accepted physiologically.

The compounds of the invention can form salts which form an additional aspect of the invention. Appropriate pharmaceutically acceptable salts of the compounds of Formula Ig include salts of organic acids, especially carboxylic acids, including but not limited to acetates, trifluoroacetates, lactates, gluconates, citrates, tartrates, maleates, malates, pantothenates, isethionates, adipates, alginates, aspartates, benzoates, butyrates, digluconates, cyclopentanates, glucoheptanates, glycerophosphates, oxalates, heptanoates, hexanoates, fumarates, nicotinates, palmoates, pectinates, 3-phenylpropionates, picrates, pivalates, proprionates, lactobionates, camphorates, undecanoates and succinates, organic sulphonic acids such as methanesulphonates, ethanesulphonates, 2-hydroxyethane sulphonates, camphorsulphonates, 2-napthalenesulphonates, benzenesulphonates, p-chlorobenzenesulphonates and p-toluenesulphonates; and inorganic acids such as hydrochlorides, hydrobromides, hydroiodides, sulphates, bisulphates, hemisulphates, thiocyanates, persulphates, phosphoric and sulphonic acids. The compounds of Formula Ig may in some cases be isolated as the hydrate.

The term "N-protecting group" or "N-protected" as used herein refers to those groups intended to protect the N-terminus of an amino acid or peptide or to protect an amino group against undesirable reactions during synthetic procedures. Commonly used N-protecting groups are disclosed in Greene, "Protective Groups in Organic Synthesis" (John Wiley & Sons, New York, 1981), which is hereby incorporated by reference. N-protecting groups include acyl groups such as formyl, acetyl, propionyl, pivaloyl, t-butylacetyl, 2-chloroacetyl, 2-bromoacetyl, trifluoracetyl, trichloroacetyl, phthalyl, o-nitrophenoxyacetyl, α-chlorobutyryl, benzoyl, 4-chlorobenzoyl, 4-bromobenzoyl, 4-nitrobenzoyl, and the like; sulfonyl groups such as benzenesulfonyl, p-toluenesulfonyl, and the like, carbamate forming groups such as benzyloxycarbonyl, p-chlorobenzyloxycarbonyl, p-methoxybenzyloxycarbonyl, p-nitrobenzyloxycarbonyl, 2-nitrobenzyloxycarbonyl, p-bromobenzyloxycarbonyl, 3,4-dimethoxybenzyl-oxycarbonyl, 4-methoxybenzyloxycarbonyl, 2-nitro-4,5-dimethoxybenzyloxy-car-bo-nyl, 3,4,5-trimethoxybenzyloxycarbonyl, 1-(p-biphenylyl)-1-methylethoxycarbonyl, α,α-dimethyl-3,5-dimethoxybenzyloxycarbonyl, benzhydryloxycarbonyl, t-butoxycarbonyl, diisopropylmethoxycarbonyl, isopropyloxycarbonyl, ethoxycarbonyl, methoxycarbonyl, allyloxycarbonyl, 2,2,2-trichloroethoxycarbonyl, phenoxycarbonyl, 4-nitrophenoxycarbonyl, fluorenyl-9-methoxycarbonyl, cyclopentyloxycarbonyl, adamantyloxycarbonyl, cyclohexyloxycarbonyl, phenylthiocarbonyl, and the like; alkyl groups such as benzyl, triphenylmethyl, benzyloxymethyl and the like; and silyl groups such as trimethylsilyl and the like. Favoured N-protecting groups include formyl, acetyl, allyl, F-moc, benzoyl, pivaloyl, t-butylacetyl, phenylsulfonyl, benzyl, t-butoxycarbonyl (BOC) and benzyloxycarbonyl (Cbz).

Hydroxy and/or carboxy protecting groups are also extensively reviewed in Greene ibid and include ethers such as methyl, substituted methyl ethers such as methoxymethyl, methylthiomethyl, benzyloxymethyl, t-butoxymethyl, 2-methoxyethoxymethyl and the like, silyl ethers such as trimethylsilyl (TMS), t-butyldimethylsilyl (TBDMS), tribenzylsilyl, triphenylsilyl, t-butyldiphenylsilyl, triisopropyl silyl and the like, substituted ethyl ethers such as 1-ethoxymethyl, 1-methyl-1-methoxyethyl, t-butyl, allyl, benzyl, p-methoxybenzyl, diphenylmethyl, triphenylmethyl and the like, aralkyl groups such as trityl, and pixyl (9-hydroxy-9-phenylxanthene derivatives, especially the chloride). Ester hydroxy protecting groups include esters such as formate, benzylformate, chloroacetate, methoxyacetate, phenoxyacetate, pivaloate, adamantoate, mesitoate, benzoate and the like. Carbonate hydroxy protecting groups include methyl vinyl, allyl, cinnamyl, benzyl and the like.

In keeping with the usual practice with retroviral and HBV inhibitors it is advantageous to co-administer one to three or more additional antivirals, such as AZT, ddI, ddC, d4T, 3TC, H2G, foscarnet, ritonavir, indinavir, saquinavir, nevirapine, delaviridine, Vertex VX 478 or Agouron AG1343 and the like in the case of HIV or lamivudine, interferon, famciclovir etc in the case of HBV. Such additional antivirals will normally be administered at dosages relative to each other which broadly reflect their respective therapeutic values. Molar ratios of 100:1 to 1:100, especially 25:1 to 1:25, relative to the compound or salt of formula Ig will often be convenient. Administration of additional antivirals is generally less common with those antiviral nucleosides intended for treating herpes infections.

While it is possible for the active agent to be administered alone, it is preferable to present it as part of a pharmaceutical formulation. Such a formulation will comprise the above defined active agent together with one or more acceptable carriers/excipients and optionally other therapeutic ingredients. The carrier(s) must be acceptable in the sense of being compatible with the other ingredients of the formulation and not deleterious to the recipient.

The formulations include those suitable for rectal, nasal, topical (including buccal and sublingual), vaginal or parenteral (including subcutaneous, intramuscular, intravenous and intradermal) administration, but preferably the formulation is an orally administered formulation. The formulations may conveniently be presented in unit dosage form, e.g. tablets and sustained release capsules, and may be prepared by any methods well known in the art of pharmacy.

Such methods include the step of bringing into association the above defined active agent with the carrier. In general, the formulations are prepared by uniformly and intimately bringing into association the active agent with liquid carriers or finely divided solid carriers or both, and then if necessary shaping the product. The invention extends to methods for preparing a pharmaceutical composition comprising bringing a compound of Formula Ig or its pharmaceutically acceptable salt in conjunction or association with a pharmaceutically acceptable carrier or vehicle. If the manufacture of pharmaceutical formulations involves intimate mixing of pharmaceutical excipients and the active ingredient in salt form, then it is often preferred to use excipients which are non-basic in nature, i.e. either acidic or neutral.

Formulations for oral administration in the present invention may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus etc.

With regard to compositions for oral administration (e.g. tablets and capsules), the term suitable carrier includes vehicles such as common excipients e.g. binding agents, for example syrup, acacia, gelatin, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatin, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc, waxes, oils and colloidal silica. Flavouring agents such as peppermint, oil of wintergreen, cherry flavouring or the like can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the active agent in a free flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally be coated or scored and may be formulated so as to provide slow or controlled release of the active agent.

Other formulations suitable for oral administration include lozenges comprising the active agent in a flavoured base, usually sucrose and acacia or tragacanth; pastilles comprising the active agent in an inert base such as gelatin and glycerin, or sucrose and acacia; and mouthwashes comprising the active agent in a suitable liquid carrier.

Compounds of Formulae I or Ic are prepared by a method comprising the acylation of the nucleoside, represented here by FLG, Formula V, typically at the 5' hydroxy group: 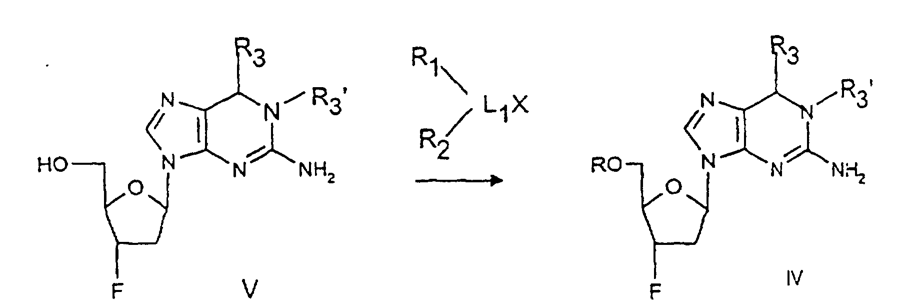 in which R₁(R₂)L₁X represents an activated acid, such as the carboxylic derivatives of Formula IIa or IIb, where R₁, R₂, and L₁ are as defined above or protected derivatives thereof.

Alternatively the activated acid may comprise a compound of the formula R₁(R₂)glycerol-D-X, where R₁, R₂ and D are as defined in formula IIc or an activated Rz-O-Alk-C(=O)X derivative in the case of compounds of formula Ia. In the latter cases the linkers may be built up sequentially by first esterifying a suitably protected D or ω-hydroxy carboxylic acid to the nucleoside, deprotecting the terminal carboxy or hydroxy function and esterifying the suitably protected glycerol or Rz moiety thereon.

The activated derivative used in the acylation may comprise e.g, the acid halide, acid anhydride, activated acid ester or the acid in the presence of coupling reagent, for example dicyclohexylcarbodiimide. Representative activated acid derivatives include the acid chloride, anhydrides derived from alkoxycarbonyl halides such as isobutyloxycarbonylchloride and the like, N-hydroxysuccinamide derived esters, N-hydroxyphthalimide derived esters, N-hydroxy-5-norbornene- 2,3-dicarboxamide derived esters, 2,4,5-trichlorophenol derived esters and the like. Further activated acids include those where X in the formula RX represents an OR'moiety where R is R₂ as defined herein, and R' is, for example COCH₃, COCH₂CH₃ or COCF₃ or where X is benzotriazole.

Corresponding methodology will be applicable when other monohydroxylated nucleosides are to be prepared, that is the activated derivative is correspondingly esterified to the free 5' hydroxy (or equivalent) of monohydric nucleosides such as acyclovir, ddI, FTC, lamivudine, 1592U89, DAPD, F-ddA and the like.

The intermediates used in the above methods themselves define novel compounds, especially those of the formula: IIc' 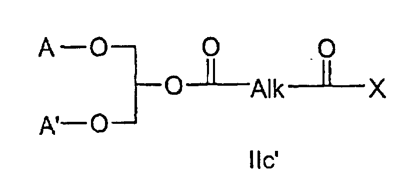 where A, A' and Alk are as defined above (A and A' being optionally protected with conventional protecting groups) and X represents the free acid or an activated acid as illustrated above.

Representative compounds of the formula IIc' include:
malonic acid 2,3-*bis*-(L-valyloxy)-propyl ester,
malonic acid 2,3-*bis*-(N-CBZ-L-valyloxy)-propyl ester,
malonic acid 2,3-*bis*-(N-Fmoc-L-valyloxy)-propyl ester,
malonic acid 2,3-*bis*-(N-Boc-L-valyloxy)-propyl ester,
malonic acid 2,3-*bis*-(L-isoleucyloxy)-propyl ester,
malonic acid 2,3-*bis*-(N-CBZ-L-isoleucyloxy)-propyl ester,
malonic acid 2,3-*bis*-(N-Fmoc-L-isoleucyloxy)-propyl ester,
malonic acid 2,3-*bis*-(N-Boc-L-isoleucyloxy)-propyl ester,
succinic acid 2,3-*bis*-(L-valyloxy)-propyl ester,
succinic acid 2,3-*bis*-(N-CBZ-L-valyloxy)-propyl ester,
succinic acid 2,3-*bis*-(N-Fmoc-L-valyloxy)-propyl ester,
succinic acid 2,3-*bis*-(N-Boc-L-valyloxy)-propyl ester,
succinic acid 2,3-*bis*-(L-isoleucyloxy)-propyl ester,
succinic acid 2,3-*bis*-(N-CRZ-L-isoleucyloxy)-propyl ester,
succinic acid 2,3-*bis*-(N-Fmoc-L-isoleucyloxy)-propyl ester,
succinic acid 2,3-*bis*-(N-Boc-L-isoleucyloxy)-propyl ester,
glutaric acid 2,3-*bis*-(L-valyloxy)-propyl ester,
glutaric acid 2,3-*bis*-(N-CBZ-L-valyloxy)-propyl ester,
glutaric acid 2,3-*bis*-(N-Fmoc-L-valyloxy)-propyl ester,
glutaric acid 2,3-*bis*-(N-Boc-L-valyloxy)-propyl ester,
glutaric acid 2,3-*bis*-(L-isoleucyloxy)-propyl ester,
glutaric acid 2,3-*bis*-(N-CBZ-L-isoleucyloxy)-propyl ester,
glutaric acid 2,3-*bis*-(N-Fmoc-L-isoleucyloxy)-propyl ester,
glutaric acid 2,3-*bis*-(N-Boc-L-isoleucyloxy)-propyl ester,
and the corresponding acid halides, in particular the chloride, acid anhydrides and diesters of each of the above, for instance
succinic acid 2,3-*bis*-(N-CBZ-L-valyloxy)-propyl ester,4-methoxybenzyl ester
succinic acid 2,3-*bis*-(N-CBZ-L-valyloxy)-propyl ester, 1,1-dimethylethyl ester, etc.

A further preferred group of intermediates comprise those of the formula IIa': 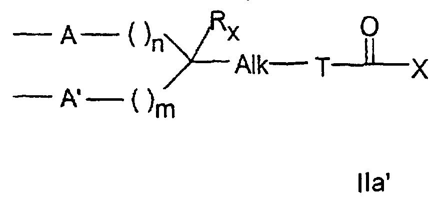 where Rₓ, Alk, m, n and T are as described above, A and A' represent acyl residues of L'-aliphatic amino acids (N-protected as necessary) esterified to hydroxy functions on the linker or one of A and A' is the acyl residue and the other is a free hydroxy group, and X represents the free acid or an activated acid as illustrated above. Preferably A and A' are the same amino acid residue.

Other novel intermediates include the free or activated acid precursors of compounds of the formula Ia such as:
3-N-Boc-L-valyloxypropanoic acid, 3-N-Fmoc-L-valyloxypropanoic acid, 3-N-CBZ-L-valyloxypropanoic acid, 3-N-Boc-L-isoleucyloxypropanoic acid, 3-N-Fmoc-L-isoleucyloxypropanoic acid, 3-N-CBZ-L-isoleucyloxypropanoic acid, 4-N-Boc-L-valyloxybutyric acid, 3-N-Fmoc-L-valyloxybutyric acid, 4-N-CBZ-L-valyloxybutyric acid, 4-N-Boc-L-isoleucyloxybutyric acid, 3-N-Fmoc-L-isoleucyloxybutyric acid, 3-N-CBZ-L-isoleucyloxybutyric acid and the like; and the activated derivatives, such as the acid halides

Further novel intermediates include precursors of compounds of the formula IIe and IIf above, especially those derived from "natural" configurations such as L-malic nd L-tartaric acid; for instance:
3-ethoxycarbonyl-2-valyloxy-propionic acid
3-ethoxycarbonyl-2-isoleucyloxy-propionic acid
4-ethoxycarbonyl-2,3-bis-valyloxy-butyric acid
4-ethoxycarbonyl-2,3-bis-isoleucyloxy-butyric acid
3-benzyloxycarbonyl-2-valyloxy-propionic acid
3-benzyloxycarbonyl-2-isoleucyloxy-propionic acid
4-benzyloxycarbonyl-2,3-bis-valyloxy-butyric acid
4-benzyloxycarbonyl-2,3-bis-isoleucyloxy-butyric acid, and the like;
and the corresponding activated derivatives such as the acid halides.

Still further novel intermediates include precursors corresponding to structure IId, such as; 2-(L-valyloxy)propanoic acid, 2-(N-Boc-L-valyloxy)propanoic acid, 2-(N-Fmoc-L-valyloxy)propanoic acid, 2-(N-CBZ-L-valyloxy)propanoic acid, 2-(L-isoleucyloxy)propanoic acid, 2-(N-Boc-L-isoleucyloxy)propanoic acid, N-(Fmoc-L-isoleucyloxy)propanoic acid, N-(CBZ-L-isoleucyloxy)propanoic acid, 2-(L-valyloxy)butyric acid, 2-(N-Boc-L-valyloxy)butyric acid, 2-(N-Fmoc-L-valyloxy)butyric acid, 2-(N-CBZ-L-valyloxy)butyric acid, 2-(L-isoleucyloxy)butyric acid, 2-(N-Boc-L-isoleucyloxy)butyric acid, N-(Fmoc-L-isoleucyloxy)butyric acid, N-(CBZ-L-isoleucyloxy)butyric acid, and the like; and activated derivatives therof, such as the acid halides.

Preparation of 3' fluoronucleosides such as those of formula V has been extensively reviewed by Herdiwijn et al. in Nucleosides and Nucleotides 8 (1) 65-96 (1989). The preparation of other monohydric nucleosides such as acyclovir, ddI (didanosine), ddC (zalcitabine), d4T (stavudine), FTC, lamivudine (3TC), 1592U89 (4-[2-amino-6-(cyclopropylamino)-9H-purin-9-yl]-2-cyclopentene-1-methanol), AZT (zidovudine), DAPD (D-2,6-diaminopurine dioxolane), F-ddA and the like are well known and extensively described in the literature.

The reactive derivatives of the R₁(R₂)L₁L₂X group may be pre-formed or generated in situ by the use of reagents such as dicyclohexylcarbodiimide (DCC) or O-(1H-benzotriazol-1-yl) N,N,N',N'-tetramethyluronium tetrafluoroborate (TBTU). When an acid halide, such as the acid chloride is used, a tertiary amine catalyst, such as triethylamine, N,N'-dimethylaniline, pyridine or dimethylaminopyridine may be added to the reaction mixture to bind the liberated hydrohalic acid.

The reactions are preferably carried out in an unreactive solvent such as N,N-dimethylformamide, tetrahydrofuran, dioxane, acetonitrile or a halogenatated hydrocarbon, such as dichloromethane. If desired, any of the above mentioned tertiary amine catalysts may be used as solvent, taking care that a suitable excess is present. The reaction temperature can typically be varied between 0° C and 60° C, but will preferably be kept between 5° and 50° C. After a period of 1 to 60 hours the reaction will usually be essentially complete. The progress of the reaction can be followed using thin layer chromatography (TLC) and appropriate solvent systems. In general, when the reaction is completed as determined by TLC, the product is extracted with an organic solvent and purified by chromatography and/or recrystallisation from an appropriate solvent system.

By-products where acylation has taken place on the nucleoside base can be separated by chromatography, but such misacylation can be minimized by controlled reaction conditions. These controlled conditions can be achieved, for example, by manipulating the reagent concentrations or rate of addition, especially of the acylating agent, by lowering the temperature or by the choice of solvent. The reaction can be followed by TLC to monitor the controlled conditions. It may be convenient to protect the 6-oxo group on the base and especially the 2 amino with conventional protecting groups to forestall misacylation.

Compounds of Formula IV in which R₃ is hydrogen may be prepared by 6-activating the correponding guanine compound of Formula I (wherein the exposed amino function of the amino acid residue of R₂ is optionally protected with conventional N-protecting groups) with an activating group such as halo. The thus activated 6-purine is subsequently reduced to purine, for instance with a palladium catalyst and deprotected to the desired compound of Formula IV or Formula V.

Compounds wherein R₃ is an R₁ or other ester may be prepared by conventional esterification (analogous to the esterification described above) of the corresponding hydroxy compound of Formula I or Formula V, optionally after conventional N-protecting the exposed amine function of the amino acid residue of R₂ and/or R₃. Compounds wherein R₃ is an ether may be prepared analogously to the process disclosed in the abovementioned WO 93 13778, again in conjunction with optional N-protection of exposed amine groups. Compounds wherein R₃ is an azide can be prepared as described in WO 97 09052.

Intermediates of the formula IId are conveniently prepared by acylation of a carboxy-protected hydroxy alkanoic acid, typically a 2-hydroxy-1-alkanoic acid, with the appropriate activated and N-protected R₂ derivative, such as N-CBZ valyl or isoleucyl in conjunction with a conventional coupling reagent such as DMAP/DCC or with the amino acid halide. The carboxy protecting group is then removed, for instance by acid hydrolysis and the resulting intermediate is activated as described above or the free acid is unsed in conjunction with a coupling reagent to esterify the the nucleoside under conventional esterification conditions.

Compounds of the formula Ia are also conveniently prepared by the methodology in the immediately preceding paragraph, namely esterification of a carboxy protected α- hydroxy, ω-carboxy acid, such as glycollic acid, lactic acid, hydroxybutyric acid etc with the appropriate N-protected R₂ derivative, either as the free acid in conjunction with a coupling agent or activated, for instance to the corresponding acid halide. The carboxy protecting group is removed and the resulting intermediate esterified with the nucleoside with the methodology described above.

Compounds comprising a structure of the formula He or IIf are prepared by carboxy protecting the terminal carboxy groups of the respective dicarboxylic acid, such as L-tartaric acid or L-malic acid, with conventional carboxy protecting groups such as benzoyl. The free hydroxy group (s) are then esterified with conventional esterification techniques, such as DMAP & DCC in DMF with the appropriate N-protected R2 amino acid, such as N-Boc-L-valyl or N-Boc-L-isoleucyl. The benzoyl carboxy protecting groups are removed and the resulting product is esterified to the 5'-hydroxy function of a monohydric nucleoside, using conventional conditions, such as those in the accompanying Examples. Finally, the See carboxy function is esterified with an R1 group or, more preferbably a conventional pharmaceutically acceptable ester, such as the ethyl ester.

Compounds comprising a phosphorylated moiety III may be prepared by reacting 2',3'-dideoxy-3'-fluoroguanine-5-monophosphate with a compound of Formula VIa 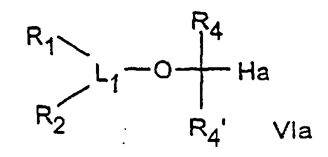 where Ha is halo, such as chloro, iodo or bromo, in analagous conditions to those described in US 4 337 201, US 5 227 506, WO 94/13682 & WO 94/13324, Starret et al J Med Chem 37 1857-1864 (1994) and Iyer et al Tetrahedron Lett 30 7141-7144 (1989). The monophosphate can be prepared by conventional phosphorylation of FLG, as described, for instance, in Herdwyn et al ibid. Corresponding methodology will apply to the monophosphates of other monohydric nucleosides.

Alternatively this esterification to the phosphate ester could take part in two steps comprising a first reaction between FLG-monophosphate and a compound of the formula VII 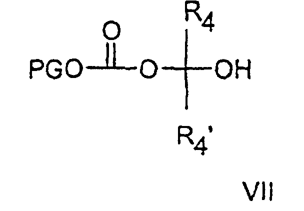 wherein R4 and R4' are as defined above and PG is a conventional hydroxy protecting group such as those described above, followed by deprotection and esterification to a linker group L1 whose third, rightmost function is an hydroxy group. Two examples of such a linker group L1 are depicted in Scheme 1 below (the penultimate compounds in each series). In this embodiment the leftmost carbonyl of formula Va is synonomous with the carbonyl of linker group of the linker of Formula IIa.

Compounds comprising an optional linker L₂ may also be prepared by a two stage process. In particular a compound of the formula ClC(=O)OC(R₄)(R₄')Cl can be reacted with the 5'-hydroxy of FLG (optionally protected on the base with conventional protecting groups) as is known in the cephalosporin art. The resulting FLG-5'-O-C(=O)OC(R₄)(R₄')chloride is then reacted with an R₁ and R₂ bearing trifunctional linker wherein the third function comprises a carboxyl function, such as the potassium salt.

It will be appreciated that trifunctional L₁ groups of formula IIa wherein n and m are 1 and Alk is absent can be prepared from glycerol by regioselective esterification as depicted below in scheme 1 by reference to a stearoyl/L-valyl combination. In short R₁ and R₂ are regioselectively esterified to positions 1 and 3 of the glycerol and position 2 is then converted to the appropriate-T-C(=O)- group, which is then esterified to the 5'-position of the fluoronucleoside or to a cooperating function on L₂ (not depicted). Alternatively the hydroxy at position 2 of the glycerol derivative can be esterified with an L₂ group containing a cooperating carbonyl function on its left hand end.

L₁ groups of formula IIa wherein m is 1, n is 0 and Alk is methylene can also be prepared from glycerol by regioselectively esterifying R₁ and R₂ to positions 1 and 2 of the glycerol, as also depicted below in scheme 1, followed by conversion of the hydroxy at position 3 to the appropriate -T-C(=O)-group. The leftmost series of reactions on Scheme 1 shows the situation where R₁ is esterified to position 1 of the glycerol and R₂ is esterified to position 2. The corresponding arrangement where R₁ is esterified to position 2 and R₂ to position 1 can be achieved by first treating the glycerol with CBz-L-valine/DCC/DMAPIDMF and then protecting the 3 position with pixyl chloride prior to esterifying the fatty acid of R₁ to position 2 of the glycerol, deprotecting and converting the 3 position as necessary.

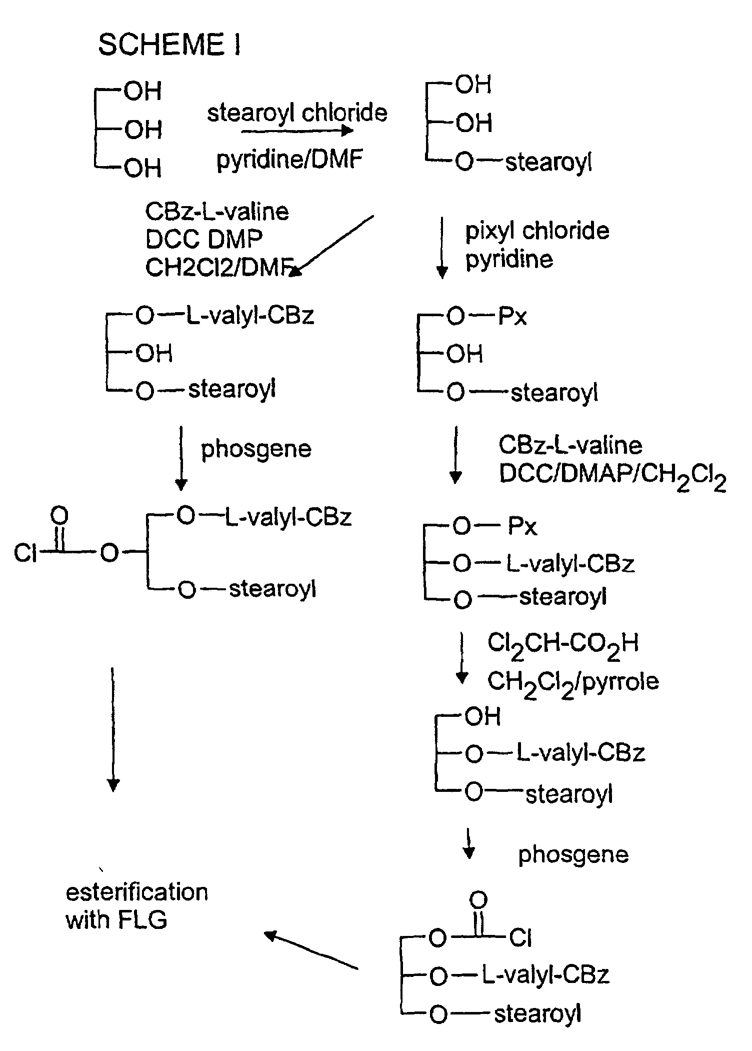

Although Scheme 1 has been illustrated by reference to a combination wherein R₁ is stearoyl and R₂ is L-valyl, it will be appreciated that this basic scheme will also be applicable to other amino acids, where present other fatty acids, or using conventional protection groups, to combinations of R₂ as an amino acid derivative and R₁ as hydroxy. Linkers where T comprises an -NH- group can be prepared by analogous regioselective esterification followed by conversion of the free hydroxyl to amine, reduction to azide and reaction with phosgene to form the corresponding chlorocarbamate.
A variation of scheme I allows the preparation of linkers of the formula IIc. In this variation, the phosgene step shown above is replaced by reaction with an activated dicarboxylic acid, such as succinic anhydride. This results in a glycerol triester (comprising the (optionally protected) R₁ ester, the protected R₂ ester and the ester of the dicarboxylic acid) and the free carboxy on the dicarboxylic acid is then activated and esterified to the nucleoside in a conventional fashion. Alternatively linkers of formula IIc can be built up in situ on the nucleoside. In this variant, the dicarboxylic acid is esterified to a suitably protected glycerol derivative. This succinyl monoester is then esterified to the 5'-hydroxy function of the nucleoside in a conventional manner. Finally one or both of the protecting groups on the glycerol moiety is replaced with the L-amino acid ester, and, if present, the remaining protecting group is replaced with a fatty acid ester or removed to leave a free hydroxy group This is depicted in Scheme IA which illustrates an example wherein the nucleoside is acyclovir (FLG shown in shadow), the dicarboxylic acid is succinyl and R₁ and R₂ are both CBZ-protected valyl, but will, of course be applicable to other variations of Formula Ic. In each case coupling conditions means standard esterification conditions such as coupling reagents DMAP, DCC etc or alternatively conversion of the relevant carboxy function to an activated derivative such as the acid chloride or the activated succinic moiety can also comprise the anhydride.

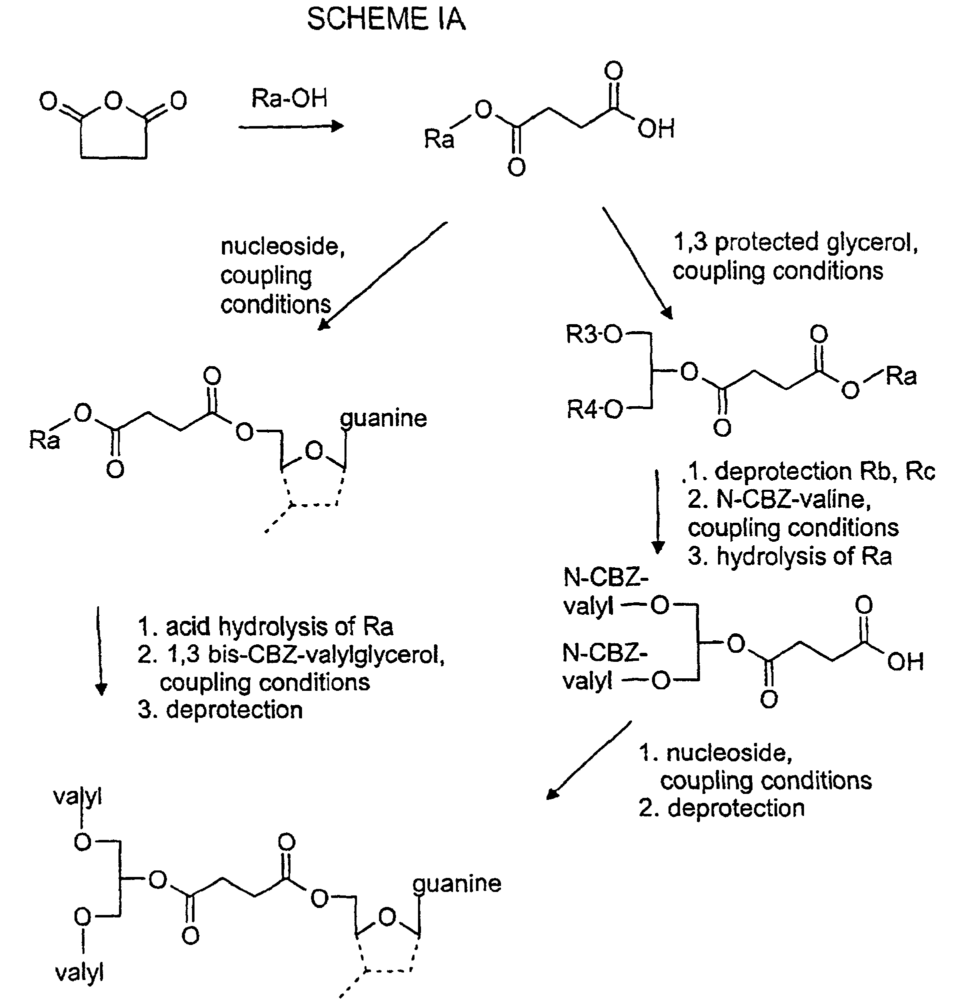

In a variation of Scheme IA, the succinic anhydride is reacted directly with the nucleoside, thus avoiding the first protection and deprotection steps. A further alternative is to regioselectively esterify the glycerol moiety with the N-protected amino acid moiety(ies), generally in conjunction with protection of the hydroxy function intended for coupling to the nucleoside, followed by deprotection of that hydroxy and coupling to the nucleoside. 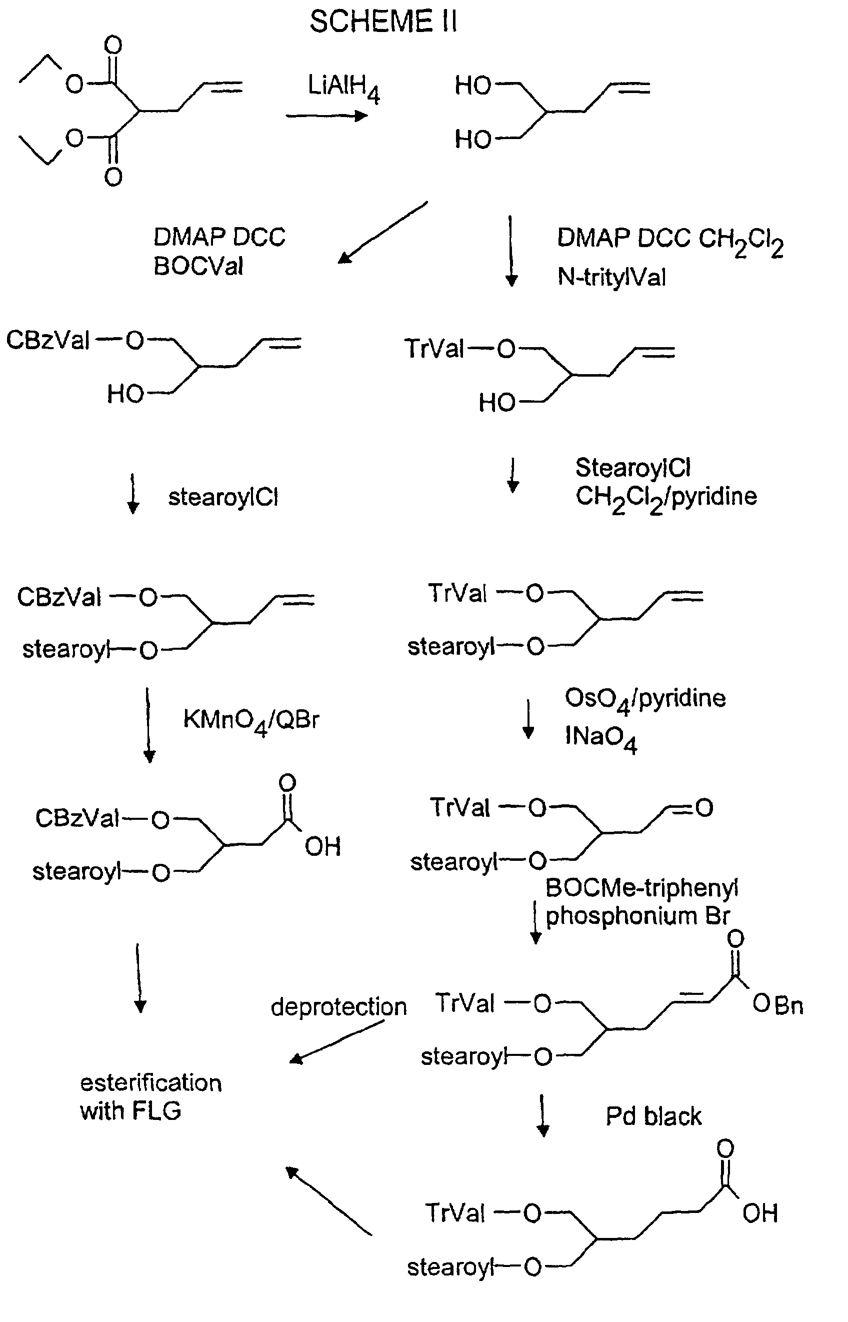 Linkers where m and n are 1, Alk is alkylene or alkenylene and T is a bond can be prepared as shown in Scheme II above. Other permutations of m, n, Alk and the various functions in the trifunctional linker group L₁ of formula IIa can be prepared analagously to the above with the corresponding starter materials, such as 1,2,4-trihydroxybutane (CA registry number 3968-00-6), 3,4-dihydroxybutanoic acid (1518-61-2 & 22329-74-4), (S)-3,4-dihydroxybutanoic acid (51267-44-8), (R)-3,4-dihydroxybutanoic acid (158800-76-1), 1,2,5-pentanetriol (51064-73-4 & 14697-46-2), (S)-1,2,5-pentanetriol (13942-73-9), (R)-1,2,5-pentanetriol (171335-70-9), 4,5-dihydroxypentanoic acid (66679-29-6 & 129725-14-0), 1,3,5-pentanetriol (4328-94-3) and 3-(2-hydroxyethyl)-1,5-pentanediol (53378-75-9). The preparation of each of these starting materials is described in the references to the respective registry number. Ohsawa et al in Chem Pharm Bull 41 (11) 1906-1909 (1993) and Terao et al Chem. Pharm. Bull. 39(3) 823-825 (1991) describe the control of the sterochemistry of trifunctional linker groups with lipase P.

The amino acid derivative of R₂ and, if present, R₁ can alternatively be esterified to the linker group with the 2-oxa-4-aza-cycloalkane-1,3-dione methodology described in international patent application no. WO 94/29311.

Linking of the carboxy function of R₁ and/or R₂ to an amine group on the linker derivative proceeds by conventional peptide chemistry, generally in conjunction with protection of the α-amine with conventional N-protecting groups. Formation of an amide bond between a carboxyl function on the linker and the α-amine group of R₂ also proceeds by conventional peptide chemistry, generally in conjunction with protection of the α-carboxy function. Esterification of R₁ as a fatty alcohol to a carboxy function on the linker proceeds analogously, but conversely, to the above esterifiation of R₁ as a fatty acid.

### Brief Description of the Drawings

Various aspects of the invention will now be described by way of example only with reference to the following Examples and the accompanying drawings in which
Figure 1 depicts serum viral-DNA levels in treated and untreated, DHBV-infected ducks as a function of time, as described in Biological Example 3;
Figure 2 depicts weight gain in treated, DHBV-infected ducks as a function of time, as described in Biological Example 3.

### EXAMPLE 1

### 2-(stearoyloxymethyl)-2-(N-(fluorenylmethoxycarbonyl)-L-valyloxymethyl)-propionic acid

To a solution of 2,2-bis(hydroxymethyl) propionic acid (28.16 g, 210 mmole) in water (50 ml), was added potassium hydroxide (11.78 g, 210 mmole). After 5 min, the solution was evaporated in vacuo and the residue was co-evaporated with dry DMF for three times. The residue was then dissolved in DMF (500 ml), and to the solution was added benzyl bromide (3.57 ml, 30 mmole). After stirring for 30 min, the reaction mixture was filtered through the Celite, poured into sodium hydrogen carbonate aqueous solution and extracted with dichloromethane. The organic phase was collected and then washed with sodium hydrogen carbonate aqueous solution. It was then evaporated in vacuo to give benzyl 2,2-bis(hydroxymethyl) propionate (4.37 g).
¹H-NMR (CDCl₃): 7.35 (s, 5H), 5.20 (d, 2H), 3.91-3.71 (m, 4H), 1.10 (s, 3H).

To a solution of benzyl 2,2-bis(hydroxymethyl) propionate (4.37 g, 19.5 mmole) in pyridine (58 ml) was added dropwise stearoyl chloride (4.13 g, 13.6 mmole) in dichloromethane over 40 min. The reaction was then kept for 16 hr and then poured into sodium hydrogen carbonate aqueous solution and extracted with dichloromethane. The organic phase was collected and evaporated in vacuo. The product benzyl-2-(hydroxymethyl)-2- (stearoyloxymethyl) propionate was isolated by silica gel column chromatography (1.97 g)
¹H-NMR (CDCl₃): 7.34 (s, 5H), 5.17 (d, 2H), 4.28 (dd, 2H) 3.69 (dd, 2H), 2.24 (t, 2H), 1.57 (m, 2H, 1.25 (s, 28H), 1.22 (s, 3H), 0.87 (t, 3H).

Benzyl-2-(hydroxymethyl)-2-(stearoyloxymethyl) propionate (1.86 g, 3.8 mmole) was dissolved in pyridine (30 ml). To the solution were added toluenesulfonic acid (73 mg, 0.39 mmole), N-fluorenylmethoxycarbonyl-L-valine (3.94 g, 11.6 mmole), and DCC (3.58 g, 17.4 mmole). The reaction was kept at 4 °C for 16 hr and then filtered through Celite. The filtrate was poured into sodium hydrogen carbonate aqueous solution and extracted with dichloromethane. The organic phase was collected and evaporated in vacuo. The product, benzyl-2-(N-(fluorenylmethoxycarbonyl)-L-valyloxymethyl)-2-(stearoyloxymethyl)propionate, was isolated by silica gel column chromatography. Yield: 2.38 g.
¹H-NMR (CDCl₃): 7.78-7.25 (m, 13H), 5.29 (m, 1H), 5.15 (d, 2H), 4.38 - 4.23 (m, 7H), 2.19 (t, 2H), 2.10 (m, 1H), 1.55 (m, 2H), 1.24 (m, 31H), 0.94 - 0.83 (m, 9H).

To the solution of benzyl 2-(N-(fluorenylmethoxycarbonyl)-L-valyloxymethyl )-2-(stearoyloxymethyl) propionate (1.86 g, 3.8 mmole) in a mixed solvent of THF/methanol (16ml/8ml) were added ammonium formate (376 mg, 6 mmole), formic acid (1.87 ml), and palladium black (40 mg). The reaction was kept at room temperature for 16 hr, and then filtered through Celite. After evaporation, the product was isolated by silica gel column chromatography. Yield: 1.05 g.

### EXAMPLE 2

### 1-O-stearoyl-2-O-(N-CBz-L-valyl)glycerol

### a) Preparation of 1-O-stearoylglycerol

To a mixture of glycerol (30 g, 326 mmol) and pyridine (25 ml) dissolved in DMF (300 ml) was added dropwise stearoyl chloride (10 g, 33 mmol) dissolved in DMF 100 ml). The mixture was cooled on an ice bath until addition was complete, whereupon the reaction was maintained under an N₂ atmosphere overnight. After 15 hours CH₂Cl₂ (300 ml) and saturated NaHCO₃ (aq) was added. The phases were separated and the organic phase washed with water (50 ml) and dried with Na₂ SO₄. The solvent and any pyridine were evaporated under vacuum. The crude product was chromatographed on a silica column (CH₂Cl₂ -MeOH, 20:1) and recrystallised (CH₂Cl₂-ether) to yield around 7 grams.

### b) Preparation of pixyl chloride

Acetyl chloride (150 ml, 2.1 mol) is added to a magnetically stirred suspension of 9-hydroxy-9-phenylxanthene (20 g 72 mmol) in benzene (100 ml). An homogenous deep red solution is obtained. The solution is stirred for 30 min. at 20 °C. The volatiles are removed under reduced pressure. Excess AcCl is neutralised by careful addition to ethanol. The residue is co-evaporated with toluene (2 x 30 ml) and with cyclohexane (2 x 30 ml) to obtain a crystalline residue which is stored airtight.Pixyl chloride is alternatively available from Aldrich.

### c) Preparation of 1-O-stearoyl-3-O-pixylglycerol

The product from a) above (2.28 g) and pyridine (25 ml) were mixed and heated until dissolved. After cooling in an icebath pixyl chloride (1.92 g) from step b) was added. The mixture was maintained under agitation and an argon atmoshere in an icebath for half an hour and then at room temperature for 1.5 h. The pyridine was evaporated under vacuum, the residue dissolved in CH₂Cl₂ (70 ml) and washed with 0.5 M citricacid to remove remaining pyridine. The residue was dried with Na₂SO₄, evaporated and chromatographed (ether- hexane 1:3) to give 1.25 g pure product with a TLC R_{f} around 0.2.

### d) Preparation of 1-O-stearoyl-2-O-(N-CBz-L-valyl), 3-O-pixylglycerol

The product of step c) (237 mg, 0.39 mmol), CBz-L-valine (116 mg, 0.46 mmol), DCC (96 mg, 0.46 mmol) and DMAP (4.7 mg, 0.04 mmol) were dissolved in CH₂Cl₂ (4 ml). The mixture was maintained under agitation in a nitrogen atmosphere overnight. After 18 hours the mixture was filtered through a glass filter and chromatographed on a silica gel column (ether - hexane 1:4) to yield 230 mg with a TLC R_{f} of 0.2

### e) Preparation of 1-O-stearoyl-2-O-(N-CBz-L-valyl)glycerol

The pixyl group in the product of step d) was removed by selective deprotection by the method described in Example 3, step d to yield the title compound.
¹H-NMR (CDCl3): δ 7.35 (m, 5H), 5.3-4.9 (m, 4H), 4.35-4.25 (m, 3H), 3.8-3.6 (m, 2H), 2.31-2.25 (m, 2H), 2.20-2.10 (m, 1H), 1.60 (m, 2H), 1.02-0.86 (m, 9H).

### EXAMPLE 3

### 1-O-(N-CBz-L-valyl)-2-O-stearoylglycerol

### a) Preparation of 1-O-(N-CBz-L-valyl)glycerol

CBz-L-valine (4.35 g, 17.3 mmol) was added to a fivefold excess of glycerol (8 ml, 86.9 mmol) togehter with dicyclohexylcarbodiimide (4.29 g 20.8 mmol) and 4-dimethylaminopyridine (0.212 g) at room temperature. After stirring overnight the suspension was filtered and DMF removed in vacuo from the filtrate. The residue was redissolved in CH₂Cl₂, washed successively with saturated NaHCO₃, brine, and water and then dried. The crude material was chromatographed on silica gel with 4/1 EtOAc - hexane as eluent to yield 2.465 g. R_{f} (4/1 EtOAc - hexane) 0.17, (20/1 CH₂Cl₂ - methanol) 0.12.

### b) Preparation of 1-O-(N-CBz-L-valyl)-3-O-pixylglyerol

The product of step a) (0.672 g, 20.1 mmol) was dissolved in dry pyridine (3.5 ml) under nitrogen. 9-Chloro-9-phenylxanthene (pixyl chloride, 0.65 g, 22.0 mmol, 1.1 eq - prepared as above) was added and the mixture stirred at room temperature for 1.5 h. MeOH (1.5 ml) was added and the mixture partitioned between 10 ml Et₂O and 10 ml saturated NaHCO₃. The aqueous layer was extracted with more ether. The organic layers were combined, dried and concentrated several times with toluene to give a white solid. The crude material was chromatographed on silica gel with 3/1 hexane - EtOAc as eluent to give 0.681 g.

Alternatively a pixyl group can be put on by the procedure described by Gaffney et al, Tetrahedron Lett 1997, 38, 2539-2542 using PxOH and acetic acid.

### c) Preparation of 1-O-(N-CBz-L-valyl)-2-O-stearoyl-3-O-pixyl glycerol

Stearoyl chloride (496 ml, 1.3 eq) in 1.5 ml CH₂Cl₂ was added dropwise to a solution of the product of step b) (0.658 g, 1.13 mmol) in 11 ml pyridine with stirring under N₂ in an ice bath. After 15 minutes the mixture was stirred at room temperature overnight. The mixture was diluted with 20 ml Et₂O and washed with 10 ml saturated NaHCO₃. The aquesous layer was extracted with more Et₂O. The organic layers were combined, washed with brine (20 ml), dried over Na₂SO₄ and concentrated several times with toluene. The crude material (1.37 g) was chromatographed on 130 g silica gel with 6/1 hexane - EtOAc. An initial fraction of 500 ml was taken followed by 100 ml fractions. The desired material eluted in fractions 2 - 5 yielding 0.748 g.

### d) Preparation of 1-O-(N-CBz-L-valyl)-2-O-stearoylglycerol

To a solution of the product of step c) (0.748 g, .872 mmol) dissolved in 35 ml CH₂Cl₂ to make 0.025 M) was added pyrrole (16.5 mol eq) and dichloroacetic acid (5.5 mol eq) at room temperature. TLC after 5 minutes showed complete reaction. The mixture was diluted with 300 ml CH₂Cl₂ and washed with 30 ml saturated NaHCO₃. The aqueous layer was extracted with more CH₂Cl₂. The organic phases were combined, washed with brine (30 ml), dried over Na₂SO₄ and concentrated. Crude material was chromatographed on silica gel with 2/1 hexane - EtOAc (with 0.3% acetic acid) as eluent to yield 0.363 g with R_{f} (2/1 hexane - EtOAc) 0.21.
¹H NMR (CDCl₃) δ ppm 0.86-0.99 (m, 9H), 1.25 (s, 28H), 1.61 (m, 2H), 2.16 (m, 1H), 2.32 (m, 2H), 3.74 (br s, 2H), 4.28-4.44 (m, 3H), 5.09 (m, 1H), 5.11 (s, 2H), 5.22 (d, 1H), 7.36 (m, 5H)

### EXAMPLE 4

### 1-O-stearoyl-3-O-(NCBz-L-valyl)glycerol

The product of Example 2, part a) (2.86 g, 7.99 mmol), DCC (0.9g, 4.36 mmol), 4-(N,N-dimethyl)aminopyridine (DMAP) (0.048 mg, 0.39 mmol) and N-CBz-L-valine (1g, 3.98 mmol) were dissolved in CH₂Cl₂ (60 ml) and DMF (6 ml). The reaction was left at ambient temperature for 18 hours and then filtrated. The solvent was evaporated under reduced pressure. The residue was dissolved in CH₂Cl₂ (100 ml) and filtrated. The crude title compound was purified by chromatography [SiO₂, ether/hexane (1:2)] to yield 1.3 g of the desired product. Unreacted 1-stearoylglycerol may be recovered by eluting with CH₂Cl₂/MeOH (20:1).
¹H-NMR (CDCl₃): δ 5.25 (d, 1H), 5.11 (s, 2H), 4.30-4.05 (m, 6H), 2.65 (d, 1H), 2.35 (t, 2H), 2.06 (m, 1H), 1.62 (m, 2H), 1.26 (s, 28H), 1.00-0.84 (m, 9H).

### EXAMPLE 5

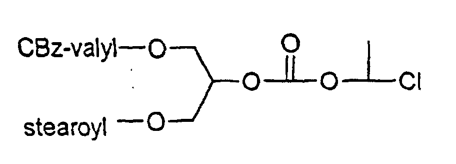

To an ice cooled solution of 1-chloroethyl chloroforrnate (1.89 g, 13.2 mmol) in dry CH₂Cl₂ (5 ml), was added the compound of Example 4 in CH₂Cl₂ (20 ml) followed by dry pyridine (1.2 ml, 29.6 mmol). The reaction mixture was stirred with cooling under argon atmosphere until TLC (ether/hexane, 1:2) indicated consumption of the starting material. After 1.5 h, the mixture was washed with water (3 x 5 ml), sat. NaHCO₃ (5 ml) and dried (Na₂SO₄). Purification by chromatography [SiO₂ (ether-hexane (1:2)] yielded the title compound (4.0 g).
¹H-NMR (CDCl₃): δ 7.36-7.32 (m, 5H), 6.40 (m, 1H), 5.24 (m, 1H), 5.11 (s, 2H), 4.30 (m, 6H), 2.32 (m, 2H), 2.15 (m, 1H), 1.82 (m, 3H), 1.60 (m, 2H), 1.25 (br s, 28H), 0.97 (m, 3H), 0.86 (m, 6H).

### EXAMPLE 6

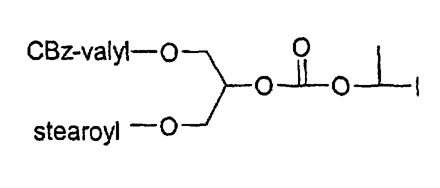

To a solution of the compound of Example 5 (3.4 g, 4,87 mmol) in dry acetonitrile (47 ml), was added sodium iodide (3.65 g, 24.3 mmol). The solution obtained was refluxed under argon atmosphere until NMR indicated consumption of the starting material. After 4.5 h, ether (50 ml) was added and the mixture was filtrated. The solvent was removed by evaporation and the crude product dissolved in ether (50 ml). The ether solution was washed with water (2 x 10 ml) and dried (Na₂SO₄) and evaporated under reduced pressure. Purification by chromatography [SiO₂, ether-hexane (1:2)] yielded the title compound (2.15 g).
¹H-NMR (CDCl₃): 67.37 (m, 5H), 6.75 (m, 1H), 5.22 (m, 1H), 5.15 (s, 1H), 4.3 (m, 6H), 2.32 (m, 1H), 2.22 (m, 2H), 1.6 (m, 2H), 1.25 (s, 28H), 0.95 (m, 9H).

### EXAMPLE 7

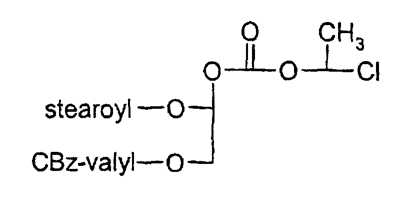

A solution of the compound of Example 3 (810 mg, 1.37 mmol) in 2.2 mL dry dichloromethane was cooled in an ice bath with stirring under argon. 1-Chloroethyl chloroformate (298 µL, 2.74 mmol) was added, followed by the dropwise addition of pyridine (665 µL, 8.22 mmol) in 2.5 mL dichloromethane. After 2.5 hr, the mixture was diluted with 25 mL dichloromethane and washed successively with 10 mL water and 10 mL brine. The organic phase was dried over anhydrous sodium sulfate and concentrated several times with toluene to give a yellow oil. Purification by flash column chromatography on silica gel with 40/1 dichloromethane-diethyl ether gave the title compound as an oil (96 mg, quantitative yield).
¹H NMR (CDCl₃) δ ppm 0.85-0.98 (m, 9H), 1.25 (s, 28H), 1.60 (m, 2H), 1.83 (d, 3H, J= 5.8 Hz), 2.17 (m, 1H), 2.31 (t, 2H), 4.19-4.48 (m, 5H), 5.11 (s, 2H), 5.22 (d, 1H), 5.27 (m, 1H), 6.38-6.43 (m, 1H), 7.36 (m, 5H).

### EXAMPLE 8

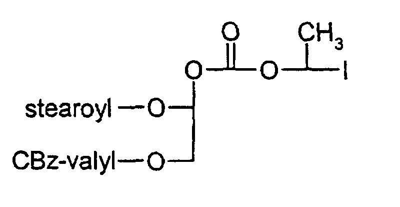

A solution of the compound of Example 7 (1.896 g, 2.71 mmol) and sodium iodide (1.80 g, 12.0 mmol) in acetonitrile (27 mL) was refluxed at 80 °C under nitrogen. After 4.5 hours the reaction mixture was diluted with 100 mL 1/1 hexane-diethyl ether and washed with 25 mL water. The aqueous phase was extracted with more solvent (25 mL). The organic phases were combined, washed successively with 5% aqueous sodium thiosulfate solution (25 mL) and brine (25 mL), dried over anhydrous sodium sulfate, and concentrated *in vacuo*. Purification by flash column chromatography on silica gel with 80/1 dichloromethane-methanol as eluant gave an oil (1.45 g) containing 90% of the title compound with 10% of the compound of Example 7.
¹H NMR (CDCl₃) δ ppm 0.85-0.99 (m, 9H), 1.25 (s, 28H), 1.60 (m, 2H), 2.17 (m, 1H), 2.23 (d, 3H, J= 6 Hz), 2.31 (t, 2H), 4.16-4.49 (m, 5H), 5.10 (s, 2H), 5.20-5.29 (m, 2H), 6.69-6.79 (m, 1H), 7.36 (m, 5H).

### EXAMPLE 9

### 4-Benzyloxy-2-(N-trityl-L-valyloxymethyl)-1-stearoyloxybutane

### a) Synthesis of diethyl-2-(2-benzyloxyethyl) malonate

To a freshly prepared solution of sodium (0.95g, 41.4 mmole) in 50 ml ethanol was added a solution of diethylmalonate (6.4g, 40 mmole) in 10 ml ethanol and the mixture was stirred for 15 minutes.Then a solution of 2-benzyloxy-1-iodoethane (11.5g, 41,35 mmole) was added drppwise. The mixture was refluxed for four hours and than evaporated in vacuo. 100ml of water was added and the mixture was extracted three times with 50ml portions of diethylether. The organic phase was dried with sodium sulfate and evaporated in vacuo and the product was isolated by silica gel column chromatography. Yield: 8.6g
¹H-NMR (CDCl₃) 1.26 (m, 6H) 2.26 (m , 2H) 3.54 (m, 3H) 4.16 (m, 4H) 4.57 (s, 2H) 7.32 (m, 5H)

### b) Synthesis of 4-benzyloxy-2-hydroxymethyl-butanol-1.

To a stirred suspension of lithium aluminium hydride (3.0g, 80 mmol) in 100 ml diethylether was added dropwise a solution of diethyl-2-(2-benzyloxyethyl) malonate (8.5g, 28.8mmol) in 20 ml diethylether at about 15°C .The mixture was refluxed for two hours. About 4 ml water was dropwise added while cooling. The mixture was filtered and washed with dioxane. The filtrate was evaporated under reduced pressure and the product was isolated by silica gel column chromatography.
Yield: 3.4g
¹H-NMR (CDCl₃) 1.60 (m, 2H) 1.82 (m, 1H) 3.00 (m, 2H) 3.56 (t, 2H) 3.69 (m, 4H) 4.50 (s, 2H) 7.32 (m, 5H)

### c) Synthesis of 4-benzyloxy-2-(N-trityl-L-valyloxymethyl)-butanol-1

To a solution of N-trityl-L-valine (4.66g, 13 mmol) and 4-benzyloxy-2-hydroxymethyl-butanol-1 (3.3g, 15.6 mmole) in 50 ml dichloromethane was added DCC (3.0g, 14.5mmole) and DMAP (0.18g, 1.45mmole) and the mixture was stirred for three days. The mixture was cooled to 5°C and the urethane was filtered. The solution was evaporated under reduced pressure and the product was isolated by silica gel column chromatography .
Yield: 2.5g
¹H-NMR (CDCl₃) 1.00 (m, 6H) 1.55 (m, 4H) 1.72 (m, 1H) 2.18 (m, 1H) 2.70 (m, 1H) 3.27 (m, 2H) 3.43 (m, 3H) 4.50 (s, 2H) 7.26 (m, 20H)

### d) Synthesis of 4-benzyloxy-2-(N-trityl-L-valyloxymethyl)-1-stearoyloxybutane .

To a solution of 4-benzyloxy-2-(N-trityl-1-valyloxymethyl)-butanol-1 (2,4g, 4.35 mmol) in 50 ml dichloromethane was added pyridine (1.72g, 21.7 mmol). The solution was cooled to 10°C and a solution of stearoyl chloride (2.64g, 8.7 mmol) in 10ml dichloromethane was added dropwise between 10°C and 15°C . The mixture was stirred overnight at room temperature. 100 ml of 5% sodium hydrogen carbonate solution was added and the mixture stirred for 30 minutes. The organic phase
was seperated and the water phase was extracted two times with dichloromethane. The combined organic phases were dried with sodium sulfate and concentrated in vacuo. The product was isolated by silica gel column chromatography.
Yield: 3.0g
¹H-NMR (CDCl₃) 0.98 (m, 9H) 1.26 (m, 28H) 1.54 (m, 2H) 1.94 (m, 1H) 2.25 (m, 2H) 3.23 (m, 2H) 3.44 (m, 2H) 3.58 (m, 1H) 3.91 (m, 2H) 4.10 (m, 1H) 4.47 (s, 2H) 7.28 (m, 20H)

### EXAMPLE 10

### 5-( N-trityl-L-valyloxymethyl)-6-stearoyloxyhexanoic acid

### a) Preparation of 2-allyl 1,3-propanediol

Diethyl allylmalonate (20 ml, 101 mmol) in anhydrous ether (100 ml) was added dropwise to a stirred solution of lithium aluminium hydride (9.6 g, 253 mmol) at 0°C. The reaction was warmed up to room temperature and kept for 5 hours. It was cooled down to 0 °C and water (12 ml) was carefully added dropwise. After stirring for 30 min, the mixture was filtered through Celite and then washed with ethanol (2 x 500 ml). The solution was dried under vacuum giving 9.5 g product
¹H-NMR (CDCl₃): 5.78 m, 1H), 5.03 (m, 2H), 3.78 (m, 2H), 3.69 (m, 2H), 2.06 (t, 2H), 1.87 (m, 1H).

### b) Preparation of 1-O-(N-trityl-L-valyl )-2-allyl-1,3-propandiol

To a solution of N-trityl-L-valine (5.5 g, 15.2 mmole), 2-allyl-1,3-propandiol (4.4 g, 38 mmol), N,N-dimethylamino pyridine (183 mg, 1.5 mmol) in dichloromethane (120 ml) was added DCC (3.5 g, 16.7 mmol). The reaction was kept under reflux overnight. After filtration through Celite, the organic phase was washed with sodium hydrogen carbonate aqueous solution and dried . Silica gel column chromatography gave 4.6 g intermediate 1-O-(N-trityl-L-valyl )-2-allyl-1,3-propandiol.

### c) Preparation of 1-O-(N-trityl-L-valyl)-2-allyl-3-stearoyl-1,3-propandiol.

To a solution of 1-O-(N-trityl-L-valyl)-2-allyl-1,3-propandiol (1.83 g, 4 mmol) in dichloromethane (40 ml) and pyridine (3.2 ml, 40 mmol) at 0 °C was added dropwise stearoyl chloride (3.62 g, 12 mmol) in dichloromethane. The solution was warmed up to room temperature, and kept for 3 hr. It was then washed with sodium hydrogen carbonate aqueous solution and dried. The product was isolated by silica gel column chromatography. 1.9 g
¹H-NMR (CDCl₃): 7.30 (m, 15 H), 5.70 (m, 1H), 4.99 (m, 2H), 3.93 (m, 2H), 3.55 (m, 1H), 3.27 (m, 2H), 2.68 (m, 1H), 2.30 (m, 2H), 2.23 (m, 1H), 2.01 (m, 2H), 1.85 (m, 1H), 1.62 (m, 2H), 1.3 (m, 28H), 0.98 (dd, 6H), 0.91 (t, 3H).

### d) Preparation of 3-(N-trityl-L-valyloxymethyl)-4-stearoyloxy-butyraldehyde

1-O-(N-trityl-L-valyl)-2-allyl-3-stearoyl-l,3-propandiol (580 mg, 0.8 mmol) was dissolved in dioxane (5 ml). To the solution were added osmium tetraoxide (20 mg, 0.08 mmole) and pyridine (0.05 ml, 0.64 mmole). A solution of sodium periodate in water (3.5 ml) was added to the reaction mixture. The reaction was kept overnight and then cooled down to 0 °C. An aqueous solution of sodium hydrogen sulfite was added and the mixture was extracted with dichloromethane. The organic phase was dried and purified by silica gel column chromatography. Yield. 250 mg
¹H-NMR (CDCl₃): 9.68 (s, 1H), 7.25 (m, 15 H), 3.92 (m, 2H), 3.58 (m, 1H), 2.32 (m, 2H), 2.68 (m, 1H), 2.34 (m, 7 H), 1.58 (m, 2H), 1.53 (m, 28 H), 0.96 (dd, 6H), 0.86 (t, 3H).

### f) Preparation of benzyl 3-(N-trityl-L-valyloxymethyl)-4-stearoyloxyhexen-2-oate

To the solution of 3-( N-trityl-L-valyloxymethyl)-4-stearoyloxy-butyraldehyde (15.8 g, 21.8 mmole) in dichloromethane were added (benzyloxycarbonylmethyl) triphenylphosphonium bromide (10.7 g, 21.8 mmole) and triethylamine (2.21 g, 21.8 mmole). The reaction was kept overnight at room temperature, and the mixture was evaporated. To the residue was added diethyl ether (200 ml)and kept at 4 °C for two hours. It was then filtered and the filtrate was evaporated and the product was purified by silica gel column chromatography. Yield. 10 g
¹H-NMR (CDCl₃): 7.30 (m, 20 H), 6.89 (m, 1H), 5.88 (d, 1H), 5.19 (d, 2H), 3.95 (m, 2H), 3.57 (m, 1H), 3.29 (, 2H), 2.68 (m, 1H), 2.23 (m, 5H), 1.93 (m, 1H), 1.60 (m, 2H), 1.32 (m, 28 H), 0.95 (dd, 6H), 0.89 (t, 3 H).

### g) Preparation of 3-(N-trityl-L-valyloxymethyl)-4-stearoyloxyhexanoate

To a solution of benzyl 3-(N-trityl-L-valyloxymethyl)-4-stearoyloxyhexen-2-oate (70 mg, 0.08 mmole) in methanol (3 ml) and ethyl acetate (1 ml) was added sodium hydrogen carbonate (10 mg) and palladium black (20 mg). The reaction was kept under hydrogen at atmospheric pressure for 2 hr. The mixture was filtered and evaporated. The residue was dissolved in dichloromethane and washed successively with aqueous EDTA solution and cold aqueous 2 % citric solution. The organic phase was evaporated to give 61 mg product
¹H-NMR (CDCl₃): 7.30 (m, 15 H), 3.93 (m, 2H), 3.57 (m, 1H), 3.25 (m, 2H), 2.30 (dt, 4H), 2.20 (m, 1H), 1.70 (m, 1H), 1.62 (m, 4H), 1.30 (m, 28 H), 0.95 (dd, 6 H), 0.87 (t, 3 H).

### EXAMPLE 11

### 3-(N-benzyloxycarbonyl-L-valyloxymethvl)-4-stearoyloxy-butyric acid

### a) Preparation of 1-O-(N-benzyloxycarbonyl-L-valyl)-2-allylyl-1,3-propandiol

To a solution of 2-allyl-1,3-propandiol (4.6 g, 40 mmole) and N-benzyloxycarbonyl valine (5.02 g, 20 mmole) in dichloromethane was added dimethylaminopyridine (244 mg, 2 mmol), and DCC (4.5 g, 22 mmol). After two hours, the mixture was filtered through Celite, evaporated and the product, 1-O-(N-benzyloxycarbonyl-L-valyl)-2- allylyl-1,3-propandiol, isolated to yield 5.01 g.
¹H-NMR (CDCl₃): 7.36 (m, 5H), 5.78 (m, 1H), 5.26 (d, 1H), 5.11 (s, 2H), 5.06 (d, 2H), 4.22 (m, 3H), 3.59 (m, 2H), 2.13 (m, 3H), 1.98 (m, 2H), 0.94 (dd, 6 H).

### b) Preparation of 1-O-(N-benzyloxycarbonyl-L-valyl)-2-allylyl-3-O-stearoyl-1,3-propandiol.

To a solution of 1-O-(N-benzyloxycarbonyl-L-valyl)-2-allylyl-1,3-propandiol (4.46 g, 12.7 mmol) in dichloromethane (70 ml) and pyridine (6.1 ml, 76 mmole) in ice bath was added stearoyl chloride (7.8 g, 26 mmole). The reaction mixture was warmed up to room temperate and kept for one hour. It was then poured into aqueous sodium hydrogen carbonate solution, the organic phase was dried and the product 1-O-(N-benzyloxycarbonyl-L-valyl)-2-allylyl-3-O-stearoyl-1,3-propandiol was purified by silica gel column chromatography. 6.7 g
1H-NMR (CDCl₃): 7.34 (m, 5H), 5.77 (m, 1H), 5.30 (d, 1H), 5.11 (s, 2H), 5.08 (d, 2H), 4.32 (m, 1H), 4.10 (m, 4 H), 2.29 (t, 2H), 2.13 (m, 4H), 1.62 (m, 3 H), 1.25 (m, 28H), 0.90 (m, 9 H).

### c) Preparation of 3-(N-benzyloxycarbonyl-L-valyloxymethyl)-4-stearoyloxy-butyric acid.

Potassium permanganate (756 mg, 4.8 mmole) was dissolved in water (7.5 ml). The solution was kept under strong stirring for 10 min. A solution of 1-O-(N-benzyloxycarbonyl-L-valyl)-2-allylyl-3-O-stearoyl-1,3-propandiol (1 g, 1.6 mmol) and tetrabutylammonium bromide (77 mg, 0.24 mmole) in benzene (5 ml) was added. The slurry was stirred for 1.5 hr, and dichloromethane was added. A sodium bisulfite aqueous solution was added to the slurry until the mixture discolored. The organic phase was acidified with acetic acid and washed with water. After evaporation, the product 3 -(N-benzyloxycarbonyl-L-valyloxymethyl)-4-stearoyloxy-butyric acid (390 mg) was isolated by silica gel column chromatography.
¹H-NMR (CDCl₃): 7.33 (m, 5H), 5.38 (d, 1H), 5.11 (s, 2H), 4.14 (m, 5 H); 2.60 (m, 1H), 2.45 (m, 2 H), 2.29 (t, 2 H), 2.18 (m, 1 H), 1.58 (m, 2 H), 1.25 (m, 28 H), 0.90 (m, 9 H).

### EXAMPLE 12

### 2',3'-dideoxy-3'-fluoro-5'-O-[5-(L-valyloxymethyl)-6-stearoyloxyhexanoyl] guanosine

### a) Preparation of 2',3'-dideoxy-3'-fluoro-5'-O-[5-(N-trityl-L-valyloxymethyl)-6-stearoyloxyhexanoyl] guanosine

To a solution of 5-( N-trityl-L-valyloxymethyl)-6-stearoyloxyhexanoic acid (462 mg, 0.6 mmole) and 2',3'-dideoxy-3'-fluoroguanosine (340 mg, 1.25 mmol) in DMF (3 ml) were added dimethylaminopyridine (7 mg, 0.06 mmole), and DCC (136 mg, 0.66 mmol). The reaction was kept at room temperature overnight, and then at 40°C for two hours. The reaction mixture was filtered through Celite and poured into dichloromethane, and washed with aqueous sodium hydrogen carbonate solution. The product 2',3'-dideoxy-3'-fluoro-5'-O-[5-(N-trityl-L-valyloxymethyl)-6-stearoyloxyhexanoyl] guanosine was isolated by silica gel column chromatography. (93 mg)
¹H-NMR (DMSO δ-6): 7.88 (s, 1H), 7.29 (m, 15 H), 6.52 (s, 2H), 6.17 (dd, 1H), 5.45 (m, 1H), 4.35 (m, 1H), 4.20 (m, 2 H), 3.82 (m, 2H), 3.50 - 2.60 (m, 5 H), 2.30 (m, 4 H), 2.10 (m, 1 H), 1.70 (m, 1H), 1.50 (m, 4 H), 1.22 (m, 28 H), 0.85 (m, 9 H).

### b) Preparation of 2',3'-dideoxy-3'-fluoro-5'-O-[5-(L-valyloxymethyl)-6-stearoyloxyhexanoyl] guanosine

The compound of step b) (90 mg, 0.088 mmole) was N-deprotected by treatment with 80 % acetic acid (5 ml) at room temperature for 30 min. It was evaporated and product was purified by silica gel column chromatography to yield 72 mg of the title compound.
¹H-NMR (DMSO δ-6): 7.88 (s, 1H), 6.54 (s, 2H), 6.18 (dd, 1H), 5.48 (dd, 1H), 4.27 (dt, 1 H), 4.19 (m, 2 H), 3.98 (m, 4H), 3.17 - 2.55 (m, 4 H), 2.29 (m, 4 H), 1.95 (m, 1 H), 1.75 (m, 1 H), 1.50 (m, 4H), 1.21 (m, 28 H), 0.84 (m, 9 H).

### EXAMPLE 13

### 2',3'-Dideoxy-3'-fluoro-5'-O-[3-(L-valyloxymethyl)-4-stearoyloxy-butanoyl]guanosine

### a) Preparation of 2', 3'-dideoxy-3'-fluoro-5'-O-[3-(N-benzyloxycarbonyl-L-valyloxy)-4-stearoyloxy-butanoyl]guanosine

To a solution of 2',3'-dideoxy-3'-fluoroguanosine (113 mg, 0.42 mmol) and 3-(N-benzyloxycarbonyl-L-valyloxymethyl)-4-stearoyloxy-butyric acid (140 mg, 0.21mmol) in DMF (2 ml) were added dimethylaminopyridine (3 mg, 0.02 mmol) and DCC (52 mg, 0.25 mmol). After two days, dichloromethane (10 ml) and a few drops of acetic acid were added and the organic phase was filtered through Celite. The filtrate was washed with aqueous sodium hydrogen carbonate solution and the product 2',3'-dideoxy-3'-fluoro-5'-O-[3-(N-benzyloxycarbonyl-L-valyloxymethyl)-4-stearoyloxy-butanoyl]guanosine was isolated by silica gel column chromatography to yield 51 mg.
¹H-NMR (CDCl₃): 7.79 (d, 1H), 7.26 (m, 5 H), 6.38 (s, 2H), 6.23 (t, 1H), 5.44 (m, 2H), 5.08 (s, 2H), 4.50-4.10 (m, 8H), 3.15-2.40 (m, 5 H), 2.30 (t, 2 H), 2.14 (m, 1H), 1.58 (m, 2H), 1.24 (m, 28H), 0.87 (m, 9 H).

### b) Preparation of 2',3'-Dideoxy-3'-fluoro-5'-O-[3-(L-valyloxymethyl)-4-stearoyloxy-butanoyl]guanosine

The product of step a) (76 mg, 0.084 mmole) was dissolved in a mixed solvent of methanol (3 ml), ethyl acetate (0.5 ml) and acetic acid (0.01 ml). To the solution was added palladium black (10 mg). After 2 hr, additional 10 mg palladium black was added. After 3 hr, the mixture was filtered and evaporated. The residue was dissolved in dichlorometahne and washed with aqueous EDTA solution. The organic phase was dried and co-evaporated with toluene giving the title compound as the acetate salt. Yield 65 mg.
¹H-NMR (DMSO δ-6 + D₂O): 7.87 (s, 1H), 5.16 (dd, 1H), 5.37 (dd, 1H), 4.24 (m, 3H), 4.01 (m, 4H), 3.10-2.60 (m, 3H), 2.40 (m, 2H), 2.24 (t, 2 H), 1.70 (m, 1H), 1.48 (m, 2H), 1.25 (m, 28H), 0.82 (m, 9H).

### EXAMPLE 14

### 3-[1-(N-CBz-L-valyl)-2-stearoyl] propyl chloroformate

1-(N-CBz-L-valyl)-2-stearoyl) glycerol ( 300 mg, 0.5 mmole ) was dissolved in 20 % phosgene in toluene (15 ml). After 18 h, the solution was evaporated and the residue was co-evaporated with toluene for several time, giving title product in quantitative yield. This product forms a carbonate with the target nucleoside using standard methodology, for instance reacting in a 10:1 DMF/pyridine solution at 0°C for 3 to 24 hours, pouring into NaHCO₃ solution and extraction with dichloromethane. The amino acid is deprotected, for instance with palladium black in a methanol, ethyl acetate, acetic acid solution to yield the nucleoside-O-[1-(L-valyl)-2-stearoyl-3-propyloxy carbonyl]
¹H-NMR ( CDCl₃): 7.40 (m, 5H), 5.28 (m, 2H), 5.10 (s, 2H), 4.35 (m, 5H), 2.35 (m, 2H), 2.17 (m, 1H), 1.56 (m, 2H), 1.30 (m, 28 H), 0.95 (m, 9H).

### EXAMPLE 15

### 5-(N-FMOC-L-valyloxy)-4-stearoyloxy-pentanoic acid

### a) Benzyl 4,5-dihydroxy-2-pentenoate.

A mixture of DL-glycerinaldehyde (4,5g, 50 mmole) and (benzyloxycarbonylmethyl)-triphenylphosphoniumbromide (24.57g, 50 mmole) in 100 ml 1,2-epoxybutane was refluxed overnight. The mixture was evaporated under vacuum and the product was isolated by silica gel column chromatography .
Yield : 8g = 71 %
¹H - NMR (CDCl₃) 2.50 (s, 1H) 2.96 (s, 1H) 3.54 (m, 1H) 3.70 (m, 1H) 4.38 (m, 1H) 5.12 (s, 2H) 6.14 (m,1H) 6.90 (m, 1H) 7.30 (m, 5H)

### b) Benzyl 5-(N-FMOC-L-valyloxy)-4-hydroxy-2-pentenoate.

A mixture of benzyl 4,5-dihydroxy-2-pentenoate (4.4g, 20 mmole), N-FMOC-L-valine (5.8g, 17 mmole) and DMAP (0.21g, 1,7 mmole) in 100 ml dichloromethane was cooled to about 10°C . A solution of DCC (4.2g, 20 mmole) in 25 ml dichloromethane was added droppwise at the same temperature and the mixture was stirred overnight at room temperature. The mixture was cooled to 5°C and the urethane was filtered . The filtrate was evaporated under reduced pressure and the product was isolated by silica gel column chromatography .
Yield : 6,6g = 71%
¹H-NMR (CDCl₃) 0.91 (m, 6H) 2.12 (m, 1H) 4.38 (m, 5H) 5.14 (s, 2H) 5.24 (m, 1H) 6.20 (m, 1H) 6.92 (m, 1H) 7.30 (m, 13H)

### c) Benzyl-5-(N-FMOC-L-valyloxy)-4-stearoyloxy-2-pentenoate

To a solution of benzyl-5-(N-FMOC-L-valyloxy)-4-hydroxy-2-pentenoate (6.5g, 12 mmol) and pyridine (2.0g, 25 mmole) in 100 ml dichloromethane at 10°C was added dropwise a solution of stearoylchloride (4.55g, 15 mmol) in 25 ml dichloromethane. The mixture was stirred overnight 100 ml of 5% sodium hydrogencarbonate solution was added and the mixture was stirred for 30 minutes . The organic phase was seperated and the water phase was extracted two times with dichloromethane. The combined organic phases were dried with sodium sulfate and concentrated in vacuo. The product was isolated by silica gel column chromatography .Yield: 7,8g = 80%
¹H-NMR (CDCl₃) 0.88 (m, 9H) 1.25 (m, 28H) 1.58 (m, 2H) 2.14 (m, 1H) 2.32 (m, 2H) 4.22 (m, 5H) 5.19 (s, 2H) 5.25 (m, 1H) 6.12 (m, 1H) 6.85 (m, 1H) 7.35 (m, 13H).

### d) 5-( N-FMOC-L-valyloxy)-4-stearoyloxy-pentanoic acid.

A solution of benzyl 5-(N-FMOC-L-valyloxy)-4-stearoyloxy-2-pentenoate (3.8g, 4.69 mmole) in 50 ml ethyl acetate was hydrogenated with 10% palladium on charcoal (0,5g) at normal pressure for five hours at room temperature. The catalyst was filtered and washed with ethyl acetate and 1,4-dioxane. The solution was evaporated under reduced pressure .Yield : 3.3g = 99%
¹H-NMR (CDCl₃) 0.92 (m, 9H) 1.25 (m, 28H) 1.54 (m, 2H) 1.98 (m, 2H) 2.18 (m, 1H) 2.28 (m, 2H) 2.41 (m, 2H) 4,32 (m, 5H) 5.13 (m, 1H) 5.33 (m, 1H) 7.50 (m, 8H)

### EXAMPLE 16

### 3-(N-FMOC-L-valyloxy)-2-stearoyloxypropionic acid

### a) Benzyl 2,3-dihydroxypropionate .

A mixture of D,L-glyceric acid, calcium salt dihydrate (2.9g, 10 mmole) and benzylbromide (3.8g, 22 mmole) in 25 ml DMF was stirred at 60°C overnight. The mixture was evaporated under reduced pressure and the product was isolated by silica gel chromatography. Yield : 4g = 100%
¹H-NMR (CDCl₃) 3.26 (s, 1H) 3.90 (m, 2H) 4.32 (m, 1H) 5.25 (s, 2H) 7.28 (m, 5H)

### b) Benzyl 3-(N-FMOC-L-valyloxy)-2-hydroxypropionate

A solution of benzyl-2,3-dihydroxypropionate ( 4,0g, 20 mmole ), N-FMOC-L-valine (5.4g, 16 mmole) and DMAP( 0.2g, 1.6 mmole) in 80 ml dichloromethane was cooled to about 10°C . A solution of DCC (4.12g, 20 mmole) in 25 ml was added dropwise at the same temperature and the mixture was stirred overnight at room temperature. The mixture was cooled to 5°C and the urethane was filtered .

The solution was evaporated under reduced pressure and the product was isolated by silica gel chromatography. Yield: 4.7g = 45%
¹H-NMR (CDCl₃) 0.88 (m, 6H) 2.05 (m,1H) 4.40 (m,6H) 5.23 (in, 3H) 7.50 (m, 13H)

### c) Benzyl 3-(N-FMOC-L-valyloxy)-2-stearoyloxypropionate

To a stirred solution of benzyl 3-(N-FMOC-L-valyloxy)-2-hydroxypropionate (4.6g 8.89 mmole) and pyridine (1.41g, 17.8 mmole) in 80 ml dichloromethane was added dropwise a solution of stearoylchloride (3.64g, 12 mmole) in 20 ml dichloromethane and the mixture was stirred overnight at room temperature. 100 ml of 5% sodium hydrogencarbonate solution was added and the mixture stirred for 30 minutes. The organic phase was seperated and the water phase was extracted two times with dichloromethane. The combined organic phases were dried with sodium sulfate and concentrated in vacuo. The product was isolated by silica gel chromatography. Yield : 6.1g = 87%
¹H-NMR (CDCl₃) 0.88 (m, 9H) 1.26 (m, 28H) 1.56 (m, 2H) 2.06 (m, 1H) 2.34 (m, 2H) 4.36 (m, 6H) 5.19 (s, 2H) 5.32 (m, 1H) 7.50 (m, 13H)

### d) 3- (N-EMOC-L-valyloxy )-2-stearoyloxypropionic acid.

A solution of benzyl 3-( N-FMOC-L-valyloxy )-2-stearoyloxypropionate (0.78g, 1 mmole) in 20 ml ethyl acetate was hydrogenated with 10% palladium on charcoal (0.2g) at normal pressure for three hours at room temperature . The catalyst was filtered and washed with ethyl acetate and 1,4-dioxane. The solution was evaporated under reduced pressure. Yield : 0.63g = 90%
¹H-NMR (CDCl₃) 0.88 (m, 9H) 1.24 (m, 28H) 1.40 (m, 2H) 2.12 (m, 3H) 4.30 (m, 5H) 5.16 (m,1H) 5.60 (m,1H) 7.40 (m, 8H)

### EXAMPLE 17

### 1-(N-Benzyloxycarbonyl-L-valyloxymethyl)-2-stearoyloxyethoxycarbonyl chloride

Bis(trichloromethyl) carbonate (160 mg; 0.54 mmol) was added with stirring to a solution of 1-(N-benzyloxycarbonyl-L-valyl)-3-stearoylglycerol; 1-(N-benzyloxycarbonyl-L-valyloxy)-3-stearoyloxy-2-propanol; (660 mg; 1.12 mmol) and triethylamine (200 mg; 2.0 mmol) in dichloromethane (5 ml) at room temperature. After 1h, n-hexane (10 ml) was added and the precipitated triethylamine hydrochloride was filtered off through a short column of silica gel, the product eluted with a further amount of n-hexane, and the solvent evaporated in vacuum to yield 650 mg (89%) of the title compound.
¹³C NMR (CDCl₃, 62.975 MHz): δ 172.8 (stear-COO); 171.2 (Val-COO); 155.9 (CONH); 154.1 (COCl); 136.0 (Ph-Cl-Val); 128.1-127.7 (Ph); 67.2 (CHOH); 66.7 (Ph CH₂); 63.1 (ValCOOCH₂); 61.8 (stear-COOCH₂); 58.7 (Val-αC); 33.7 (stear-C2); 31.6 (stear-C16); 31.0 (Val-βC); 29.3-28.8 (stear-C4-15); 24.5 (stear-C3); 18.6 and 17.1 (Val 2 CH₃); 13.8 (stear-C18).

### EXAMPLE 18

### 3-(N-CBz-L-valyloxymethyl)-4-stearoyloxybutylchloroformate

### a) 3- ( N-CBz-L-valyoxymethyl )-4-stearoyloxy-butanol.

To a stirred solution of 4-stearoyloxy-3-(N-CBz-L-valyloxymethylbutyraldehyde (prepared analogously to preparative example 6, step d) using CBz protected valine) (2.0 g, 3.2 mmole) in 25 ml methanol at 10°C was added sodium borohydride (0.6g, 16 mmole) in small portions . The mixture was stirred for 30 minutes and then acidified with acetic acid .The mixture was diluted with water and extracted three times with dichloromethane.The organic phase was dried with sodium sulfate and concentrated in vacuo .The product was isolated by silica gel column chromatography. Yield: 1,5g = 75%.
¹H-NMR (CDCl₃) 0.88 (m, 9H) 1.25 (m, 28H) 1.52 (m, 4H) 2.24 (m, 3H) 3.68 (m, 2H) 4.12 (m, 4H) 4.24 (m, 1H) 5.08 (s, 2H) 5.22 (m, 1H) 7.36 (m, 5H)

### b) 3-(N-CBz-L-valyloxymethyl)-4-stearoyloxybutyl chloroformate

A solution of the intermediate of step a) in 20 ml of a 20% solution of phosgene in toluene was stirred overnight . The mixture was evaporated under reduced pressure to yield the title compound.Yield 1.5g = 97%.
¹H-NMR (CDCl₃) 0.88 (m, 9H) 1.28 (m, 28H) 1.58 (m, 2H) 1.72 (m, 2H) 2.15 (m, 1H) 2.31 (m, 2H) 4.08 - 4.42 (m, 5H) 5.10 (s, 2H) 5.22 (m, 1H) 7.36 (m, 5H)

### EXAMPLE 19

### 2',3'-dideoxy-3'-fluoro-5'-O-[1-(L-valyloxy)-2-stearoyloxy-3-propyloxy carbonyl] guanosine

### a) Synthesis of 2',3'-dideoxy-3'-fluoro-5'-O-[1-(N-CBz-L-valyloxy )-2-stearoyloxy-3-propyloxy carbonyl] guanosine.

To a solution of 2',3'dideoxy-3'-fluoro-guanosine (270 mg, 1 mmole) in DMF (10 ml) and pyridine (1 ml) was added 3-{1-( N-CBz-L-valyl)-2-stearoyl} propyl chloroformate (619 mg, 0.5 mmole) at 0 °C. After 3 h, the reaction mixture was poured into sodium hydrogen carbonate solution and extracted with dichloromethane. The organic phase was dried in vacuo, and 2',3'-dideoxy-3'-fluoro-5'-O-[1-( N-CBz-L-valyloxy)-2-stearoyloxy-3-propyloxy carbonyl] guanosine was isolated by silica gel column chromatography (195 mg).
¹H-NMR (CDCl₃): 7.69 (s, 1H), 7.31 (m, 5H), 6.50 (m, 2H), 6.32 (m, 1H), 5.3 (m, 2H), 5.09 (m, 2H), 4.35 (m, 7H), 2.60 (m, 2H), 2.31 (t, 2H), 2.20 (m, 1 H), 1.58 (m, 2H), 1.23 (m, 28 H), 0.92 (m, 9H).

### b) Synthesis of 2',3'-dideoxy-3'-fluoro-5'-O-[1-(L-valyloxy)-2-stearoyloxy-3-propyloxy carbonyl] guanosine.

2',3'-dideoxy-3'-fluoro-5'-O-[1-( N-CBz-L-valyloxy)-2-stearoyloxy-3-propyloxy carbonyl] guanosine (190 mg), was dissolved in a mixed solvent of methanol (6 ml), ethyl acetate (2 ml) and acetic acid (1 ml). To the solution was added palladium black (30 mg), and the reaction mixture was kept under hydrogen for 2h. It was then filtered and the filtrate was evaporated and the titled product was isolated by silica gel column. 110 mg.
¹H-NMR (DMSO-δ6): 7.86 (ds, 1H), 6.51 (s, 2H), 6.17 (dd, 1H), 5.48 (m, 1H), 5.20 (m, 1H), 4.25 (m, 7H), 2.70 (m, 2H), 2.27 (m, 2H), 1.72 (m, 1H), 1.47 (m, 2H), 1.22 (m, 28 H), 0.84 (m, 9H).

### EXAMPLE 20

### 2',3'-dideoxy-3'-fluoro-5'-O-[5-(L-valyloxy)-4-stearoyloxy-pentanoyl]guanosine.

To a solution of 2', 3'-dideoxy-3'-fluoroguanosine (0.27g, 1 mmole) and 5-(N-FMOC-L-valyloxy)-4-stearoyloxypentanoic acid (0.94g, 1.3 mmole) in 30 ml DMF was added DMAP (16mg, 0.13mmol), HOBT (0.176 g, 1.3 mmole) and DCC (0.248g, 1.2 mmole). The mixture was stirred for three days at room temperature.
4g silica gel were added and the mixture evaporate in vacuo. The product, 2', 3'-dideoxy-3'-fluoro-5'-O- [5-(FMOC-L-valyloxy)-4-stearoyloxy-pentanoyl]guanosine was separated by silica gel chromatography. Yield: 0.45g
¹H-NMR (DMSOδ-6) 0.88 (m, 9H) 1.20 (m, 28H) 1.45 (m, 2H) 1.78 (m, 2H) 2.18 (m, 2H) 2.36 (m, 1H) 2.62 (m, 2H) 3.88 (m, 1H) 4.22 (m, 6H) 4.92 (m,1H) 5,45(m, 1H) 6.19 (m, 1H) 6.52 (s, 2H) 7.26 - 7.88 (m, 8H)

The protected intermediate is deprotected as shown above to yield the title compound.

### EXAMPLE 20a

### 2',3'-dideoxy-3'-fluoro-5'-O-[3-(N-FMOC-L-valyloxy)-2-stearoyloxypropanoyl] guanosine

To a stirred mixture of 3-(N-FMOC-L-valyloxy)-2-stearoyloxypropanoic acid (0.61g, 0.88 mmol) in 5 ml dry diethylether was added one drop DMF and thionyl chloride(0.52g, 4.4 mmole). The mixture was refluxed for two hours and then evaporated under reduced pressure. The product was dissolved in dry dichloromethane and added dropwise to a solution of 2', 3'-dideoxy-3'-fluoroguanosine (0.215 g, 0.8 mmole) and pyridine (0.35g, 4.4 mmole) in 20 ml DMF. The solution was stirred overnight . Two grammes of silica gel were added and the mixture was evaporated in vacuo . The product was isolated by silica gel chromatography. Yield : 0.19g = 25%
¹H-NMR (CDCl₃) 0.88 (m, 9H) 1.25 (m, 28H) 1.62 (m, 2H) 2.12(m, 1H) 2.38 (m, 2H) 2.58 (m, 2H) 4.12- 4.76 (m, 6H) 5.32 (m, 2H) 6.12 (m, 1H) 6.26 (m, 1H) 6.44 (m, 1H) 7.12-7.78 (m, 8H).

### EXAMPLE 21

### 1-( N-CBz-L-valyl)-3-stearoyl-2-propyl succinate monoester

1-( N-CBz-L-valyl)-3-stearoyl-glycerol (886 mg, 1.5 mmole) and succinic anhydride (450 mg, 4.5 mmole) were dissolved in a mixed solvent of DMF (15 ml) and pyridine (1 ml). The reaction was kept at room temperature for 3 h, and then at 60 °C for 5 h. The reaction mixture was poured into a solution of acetic acid and water and extracted with dichloromethane. The organic phase was washed with water and evaporated, and the product was isolated by silica gel column chromatography to yield 900 mg.
¹H-NMR (CDCl₃): 7.43 (m, 5H), 5.27 (m, 1H), 5.09 (m, 2H), 4.21 (m, 5H), 2.54 (m, 4H), 2.29 (t, 2H), 2.13 (m, 1H), 1.59 (m, 2H), 1.25 (m, 28 H), 0.90 (m, 9H).

### EXAMPLE 22

### 2',3'-dideoxy-3'-fluoro-5'-O-{3-[1-( L-valyloxy)-3-stearoyloxy-2-propyloxy carbonyl]-propanoyl}guanosine

To a solution of 2',3'-dideoxy-3'-fluoro-guanosine (351 mg, 1.3 mmole) and 1-(N-CBz-L-valyl)-3-stearoyl-2-propyl succinate monoester (900 mg, 1.3 mmole) in DMF (15 ml) were added dimethylaminopyridine (24 mg, 0.2 mmole), 1-hydroxybenzotriazole (175 mg, 1.3 mmole), DCC (321 mg, 1.56 mmole). After 48 h, the reaction mixture was filtered. The filtrate was poured into sodium hydrogen carbonate solution and extracted with dichloromethane. The product 2',3'-dideoxy-3 '-fluoro-5'-O-{3-[1-(N-CBz-L-valyl)-3-stearoyl glyceroloxy carbonyl]propanoyl} guanosine was isolated by silica gel column chromatography. 780 mg
¹H-NMR (DMSO-d6): 7.89 (s, 1H), 7.34 (m, 5H), 6.50 (s, 2H), 6.17 (dd, 1H), 5.46 (m, 1H), 5.38 (m, 1H), 5.02 (s, 2H), 4.22 (m, 7H), 3.32 (s, 4H), 2.80 (m, 2H), 2.57 (m, 2H), 2.31 (t, 2H), 2.05 (m, 1H), 1.48 (m, 2H), 1.21 (m, 28 H), 0.84 (m, 9H).

To the solution of 2',3'-dideoxy-3'-fluoro-5'-O-{3-[1-(N-CBz-L-valyl)-3-stearoyl-2-propyloxy carbonyl]propanoyl}guanosine (460 mg, 0.5 mmole) in a mixed solvent of methanol (10 ml), ethyl acetate (3 ml) and acetic acid (2 ml) was added palladium black (50 mg). After reaction under hydrogen atmosphere for 2 h, the mixture was filtered and the filtrate was dried. The titled product was isolated by silica gel column chromatography. 360 mg.
¹H-NMR (DMSO-d6): 7.89 (s, 1H), 6.51 (s, 2H), 6.16 (dd, 1H), 5.48 (m, 1H), 5.17 (m, 1H), 4.28 (m, 7H), 2.90 (m, 2H), 2.58 (m, 4H), 2.28 (t, 2H), 1.85 (m, 1H), 1.49 (m, 2H), 1.22 (m, 28 H), 0.85 (m, 9H).

### EXAMPLE 23

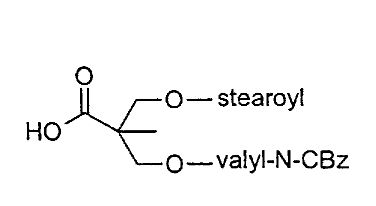

A solution of stearoyl chloride (12.1g, 40 mmol, 1.0 eq) in CH₂Cl₂ (100 ml) was slowly (1h) added to a solution of 2,2-bis(hydroxymethyl)propionic acid (26.8 g, 200 mmol, 5.0 eq) in pyridine (400 ml) at room temperature. The reaction mixture was stirred at room temperature overnight and thereafter concentrated (100 ml) under vacuum. The reaction mixture was slowly treated with saturated NaHCO₃ (400 ml) and thereafter extracted with CH₂Cl₂ (3x300 ml). The organic layers were combined, washed with brine, dried over Na₂SO₄ and concentrated in vacuum. The crude material was chromatographed on silica gel (500 g) with 19/1 to 4/1 CH₂Cl₂-MeOH as eluent, to yield the monostearoyl ester, R_{f} (9/1 CH₂Cl₂-MeOH) 0.33. 12.5 g (78%).

A solution of N-Cbz-L-valine (18.85 g, 75 mmol, 2.4 eq) and DMAP (855 mg, 7 mmol, 0.22 eq) in CH₂Cl₂ (800 ml) was cooled to 0° C and treated with DCC (14.4 g, 70 mmol, 2.2 eq). The reaction mixture was stirred at room temperature for 30 min and thereafter slowly (lh) treated with a solution of the above monostearoyl ester (12.5 g, 31.2 mmol, 1 eq) in CHCl₃ (200 ml, free of ethanol). After stirring overnight the suspention was filtered and the filtrate was washed with brine, dried with Na₂SO₄ and concentrated in vacuum. The crude material was chromatographed on silica gel (500 g) with 19/1 to 4/1 CH₂Cl₂-MeOH as eluent, to yield the above depicted diester. R_{f} (9/1 CH₂Cl₂-MeOH) 0.46. 13.8 g (70 %).
¹H-NMR (250 MHz, CDCl₃) δ 7.35-7.3 (m, 5H, ArH), 5.32 (d, 1H, CH), 5.10 (s, 2H, CH₂Ph), 4.33-4.18 (m, 4H, CH₂), 2.28 (t, 2H, CH₂), 2.22-2.05 (m, 1H, CH), 1.65-1.50 (m, 2H, CH₂) 1.35-1.15 (m, 31H), 1.00-0.82 (m, 9H, Me).

### EXAMPLE 24

### 2',3'-Dideoxy-3'-fluoro-5'-O-[5-(L-valyloxy)-4-stearoyloxy-pentanoyl]guanosine.

### a) Synthesis of 2',3'-di deoxy-3'-fluoro-5'-O-[5-(N-FMOC-L-valyoxy)-4-stearoyloxy-pentanoyl] guanosine.

A mixture of 2',3'-dideoxy-3'-fluoroguanosine (269 mg, 1.0 mmole), 5-(N-FMOC-L-valyloxy)-4-stearoyloxy-pentanoic acid (940mg, 1.3 mmole), DMAP (16mg, 0.13 mmole) and HOBT (176mg, 1.3 mmole) was co-evaporated two times with DMF and reduced to about 30ml. DCC (248mg, 1.2 mmole) was added and the mixture was stirred overnight at room temperature. The mixture was filtered and the solution was evaporated under reduced pressure. Ethyl acetate (50 ml) was added and the organic phase was washed two times with 5% acetic acid, with 5% sodium hydrogen carbonate and with water. The organic phase was dried with sodium sulfate and evaporated under reduced pressure. The product is isolated by silica gel column chromatography. Yield : 450mg
¹H-NMR (DMSO d-6) 0.88 (m, 9H) 1.22 (m, 28H) 1.45 (m, 2H) 1.83 (m, 2H) 2.21 (m, 2H) 2.37 (m, 1H) 3.90 (m, 1H) 5.36-5.58 (m, 1H) 6.18 (m, 1H) 6.50 (s, 2H) 7.28-7.91 (m, 10H)

### b) Synthesis of 2',3'-dideoxy-3'-fluoro-5'-O-[5-(L-valyloxy)-4-stearoyloxy-pentanoyl] guanosine.

A mixture of 2', 3'dideoxy-3'-fluoro-5'-O- [5-(N-CBZ-L-valyloxy)-4-stearoyloxy-pentanoyl] guanosine (300mg, 0.308 mmole) in 5ml N,N-diisopropylethyiamine and 5ml DMF was stirred for three days at room temperature. Acetic acid (5 ml) was added and the mixture was evaporated under reduced pressure . The product was isolated as the acetate salt by silica gel column chromatography. Yield: 90 mg
¹H-NMR (DMSO d-6 ) 0.88 (m, 9H) 1.24 (m, 28H) 1.55 (m, 2H) 1.91 (m, 2H) 2.31 (m, 2H) 2.44 (m, 1H) 2.56-3.08 (m, 2H) 3.15 (m, 1H) 4.00-4.49 (m, 5H) 5.08 (m, 1H) 5.40-5.62 (m, 1H) 6.24 (m, 1H) 6.54 (s, 2H) 7.96 (s, 1H)

### EXAMPLE 25

### 2',3'-Dideoxy-3'-fluoro-5'-O-[3-(L-valyloxy)-2-stearoyloxy-propanoyl] guanosine

### a) Synthesis of 2', 3'-Dideoxy-3'-fluoro-5'-O- [3-(N-CBZ-L-valyloxy)-2-stearoyloxy-propanoyl]guanosine.

A mixture of 2', 3'-dideoxy-3'-fluoroguanosine (404mg, 1.5 mmole), 3-(N-CBZ-L-valyloxy)-2-stearoyloxy-propanoic acid (1.06g, 1.75 mmole), DMAP (24mg, 0.2 mmole) and HOBT (264mg, 1.82 mmole) was co-evaporated two times with DMF and reduced to about 30ml. DCC (372mg, 1.8 mmole) was added and the mixture was stirred overnight at room temperature. The mixture was filtered and the solution was evaporated under reduced pressure. Ethyl acetate (50 ml) was added and the organic phase was washed twice with 5% acetic acid, with 5% sodium hydrogen carbonate and with water. The organic phase was dried with sodium sulfate and evaporated under reduced pressure. The product was isolated by silica gel column chromatography. Yield: 0.73g
¹H-NMR (DMSO d-6) 0.82 (m,9H) 1.22 (m, 28H) 1.48 (m, 2H) 2.31 (m, 2H) 2.50-3.00 (m, 2H) 3.91 (m, 1H) 4.18-4.52 (m, 5H) 5.00 (s, 2H) 5.30-5.61 (m, 2H) 6.16 (m, 1H) 6.50 (s, 2H) 7.32 (m, 5H) 7.71 (m, 1H) 7.92 (s, 1H) 10.18 (s, 1H)

### b) Synthesis of 2', 3'-dideoxy-3'-fluoro-5'-O- [3-(L-valyloxy)-2-stearoyloxy-propanoyl]guanosine.

A solution of 2', 3'-dideoxy-3'-fluoro-5'-O-[3- (N-CBZ-L-valyloxy)-2-stearoyloxy-propanoyl]guanosine (350mg, 0.4 mmole) in ethyl acetate (25ml), methanol (5ml) and acetic acid (5ml) was hydrogenated with palladium black (300mg) with normal pressure for three hours. The catalyst was filtered and washed with ethyl acetate and methanol. The solution was evaporated under reduced pressure and the product was isolated as the acetate salt by silica gel column chromatography. Yield: 120mg
¹H-NMR (DMSO d-6) 0.84 (m, 9H) 1.22 (m, 28H) 1.50 (m, 2H) 2.32 (m, 2H)
2.50-3.00 (m, 2H) 3.07 (m, 1H) 4.21-4.59 (m, 5H) 5.38-5.59 (m, 2H) 6.17 (m, 1H) 6.0 (s, 2H) 7.90 (s, 1H)

### EXAMPLE 26

### 2',3'-Dideoxy-3'-fluoro-5'-O-[3,3-bis(L-valyloxymethyl)-propionic acid] guanosine

### a) Synthesis of 4,4-bis (N-CBZ-L-valyloxymethyl)-but-1-ene.

To a solution of 2-allyl-1,3-propandiol (2.32g, 20 mmole), N-CBZ-L-valine (10.06g, 40 mmole) and DMAP (0.488g, 4 mmole) in 120ml dichloromethane was added DCC (9.08g, 44 mmole) in portions and the mixture was stirred overnight at room temperature. The mixture was cooled to 5°C and the urethane was filtered. The filtrate was evaporated and the product was isolated by silica gel column chromatography. Yield : 9.0g
¹H-NMR (CDCl₃) 0.89 (m, 12H) 5.11 (s, 2H) 5.73 (m, 1H)

### b) Synthesis of 3,3-Bis (N-CBZ-L-valyloxymethyl)-propionic acid.

To a cooled solution of 4,4-bis (N-CBZ-L-valyloxymethyl)-but-1-ene (14.6g, 25 mmole) and tetrabutylammonium bromide (1.3g, 4 mmole) in 120ml benzene was added 100ml water. Under strong stirring potassium permanganate (15.8g, 100 mmole) was addded in portions and the mixture was stirred for 2 hours between 15°C and 20°C . A sodium bisulfite aqueous solution was added to the slurry until the mixture was discolored. The mixture was acidified with 2N hydrochloric acid and extracted four times with ethyl acetate. The organic phase was washed two times with water, dried with sodium sulfate and evaporated under reduced pressure . The product was isolated by silica gel column chromatography. Yield: 7.5g
¹H-NMR (CDCl₃) 0.89 (m, 12H) 2.05 (m, 2H) 2.46 (m, 2H) 2.62 (m, 1H) 4.20 (m, 6H) 5.11 (s, 4H) 5.30 (m, 2H) 7.35 (m, 10H)

### c) Synthesis of 2',3'-dideoxy-3'-fluoro-5'-O-[3,3-bis (N-CBZ-L-valyloxymethyl)-propionyl]guanosine.

A solution of 2',3'-dideoxy-3'-fluoroguanosine (1.35g, 5 mmole), 3,3-bis (N-CBZ-L-valyloxymethyl)-propionic acid (3.6g, 6 mmole), DMAP (0.061g, 0.5 mmole) and HOBT (0.81g, 6 mmole) was co-evaporated two times with DMF and reduced to about 120ml. DCC (1.24g, 6 mmole) was added and the mixture was stirred overnight at room temperature. The mixture was filtered and the solution was
evaporated under reduced pressure. Ethyl acetate (200 ml) was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 2.7g
¹H-NMR (DMSO d-6) 0.88 (m, 12H) 2.00(m, 2H) 2.50-3.00 (m, 2H) 3.90-4.43 (m, 10H) 5.08 (s, 4H) 5.32-5.59 (m, 1H) 6.17 (m, 1H) 6.50 (s, 2H) 7.28 (m, 10H) 7.72 (m, 2H) 7.90 (s, 1H)

### d) Synthesis of 2', 3'-Dideoxy-3'-fluoro-5'-O- [3,3-bis (L-valyloxymethyl)-propionic acid] guanosine.

A solution of 2', 3'-dideoxy-3'-fluoro-5'-O-[3,3-bis (N-CBZ-L-valyloxymethyl)-propionyl] guanosine (2.6g, 3.1 mmole) in 80ml ethyl acetate, 20ml methanol and 20ml acetic acid was hydrogenated with palladium black (0.3g) for two hours under normal pressure. The catalyst was filtered and washed with ethyl acetate and methanol. The solution was evaporated under reduced pressure and the product was isolated as the bisacetate salt by silica gel column chromatography. Yield: 1.2g
¹H-NMR (DMSO d-6) 0.90 (m, 12H) 1.78 (m, 2H) 2.50-3.00 (m, 2H) 3.09 (m, 2H) 4.02-4.45 (m, 8H) 5.34-5.59 (m, 1H) 6,17 (m, 1H) 6.62 (s, 2H) 7.88 (s, 1H)

### EXAMPLE 27

### 2',3'-Dideoxy-3'-fluoro-5'-O-[3-(L-valyloxymethyl)-4-stearoyloxy-butoxycarbonyl] guanosine

### a) Synthesis of 2', 3'-Dideoxy-3'-fluoro-5'-O- [3-(N-CBZ-L-valyloxymethyl)-4-stearoyloxy-butoxycarbonyl] guanosine.

To a solution of 2',3'-dideoxy-3'-fluoroguanosine (269mg, 1.0 mmole in absolute DMF were added pyridine (198mg, 2.5 mmole) and a solution of 3-(N-CBZ-L-valyloxymethyl)-4-stearoyloxy-butoxycarbonyl chloride (750mg, 1.1 mmole) in 5ml dichloromethane. The mixture was stirred for three days at room temperature. The solution was evaporated under reduced pressure and the product was isolated by silica gel column chromatography. Yield: 120mg
¹H-NMR (DMSO d-6) 0.88 (m 9H) 1.24 (m, 28H) 5.08 (s, 2H) 6.24 (m, 1H) 8.00 (s, 1H)

### b) Synthesis of 2', 3'-Dideoxy-3'-fluoro-5'-O-[3-(L-valyloxymethyl)-4-stearoyloxy-butoxycarbonyl]guanosine.

A mixture of 2', 3'-dideoxy-3'-fluoro-5-0-[3-(N-CBZ-L-valyloxymethyl)-4-stearoyloxy-butoxycarbonyl]guanosine in 15ml ethyl acetate, 2ml methanol and 2ml acetic acid was hydrogenated with palladium black (40mg) under normal pressure for two hours. The catalyst was filtered and washed with ethyl acetate and methanol. The solution was evaporated and the product isolated as the acetate salt by silica gel column chromatography. Yield: 78mg
¹H-NMR (DMSO d-6) 0.87 (m, 9H) 1.22 (m, 28H) 1.48 (m, 2H) 1.68 (m, 2H) 2.12 (m, 1H) 2.26 (m, 2H) 2.50-3.00 (m, 2H) 4.00-4.42 (m, 10H) 5.34-5.58 (m, 1H) 6.18 (m, 1H) 6.52 (s, 2H) 7.82 (s, 1H)

### EXAMPLE 28

### 2',3' -Dideoxy-3'-fluoro-5'-O-[2-(L-valyloxy)stearoyl]guanosine

### a) Synthesis of benzyl 2-hydroxystearate.

To a stirred solution of DL-2-hydroxystearic acid (3.0g, 10 mmole) in 20 ml dry DMF was added potassium tert-butoxide (1.23g, 11 mmole) and the mixture was stirred for one hour at 60°C. Benzyl bromide (2.14g, 12.5 mmole) was added and the mixture was stirred for six hours at 80°C. The mixture was evaporated under reduced pressure and 100 ml ethyl acatate was added. The organic phase was separated and washed four times with water.The organic phase was dried with sodium sulfate and concentrated in vacuo . The product was isolated by silica gel column chromatography. Yield: 3.3g
¹H-NMR (CDCl₃) 0.88 (m, 3H) 1.26 (m, 28H) 1.62 (m, 2H) 4.20 (m, 1H) 5.20 (s, 2H) 7.36 (m, 5H)

### b) Synthesis of benzyl-2-(N-FMOC-L-valyloxy)stearate.

To a solution of benzyl-2-hydroxystearate (3.2g, 8.2 mmole), N-FMOC-L-valine (3.4g, 10 mmole) and DMAP (0.12g, 1 mmole) in 80 ml dichloromethane was added a solution of DCC (2.5g, 12 mmole) and the mixture was stirred overnight at room temperature. The mixture was cooled to 5°C and the urethane was filtered. The filtrate was evaporated and the product was isolated by silica gel column chromatography. Yield: 4.5g
¹H-NMR (CDCl₃) 0.90 (m, 6H) 1.26 (m, 6H) 1.82 (m, 2H) 2.16 (m, 1H) 4.21 (m, 1H) 4.36 (m, 2H) 5.10 (m, 1H) 5.18 (s, 2H) 5.28 (m, 1H) 7.20 - 7.80 (m, 13H)

### c) Synthesis of 2-(N-FMOC-L-valyloxy) stearic acid

A solution of benzyl-2-(N-FMOC-L-valyloxy)stearate (4.4g, 6.2 mmole) in 50 ml ethyl acetate was hydrogenated with 10% palladium on charcoal (0.5g) with normal pressure for two hours. The catalyst was filtered and washed with ethyl acetate and 1,4-dioxane. The solution was evaporated under reduced pressure and the product was isolated by silica gel column chromatography.Yield: 3.4g
¹H-NMR (CDCl₃) 0.88 (m, 6H) 1.26 (m, 28H) 1.82 (m, 2H) 2.28 (m, 1H) 4.20 (m, 1H) 4.40 (m, 2H) 5.00 (m, 1H) 5.41 (m, 1H) 7.26 - 7.82 (m, 8H)

### d) Synthesis of 2',3'-Dideoxy-3'-fluoro-5'-O-[2-(N-FMOC-L-valyloxy)stearoyl] guanosine.

A mixture of 2',3'-dideoxy-3'-fluoroguanosine (404mg, 1.5mmole), 2-(N-FMOC-L-valyloxy)-stearic acid (1.24g, 2 mmole), DMAP (24mg, 0.2 mmole) and HOBT (264mg, 1,95 mmole) was co-evaporated two times with DMF and reduced to about 30ml. DCC (372mg, 1.8 mmole) was added and the mixture was stirred overnight at room temperature. The mixture was filtered and the solution was evaporated under reduced pressure. Ethyl acetate (50 ml) was added and the organic phase washed twice with 5% acetic acid, with 5% sodium hydrogen carbonate and with water. The organic phase was dried with sodium sulfate and evaporated under reduced pressure . The product was isolated as the acetate salt by silica gel column chromatography. Yield: 1.2g
¹H-NMR (DMSO d-6) 0.80-0.90 (m, 9H) 1.22 (m, 28H) 2.12 (m, 1H) 2.50-3.00 (m, 2H) 3.98 (m,1H) 4.96 (m, 1H) 6.17 (m, 1H) 6.50 (s, 2H) 7.32-7.95 (m, 10H)

### e) Synthesis of 2',3'-dideoxy-3'-fluoro-5'-O-[2-(L-valyloxy)-stearoyl] guanosine.

To a solution of 2',3'-dideoxy-3'-fluoro-5'-O-[2-(N-FMOC-L-valyloxy) stearoyl] guanosine (800mg, 0.89 mmole) in 15ml DMF was added DBU (1.35g, 8.9 mmole) and the mixture was stirred for 5 minutes at room temperature. Acetic acid (2 ml) was added and the mixture was evaporated under reduced pressure. Water (20 ml) were added and the mixture was extracted three times with dichloromethane.The organic phase was dried with sodium sulfate and evaporated under reduced pressure . The product was isolated by silica gel column chromatography. Yield: 165mg
¹H-NMR (DMSO d-6) 0.87 (m, 9H) 1.22 (m, 28H) 1.70 (m, 2H) 1.88 (m, 1H) 2.50-3.00 (m, 2H) 3.20 (m, 1H) 4.32 (m, 3H) 4.94 (m, 1H) 5.32-5.54 (m, 1H) 6.14 (m, 1H) 6.49 (s, 2H) 7.89 (s, 1H)

### EXAMPLE 29

### 2',3'-Dideoxy-3'-fluoro-5'-O-3-[1,3-bis-(L-valyloxy)-2-propyloxycarbonyl-propanoyl]guanosine

### a) Synthesis of 1,3-dibenzyloxy-2-propyl succinate monoester.

A solution of 1,3-dibenzyloxypropan-2-ol (6.8g, 25 mmole) and succinic anhydride (7.5g, 75 mmole) and DMAP (12.2g, 100 mmole) was stirred for one hour at 60°C. The mixture was evaporated under reduced pressure, acidified with 2N HCl and extracted two times with ethyl acetate. The combined organic phase was washed three times with water, dried with sodium sulfate and evaporated under reduced pressure.
The product was isolated by silica gel column chromatography. Yield: 7.8g

### b) Synthesis of 2', 3'-dideoxy-3'-fluoro-5'-O-[3-(1,3-dibenzyloxy-2-propyloxycarbonyl)-propanoyl] guanosine

A mixture of 2', 3'-dideoxy-3'-fluoroguanosine (1.61g, 6 mmole), HOBT (0.972g, 7.2 mmole), DMAP (73.3mg, 0.6 mmole) and 1,3-dibenzyloxy-2-propyl succinate monoester (2.68g, 7,2 mmole) was co-evaporated two times with DMF and reduced to about 150ml. DCC (1.55g, 7.5 mmole) was added and the mixture was stirred 72 hours at room temperature. The mixture was filtered and the solution was evaporated under reduced pressure. Ethyl acetate (200 ml) was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 3.3g

### c) Synthesis of 2', 3'-dideoxy-3'-fluoro-5'-O [3-(1,3-dihydoxy-2-propyloxy carbonyl)propanoyl]guanosine

A solution of 2', 3'-dideoxy-3'-fluoro-5'-O-[3-(1,3-dibenzyloxy-2-propyloxy carbonyl)propanoyl]guanosine (3.2g, 5.13 mmole) in 50 ml ethylacetate, 50 ml methanol and 10 ml acetic acid was hydrogenated with palladium black (0.6g) under 40 psi overnight. The catalyst was filtered and washed with methanol, The solution was evaporated under reduced pressure and the product was isolated by silica gel column chromatography. Yield: 1.64g

### d) Synthesis of 2',3'-dideoxy-3'-fluoro-5'-O-{3-[1,3-Bis ( N-CBZ-L-valyloxy)-2-propyloxycarbonyl]propanoyl} guanosine.

A mixture of 2',3'-dideoxy-3'-fluoro-5'-O-[3-(1,3-dihydroxy-2-propyloxy carbonyl)propanoyl]guanosine (1.93g, 2.93 mmole), N-CBZ-L-valine (1.76g, 7 mmole), HOBT (0.95g, 7 mmole) and DMAP (85.5mg, 0.7 mmole) was co-evaporated two times with DMF and reduced to about 60 ml. DCC (1.55g; 7.5 mmole) was added and the mixture was stirred overnight at room temperature. The mixture was warmed for four hours at 60°C and then cooled to about 10°C. The mixture was filtered and the solution was reduced under reduced pressure. Ethyl acetate (150 ml) was added and the organic phase was washed twice with 5% acetic acid, 5% sodium hydrogen carbonate and water. The organic phase was dried with sodium sulfate and evaporated under reduced pressure. The product was isolated by silica gel column chromatography. Yield: 1.6g.

### e) Synthesis of 2', 3'-dideoxy-3'-fluoro-5'-O-{3-[1,3-bis-(L-valyloxy)-2-propyloxycarbonyl]-propanoyl} guanosine.

A solution of 2',3'-dideoxy-3'-fluoro-5'-O-{3-[1,3-bis-(N-CBZ-L-valyloxy)-2-propyloxycarbonyl)propanoyl}guanosine(1.6g, 1.75 mmole) in 80 ml ethyl acetate, 20 ml methanol and 20 ml acetic acid was hydrogenated with palladium black (0.3g) for two hours at room temperature and normal pressure. The catalyst was filtered and washed with methanol. The solution was evaporated under reduced pressure and the product was isolated as the diacetate salt by silica gel column chromatography. Yield: 1.02g
¹H-NMR (DMSO d-6) 0.84 (m, 12H) 1.85(m, 2H) 2.58 (m, 4H) 2.60-3.10 (m, 2H) 3.11 (m, 2H) 3.61-4.39 (m, 7H) 5.19 (m, 1H) 5.35-5.56 (m, 1H) 6.16 (m, 1H) 6.62 (s, 2H) 7.89 (s, 1H)

### EXAMPLE 30

### 2',3'-Dideoxy-3'-fluoro-5'-O- {3-[1-(L-valyloxy)-3-hydroxy-2-propyloxy carbonyl]-propanoyl}guanosine

### a) Synthesis of 2',3'-dideoxy-3'-fluoro-5'-O-(3-[1-(N-CBZ-L-valyloxy)-3-hydroxy-2-propyloxy carbonyl]-propanoyl} guanosine.

A mixture of 2', 3'-dideoxy-3'-fluoro-5'-O-[3-(1,3-dihydroxy-2-propyloxy carbonyl)-propanoyl] guanosine (1.3g, 2.93 mmole), N-CBZ-L-valine (1.00g, 4 mmole), HOBT (0.54g, 4 mmole) and DMAP (48.8 mg, 0.4 mmole) was co-evaporated two times with DMF and reduced to about 60 ml. DCC (0.91g, 4.4 mmole) was added and the mixture was stirred for 72 hours at room temperature. The mixture was filtered and the solution evaporated under reduced pressure. Ethyl acetate (150 ml) was added and the organic phase washed twice with 5% acetic acid, 5% sodium hydrogen carbonate and water. The organic phase was dried with sodium sulfate and evaporated under reduced pressure. The product was isolated by silica gel column chromatography. Yield: 0.99g

### b) Synthesis of 2', 3'-dideoxy-3'-fluoro-5'-O-{3-[1-(L-valyloxy)-3-hydroxy-2-propyloxycarbonyl]-propanoyl} guanosine.

A solution of 2', 3'-dideoxy-3'-fluoro-5'-O-{3-[1-(N-CBZ-L-valyloxy)-3-hydroxy-2-propyloxycarbonyl)-propanoyl}guanosine (0.82g, 1.21 mmole) in 30 ml ethyl acetate, 15 ml methanol and 15 ml acetic acid was hydrogenated with palladium black (0.15g) for two hours at room temperature and normal pressure. The catalyst was filtered and washed with methanol. The solution was evaporated under reduced pressure and the product was isolated as the acetate salt by silica gel column chromatography. Yield: 0.5g
¹H.NMR (DMSO d-₆) 0.84 (m, 6H) 1.86 (m, 1H) 2.58 (m, 4H) 2.63-3.02 (m, 2H) 3.10-4.38 (m, 9H) 5.34-5.55 (m, 1H) 6.16 (m, 1H) 6.56 (s, 2H) 7.90 (s, 1H)

### EXAMPLE 31

### 5'-L-valyl-2',3'-dideoxy-3'-fluoroguanosine

To a solution of 2',3'- dideoxy-3'- fluoroguanosine (810 mg, 3 mmole) and 4-dimethylaminopyridine (73 mg, 0.6 mmole), N-CBz-L-valine (1.5 g, 6 mmole) and 1-hydroxybenzotriazole (810 mg, 6 mmole) in DMF (20 ml) was added DCC (1.36 g, 6.6 mmole). After 72 h, the reaction mixture was filtered and concentrated *in vacuo*. 5'-(N-CBz-L-valyl)-2', 3'-dideoxy-3'-fluoroguanosine was isolated by silica gel column chromatography (1.15 g).

This intermediate (503 mg, 1 mmole) was dissolved in a mixed solvent of ethyl acetate (10 ml), methanol (20 ml) and acetic acid (2 ml). To the mixture was added palladium black (100 mg) and the reaction mixture was kept under hydrogen at atmospheric pressure for 3 h. After filtration and concentration, the titled product was isolated by silica gel column chromatography (370 mg).
¹H-NMR (DMSO d-6): 7.94 (s, 1H), 6.52 (s, 2 H), 6.17 (dd, 1H), 5.47 (dd, 1H), 4.15 (m, 3H), 3.15 (d, 1 H), 3.01-2.62 (m, 2H), 1.80 (m, 1H), 0.82 (dd, 6 H).

### EXAMPLE 32

### 2', 3'-Dideoxy-3'-fluoro-5-O-[2-(-L-valyloxy)-propionyl]guanosine

### a) Synthesis of 4-methoxybenzyl-2-hydroxypropionate.

To a stirred solution of DL -2 hydroxypropionic acid (9.0g, 100 mmole) in 100 ml dry DMF was added potassium tert-butoxide (12.34g, 110 mmole) and the mixture was stirred for one hour at 60°C. 4-methoxybenzyl chloride (18.8g 120 mmole) was added and the mixture was stirred for eight hours at 60°C. The mixture was evaporated under reduced pressure and 250 ml ethyl acetate was added .The organic phase was washed four times with water. The organic phase was dried with sodium sulfate and concentrated in vacuo. Yield: 16.8g
¹H-NMR (CDCl₃) 1.40 (m, 3H) 3.81 (s, 3H) 4.26 (m, 1H) 5.14 (s, 2H) 6.90 (d, 2H) 7,28 (d, 2H)

### b) Synthesis of 4-methoxybenzyl-2-(N-CBZ-L-valyloxy)propionate.

To a solution of 4-methoxybenzyl-2-hydroxypropionate (4.2g, 20 mmole), N-CBZ-L-valine (5.02g, 20 mmole) and DMAP (0.24g, 2 mmole) in 100 ml dichloromethane was added a solution of DCC (4.54g, 22 mmole) and the mixture was stirred overnight at room temperature. The mixture was cooled to 5°C and the urethane was filtered. The filtrate was evaporated and the product was isolated by silica gel column chromatography. Yield: 7.9g
¹H-NMR (CDCl₃) 0.88 (m, 6H) 1.50 (m, 3H) 2.26 (m, 1H) 3.81 (s, 3H) 4.34 (m, 1H) 5.04-5.30 (m, 6H) 6.88 (d, 2H) 7.26 (m, 7H)

### c) Synthesis of 2-(N-CBZ-L-valyloxy)-propionic acid.

To a solution of 4-methoxybenzyl-2-(N-CBZ-L-valyloxy)-propionate (7.8g, 17.5 mmole) in dichloromethane (100 ml) was added trifluoroacetic acid (10 ml) and the solution was stirred for one hour at room temperature. The solution was evaporated under reduced pressure and the product was isolated by silica gel column chromatography. Yield: 5.0g
¹H-NMR (CDCl₃) 0.94 (m, 6H) 1.56 (d, 3H) 2.30 (m, 1H) 4.42 (m, 1H) 5.12-5.30 (m, 4H) 7.28 (m, 5H)

### d) Synthesis of 2',3'-dideoxy-3'-fluoro-5-O-[2-(N-CBZ-L-valyloxy)-propionyl]guanosine

A mixture of 2',3'-dideoxy-3'-fluoroguanosine (404 mg, 1.5 mmole), 2-(N-CBZ-L-valyloxy)-propionic acid (0.582g, 1.8 mmole), DMAP (22 mg, 0.18 mmole) and HOBT (243 mg, 1.8 mmole)was co-evaporated two times with DMF and reduced to about 30 ml. DCC (412 mg, 2.0 mmole) was added and the mixture was stirred overnight at room temperature. The mixture was filtered and the solution was evaporated under reduced pressure. 100 ml ethyl acetate was added and the organic phase was washed twice with 5% acetic acid, with 5% sodium hydrogen carbonate and with water. The organic phase was dried with sodium sulfate and evaporated under reduced pressure. The product was isolated by silica gel column chromatography. Yield: 0.72g
¹H-NMR (DMSO d-6) 0.92 (m, 6H) 1.40 (d, 3H) 2.10 (m, 1H) 2.50-3.06 (m, 2H)
4.03 (m, 1H) 4.20-4.44 (m, 3H) 5.04 (s, 2H) *5.12* (m, 1H) 5.44-5.58 (m, 1H) 6.18 (t, 1H) 6.52 (s, 2H) 7.36 (m, 5H) 7.70 (d, 2H) 7.92 (s, 1H)

### e) Synthesis of 2',3'-dideoxy-3'-fluoro-5-O-[2-(L-valyloxy)-propanoyl] guanosine

A solution of 2',3'-dideoxy-3'-fluoro-5-O-[2-(N-CBZ-L-valyloxy)-propanoyl] guanosine (0.6g, 1.04 mmole) in 20 ml ethyl acetate, 10 ml methanol and 10 ml acetic acid was hydrogenated with palladium black (0.1g) for two hours at room temperature and normal pressure. The catalyst was filtered and washed with methanol. The solution was evaporated under reduced pressure to yield the title compound as the acetate salt. Yield: 0.5g
¹H-NMR (DMSO d-6) 0.88 (m, 6H) 1.40 (d, 3H) 1.92 (m, 4H) 2.52-3.04 (m, 2H)
3.18 (m, 1H) 4.18-4.42 (m, 3H) 5.06 (m, 1H) 5.32-5.58 (m, 2H) 6.18 (m, 1H) 6.52 (s, 2H) 7.90 (s, 1H)

### EXAMPLE 33

### 2',3'-Dideoxy-3'-fluoro-5'-O-3-[2,3-bis-(L-valyloxy)-1-propyloxycarbonyl]-propanoyl guanosine.

### a) Synthesis of 4-methoxybenzyl succinate monoester.

To a mixture of succinic anhydride (75g, 750 mmole) and 4-methoxybenzyl alcohol (69.1g, 500 mmole) in 1,4-dioxane (300 ml) was added pyridine (79.1g, 1000 mmole) and the mixture was stirred for five hours at 80°C . The mixture was evaporated under reduced pressure and 600 ml of ethyl acetate and 60 ml of acetic acid were added. The organic phase was washed three times with water, dried with sodium sulfate and evaporated under reduced pressure. The product was recrystallized from toluene. Yield: 104 g.
¹H-NMR (DMSO d-6) 2.48 (m, 4H) 3,72 (s, 3H) 5.00 (s, 2H) 6.90 (d, 2H) 7.28 (d, 2H)

### b) Synthesis of succinic acid 2,3-dihydroxypropyl ester, 4-methoxybenzyl ester .

To a solution of glycerol (23.0g, 250 mmole), 4-methoxybenzyl succinate monoester (5.96 g, 25 mmole) and DMAP (0.36g, 3 mmole) in DMF (200 ml) was added DCC (6.2g 30 mmole) and the mixture was stirred overnight at room temperature. The mixture was evaporated under reduced pressure and 150 ml dichloromethane was added. The mixture was filtered and the solution washed twice with water. The water phase was extracted two times with dichloromethane and the combined organic phases were dried with sodium sulfate. The solution was evaporated under reduced pressure and the product was isolated by silica gel column chromatography.
Yield: 3.0g
¹H-NMR (CDCl₃) 2.65 (m, 4H) 3.61 (m, 2H) 3.80 (s, 3H) 3.90 (m, 1H) 4.18 (m, 2H) 5.05 (s, 2H) 6.89 (d, 2H) 7.26 (d, 2H)

### c) Synthesis of succinic acid 2,3-bis-(N-CBZ-L-Valyloxy)-propyl ester, 4-methoxybenzyl ester.

To a stirred solution of succinic acid 2,3-dihydroxypropyl ester, 4-methoxybenzyl ester (2.9g, 9.28 mmole), N-CBZ-L-valine (5.03g, 20 mmole) and DMAP (0.244g, 2 mmole) in dichloromethane (60 ml) was added DCC (4.5g, 22 mmole) and the mixture was stirred overnight at room temperature. The mixture was filtered and the solution was evaporated under reduced pressure. The product was isolated by silica gel column chromatography. Yield: 2.5g
¹H-NMR (CDCl₃) 0.90 (m, 12H) 2,16 (m, 2H) 2.62 (m, 4H) 3.80 (s, 3H) 4.32 (m, 4H) 5.05-5.52 (m, 9H) 6.89 (d, 2H) 7.30 (m, 12H)

### d) Synthesis of succinic acid 2,3-bis-(N-CBZ-L-valyloxy)propyl ester.

To a solution of the above intermediate (2.3g, 2.95 mmole) in dichloromethane (25 ml) was added trifluoroacetic acid (2.5 ml) and the solution was stirred for two hours at room temperature. The solution was evaporated under reduced pressure and the product was isolated by silica gel column chromatography. Yield: 1,8g
¹H-NMR (CDCl₃) 0.92 (m, 12H) 2.12 (m, 2H) 2.64 (m, 4H) 4.32 (m, 4H) 5.10 (s, 4H) 5.22-5.50 (m, 3H) 7.34 (m, 10H)

### e) Synthesis of 2',3'-dideoxy-3'-fluoro-5'-O-{3-[2,3-bis (N-CBZ-L-valyloxy)-1-propyloxycarbonyl]propanoyl} guanosine.

A mixture of 2', 3'dideoxy-3'-fluoroguanosine (0.538g, 2 mmole), HOBT (0.327g, 2.42 mmole), DMAP(29.3 mg, 0.24 mmole) and succinic acid 2,3-bis-(N-CBZ-L-valyloxy)-1-propyl ester (1.6g, 2.42 mmole) was co-evaporated two times with DMF and reduced to about 50 ml. DCC (0.536 g, 2.6 mmole) was added and the mixture was stirred 72 hours at room temperature. The mixture was filtered and the solution was evaporated under reduced pressure. 100 ml of ethyl acetate was added and the organic phase washed twice with 5% acetic acid, 5% sodium hydrogen carbonate and water. The organic phase was dried with sodium sulfate and evaporated under reduced pressure. The product was isolated by silica gel column chromatography.
Yield: 0.65g.
¹H-NMR (DMSO-d6) 0.88 (m, 12H) 2.08 (m, 2H) 2.58-3.04 (m, 6H) 3.92 (m, 2H) 4.10-4.46 (m, 7H) 5.00 (s, 4H) 5.22 (m, 1H) 5.32-5.56 (m, 1H) 6.17 (m, 1H) 6.50 (s, 2H) 7.32 (m, 10H) 7.70 (d, 2H) 7.92 (s, 1H)

### f) Synthesis of 2',3'-dideoxy-3'-fluoro-5'-O-{3-[2,3-bis-(L-valyloxy)-1-propyloxy-carbonyl]-propanoyl} guanosine.

A solution of the intermediate immediately above (0.57g, 0.626 mmole) in 20ml ethyl acetate, 10 ml methanol and 10 ml acetic acid was hydrogenated with palladium black (0.1g) for two hours at room temperature and normal pressure. The catalyst was filtered and washed with methanol. The solution was evaporated under reduced pressure and the product was isolated by silica gel column chromatography. The product was dissolved in dichloromethane and 1M hydrogen chloride in ether (1.1 ml) was added. The mixture was evaporated under reduced pressure and dried in vacuo to yield the title compound as the dihydrochloride salt. Yield: 0.37g
¹H-NMR (DMSO d-6) 0.92 (m, 12H) 2.12 (m, 2H) 2.58-3.04 (m, 6H) 3.75 (m, 2H)
4.16-4.50 (m, 7H) 5.19-5.60 (m, 2H) 6.18 (m, 1H) 6.76 (s, 2H) 7.92 (s, 1H)

### EXAMPLE 34

### 2',3'-Dideoxy-3'-fluoro-5'-O- 3-[1,3-bis-(L-valyloxy)-2-propyloxycarbonyl] propanoyl guanosine, dihydrochloride salt.

### a) Synthesis of succinic acid 1,3-dibromo-2-propyl ester, 4-methoxybenzyl ester

To a solution of 1,3-dibromopropane-2-ol (21.8g, 100 mmole), succinic acid 4-methoxybenzyl ester (28.6g,120 mmole) and DMAP (1.22g, 10 mmole) in dichloromethane (400 ml) was added DCC (24.8g, 120 mmole) in portions at about 10°C. The mixture was stirred overnight at room temperature and cooled to about 5°C. The mixture was filtered and the solution was evaporated under reduced pressure. 600 ml of ethyl acetate was added and the organic phase was washed twice with 5% acetic acid, 5% sodium hydrogen carbonate and water. The solution was dried with sodium sulfate and evaporated under reduced pressure. The product was isolated by silica gel column chromatography. Yield: 34.8g.
¹H-NMR (CDCl₃) 2.69 (m, 4H) 3.57 (m, 4H) 3.81 (s, 3H) 5.07 (s, 2H) 5.14 (m, 1H)
6,88 (d, 2H) 7.26 (d, 2H)

### b) Synthesis of succinic acid 1,3-bis-(N-CBZ-L-valyloxy)-2-propyl ester, 4-methoxybenzyl ester.

To a solution of N-CBZ-L-valine ( 58.5 g, 232.8 mmole) in dried DMF (300 ml) was added potassium-tert.-butoxide (24,68 g, 220 mmole) and the mixture was stirred for one hour at room temperature. A solution of succinic acid 1,3-dibromo-2-propyl ester, 4-methoxybenzyl ester (34 g, 77.6 mmole) in dried DMF (50 ml) was added and the mixture was stirred for eighteen hours at 60°C. The potassium bromide was filtered and the solution was evaporated under reduced pressure. 600 ml of ethyl acetate was added and the organic phase washed twice with 5% sodium hydrogen carbonate and with water. The organic phase was dried with sodium sulfate and evaporated under reduced pressure. The product was isolated by silica gel column chromatography. Yield: 45g
¹H-NMR (CDCl₃) 0.90 (m, 12H) 2.16 (m, 2H) 2.61 (m, 4H) 3.80 (s, 3H ) 4.12-4.42 (m, 6H) 5.02 (s, 2H) 5.10 (s, 4H) 5.43 (m, 3H) 6.88 (d, 2H) 7.32 (m, 12H)

### c) Synthesis of succinic acid 1,3-bis-(N-CBZ-L-valyloxy)-2-propyl ester.

To a cooled solution of the intermediate immediately above (44.5 g, 57.1 mmole) in dichloromethane (500 ml) was added trifluoroacetic acid (50 ml) between 5°C and 10°C and the solution was stirred for two hours at 10°C. The solution was evaporated under reduced pressure and two times co-evaporated with toluene. 400 ml of ethanol was added and the mixture was stirred for 30 minutes at 40°C. The mixture was cooled and the by-product filtered. The solution was evaporated under reduced pressure and the product was isolated by silica gel column chromatography.
Yield: 33g
¹H-NMR (DMSO-d6) 0.88 (m, 12H) 2.04 (m, 2H) 2.46 (m, 4H) 3.94-4.40 (m, 6H) 5.02 (s, 4H) 5.18 (m, 1H) 7.32 (m, 10H) 7,74 (d, 2H)

### d) Synthesis of 2',3'-dideoxy-3'-fluoro-5'-O-{3-[1,3-bis-(N-CBZ-L-valyloxy)-2-propyloxycarbonyl]propanoyl} guanosine.

A mixture of 2',3'dideoxy-3'-fluoroguanosine (17.8 g, 66 mmole), HOBT (10.64 g, 78.8 mmole), succinic acid 1,3-*bis*-(N-CBZ-L-valyloxy)-2-propyl ester (52 g, 78.8 mmole) and DMAP (0.96 g, 7.88 mmole) was co-evaporated two times with DMF and reduced to about 500 ml. DCC (17.3 g, 84 mmole) was added and the mixture was stirred overnight at room temperature. The mixture was warmed for six hours at 60°C and then cooled to about 10°C . The mixture was filtered and the solution was reduced under reduced pressure. 1200 ml of ethyl acetate was added and the organic phase was washed twice with 5% acetic acid, 5% sodium hydrogen carbonate and water. The organic phase was dried with sodium sulfate and evaporated under reduced pressure. The product was isolated by silica gel column chromatography. Yield: 42g.
¹H-NMR (DMSO-d6) 0.90 (m, 12H) 2.02 (m, 2H) 2.5-3.02 (m, 6H) 3.94 (m, 2H)
4.22 (m, 7H) 5.02 (s, 4H) 5.18 (m, 1H) 5.22-5.50 (m, 1H) 6.16 (m, 1H) 6.50 (s, 2H)
7.32 (m, 10H) 7.72 (d, 2H) 7.92 (s, 1H)

### e) Synthesis of 2',3'-dideoxy-3'-fluoro-5'-O-{3-[1,3-bis-(L-valyloxy)-2-propyloxy-carbonyl]-propanoyl}guanosine dihydrochloride salt.

A solution of 2',3'-dideoxy-3'-fluoro-5'-O-{3-[1,3-*bis*-(N-CBZ-L-valyloxy)-2-propyloxy carbonyl]propanoyl} guanosine (5.0, 5.9 mmole) in 75 ml ethyl acetate and 75 ml methanol was hydrogenated with palladium on activated carbon 10% Pd (1 g) one hour at room temperature and normal pressure. The catalyst was filtered and washed with methanol. The solution was evaporated under reduced pressure. The product was dissolved in dichloromethane and a solution of 1M hydrogen chloride in ether (6 ml) was added, while cooling. The mixture was evaporated under reduced pressure. Yield: 3.5g
¹H-NMR (DMSO d-6) 0.94 (m, 12H) 2.18 (m, 2H) 2.5-3.04 (m, 6H) 4.20-4.54 (m, 7H) 5.24 (m, 1H) 5.34-5.64 (m, 1H) 6.22 (m, 1H) 6.92 (s, 2H) 8.30 (s, 1H) 8.62 (s, 6H)

### EXAMPLE 35

### Alternative synthesis of 2',3'-dideoxy-3'-fluoro-5'-O- 3-[1,3-bis-(L-valyloxy)-2-propyloxycarbonyl] propanoyl guanosine

### a) Synthesis of succinic acid 1,3-dibromo-2-propyl ester, 1,1-dimethylethyl ester.

To a solution of 1,3-dibromopropane-2-ol (10.9 g 50 mmole), succinic acid 1,1-dimethylethyl ester (J. Org.Chem 59 (1994) 4864) (10.45g, 60 mmole) and DMAP (0.61 g, 5 mmole) in dichloromethane (180 ml) was added DCC (12.4 g, 60 mmole) in portions at about 10°C. The mixture was stirred overnight at room temperature and cooled to about 5°C. The mixture was filtered and the solution was evaporated under reduced pressure. 250 ml ethyl acetate was added and the organic phase was washed twice with 5% citric acid, 5% sodium hydrogen carbonate and water. The solution was dried with sodium sulfate and evaporated under reduced pressure. The product was distilled in vacuo. (bp 0,5 135-140°C) Yield: 16.8 g
¹H-NMR (CDCl₃) 1.45 (s, 9H) 2.58 (m, 4H) 3.61 (m, 4H) 5.12 (m, 1H)

### b) Synthesis of succinic acid 1,3-bis-(N-CBZ-L-valyloxy)-2-propyl ester, 1,1-dimethylethyl ester.

To a solution of N-CBZ-L-valine (18.85 g, 75 mmole) in dried DMF (100 ml) was added potassium tert.-butoxide (7.85 g, 70 mmole) and the mixture was stirred for one hour at room temperature. A solution of succinic acid 1,3-dibromo-2-propyl ester, 1,1-dimethylethyl ester (9.35g, 25 mmole) in dried DMF (20 ml) was added and the mixture was stirred for eighteen hours at 60°C. The potassium bromide was filtered and the solution evaporated under reduced pressure. 300 ml of ethyl acetate were added and the organic phase washed twice with 5% sodium hydrogen carbonate and with water. The organic phase was dried with sodium sulfate and evaporated under reduced pressure. The product was isolated by silica gel column chromatography. Yield: 14g
¹H-NMR (CDCl₃) 0.90 (m, 12H) 1.42 (s, 9H) 2.14 (m, 2H) 2.52 (m, 4H) 4.32 (m, 6H) 5.10 (s, 4H) 5.32 (m, 3H) 7.26 (m, 10H)

### c) Synthesis of 1,3-bis-(N-CBZ-L-valyloxy )-2-propyl succinate monoester.

To a cooled solution of succinic acid 1,3-bis-(N-CBZ-L-valyloxy)-2-propyl ester, 1,1-dimethylethyl ester (13 g, 18.18 mmole) in dichloromethane (100 ml) was added trifluoroacetic acid (20 ml) and the solution was stirred for six hours at room temperature. The solution was evaporated under reduced pressure. 200 ml ethyl acetate was added and the organic phase was washed with 5% sodium hydrogen carbonate and water. The solution was evaporated under reduced pressure.
Yield: 11.7g
¹H-NMR (DMSO-d6) 0.88 (m, 12H) 2.04 (m, 2H) 2.46 (m, 4H) 3.94-4.40 (m, 6H) 5.02 (s, 4H) 5.18 (m, 1H) 7.32 (m, 10H) 7.74 (d, 2H)

### d) Synthesis of 2',3'-dideoxy-3'-fluoro-5'-O- 3-[1,3-bis-(L-valyloxy)-2-propyloxycarbonyl] propanoyl guanosine

The intermediate from step c) is esterified to FLG as shown in example 34 step d) and the N-protecting groups on the valyl moieties removed by conventional techniques, such as shown in Example 35 step e) or Example 29 step e).

### BIOLOGICAL EXAMPLE I

### Pharmacokinetics

Confirmation that orally administered prodrugs as described herein release FLG in vivo is obtained in a rat model which is recognized as a useful model for assessing pharmacokinetic parameters of nucleoside analogues. The oral compositions are administered in a pharmaceutical vehicle comprising propylene glycol, or in the case of the more soluble compounds such as that of Example 26 or Example 34, in water, to duplicate fasted animals in a dosage corresponding to 0.1 mmol/kg. For comparison, a set of rats is iv dosed with 0.01 mmol/kg of the metabolite 2',3'-dideoxy-3'-fluoroguanosine. Serum levels of the metabolite are then monitored in serum collected at intervals from individual animals from 0.5 to up to 12 hours following administration (5 min to 6 hours for FLG).

The metabolite is analysed with HPLC with UV detection at 254 nm, in a manner analogous to Ståhle et al 1995, J Pharm. Biomed. Anal. 13, 369-376. An HPLC system can be based on a 0.05 M ammonium-dihydrogen-phosphate buffer, with 1.2 % 2-propanol solvent, buffered to pH 4.5 or 30 mM sodium dihydrogen phosphate buffer with 2% acetonitrile solvent buffered to pH 7.0. The column may be a 100 x 2.1 mm BAS C18 5 µm particle size with a 7 µm C18 guard column or Zorbax SB-CN C18 150x4.6mm, 5µm column. Protein binding of the compounds described herein is neglible as is that of the metabolite and ultrafiltration through Amicon or Microcon 30 filters is useful for serum samples. Advantageously the main peak is subject to further column chromatography to better aid in resolution of FLG over low weight serum components. The iv levels are multiplied by a factor of ten in order to obtain AUC values for comparison with the oral values. Absolute oral bioavailability is determined as the ratio between ^{0-∞}AUCᵢᵥ and ^{0-∞}AUCₒᵣₐₗ.

**Table 1**

| | 6h absolute bioavail. % | 12h absolute bioavail. % |
|---|---|---|
| FLG | | 9%^{**} |
| Example 22 | 39% | >80%^{*} |
| Example 13 | 37% | |
| Example 12 | 29% | |
| Example 25 | 81.5 % | |
| Example 28 | 47.5% | |
| Example 24 | 60.5% | |
| Example 26 | | 67.5% |
| Example 29 | 51% | |

| | | |
|---|---|---|
| ^{*} estimated. | | |
| ^{**}literature value | | |

The compounds described herein including the compounds of the invention thus provide significantly enhanced oral bioavailability relative to the metabolite metabolite 2',3'-dideoxy-3'-fluoroguanosine. Notably, the compounds are released into the blood in a relatively sustained manner, rather than in an immediate peak. This means that effective amounts of the active metabolite are available in the blood for many hours assisting once daily dosage. Additionally, a sustained release avoids the problems of acute toxicity seen in compounds with a more rapid release rate.

Although the rat is well recognized as a good model for predicting human bioavailability of nucleoside analogues, species independent bioavailability of a compound (Example 34) was confirmed in ≅11.5 kg male and female beagle dogs administered orally with 0.05 mmol/kg (38 mg/kg) compound in water or iv 0.005 mmol/kg (1.35 mg/kg) metabolite in water. Plasma collection and analysis as above.

| | | |
|---|---|---|
| Male dog | 12 hour absolute bioavailability | 51% |
| Female dog | 12 hour absolute bioavailability | 74% |

### BIOLOGICAL EXAMPLE 2

### Antiviral activity - Retroviruses

As can be demonstrated by the methodology of Biological Example 1, the compounds tested release, *in vivo*, the metabolite 2',3'-dideoxy-3'-fluoroguanosine. *In vitro* measurement of the antiviral activity of this metabolite will thus reflect the *de facto* activity of these compounds.

In the XTT dye uptake assay of Koshida et al Antimicrob Agents Chemother. 33 778-780, 1989) utilising MT4 cells, the metabolite measured in Biological Example 1 above showed the following *in vitro* activities against retroviruses:

**Table 2**

| HIV or retroviral strain | IC₅₀^{*} |
|---|---|
| HIV-1_{111B} | 1 µg/ml |
| HIV-1²⁴⁴¹ AZT^{r} | 1 µg/ml |
| HIV-1_{111B} TIBO^{r} | 1 µg/ml |
| HIV-1^{29/9} | 0.7 µg/ml |
| HIV-2_{SBL6669} | 2 µg/ml |
| SIV_{SM} | 1 µg/ml |

| | |
|---|---|
| ^{*}Concentration of metabolite inducing 50% inhibition of viral replication | |

It will thus be apparent that administration of the compounds of the invention as well as the compounds mentioned in this application induce powerful antiviral activities against the retroviruses HIV-1, HIV-2 and SIV. It should also be noted from the HIV-1²⁴⁴¹ AZT^{t} and HIV-1_{111B} TIBO^{r} results that the antiviral activity of these compounds does not show cross resistance against strains of HIV which have become resistant to other HIV agents such as the nucleoside analogue AZT or the non-nucleoside reverse transcriptase inhibitor TIBO.

### BIOLOGICAL EXAMPLE 3

### Antiviral activity - HBV

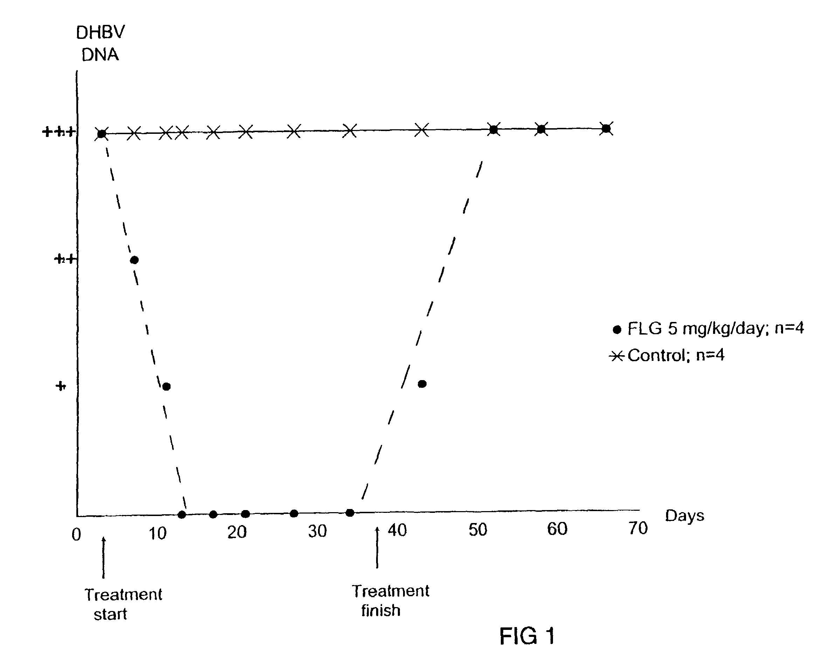

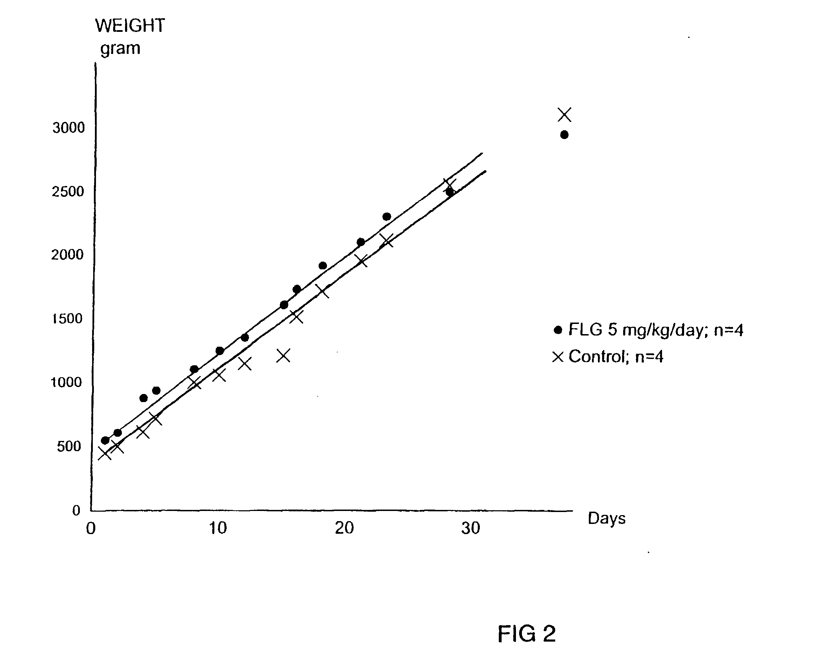

The activity of antivirals on duck hepatitis B virus (DHBV) in ducks is an acknowledged animal model for the validation of *in vivo* hepatitis B activity in humans. The activity of the *in vivo* metabolite measured in Biological Example 2 above has been assayed in the DHBV model described by Sherker et al (1986) Gastroenterology 91, pp 818-824. The results are depicted in Figures 1 and 2 In short, 4 control ducks were treated with phosphate buffered saline (PBS) and 4 ducks with 5 mg/kg/day of the active metabolite. The ducks were two days old when inoculated with DHBV and 18 days old when treatment was commenced. The metabolite and PBS (controls) were given intraperitoneally for 10 days as twice daily injections, at 8 am and 4 pm. Treatment lasted 33 days and the animals were followed 5 weeks after the end of treatment.

The efficacy of treatment was followed by dot blot-hybridisation of DHBV DNA in serum using a radioactive probe and the amount of DHBV measured as the amount of radioactivity hybridised. Figure 1 plots the amount of DHBV DNA in serum at different timepoints before, during and after treatment.

As can be seen in Figure 1, there is no decrease in the amount of DHBV in serum during treatment with PBS (control, solid line). The animals given the metabolite measured in Biological Example 2 (broken line) showed a dramatic decrease in the amount of DHBV in serum during the first 10 days of treatment, whereupon for the remainder of treatment the level of DHBV DNA was below the detection limit at this dose of 5 mg/kg/day. Repeat experiments at dosages of 30 and 3 mg/kg/day and with congenitally infected ducks (not shown) also produced similar results, that is a dramatic fall in serum DHBV DNA to under the detection threshold. Even at the very low dose of 0.3 mg/kg/day the metabolite caused a considerable inhibition of DHBV *in vivo*. After the finish of treatment, virus reappeared in the serum, as shown in Fig. 1. Reappearance of HBV after short term treatment with conventional antivirals has been observed earlier in both humans and animals with chronic hepatitis B infection.

As can be seen in Fig 2, the weight of the ducks increased in the same way as in the control (PBS treated) animals. The weight increase from about 270 g to about 800 g which was observed during the treatment period is so large that toxic effects, had they occurred, should be easily visible as a change in growth rate. Similar growth curves were also observed for the ducks receiving the higher dosage rate of 30 mg/kg/day. This metabolite is thus clearly non-toxic. As the compounds described and the compounds of the invention are hydrolysed *in vivo* to give this metabolite, as established in Example 2 above, and a nature identical and therefore easily metabolized fatty acid, it can therefore be inferred that no long term toxicity problem can be expected from administration of these compounds. The absence of acute (short term) toxicity of these compounds when administered orally is established in Biological Example 2 above.

### FORMULATION EXAMPLE 1

### Tablet formulation

The following ingredients are screened through a 0.15 mm seive and dry-mixed

| | |
|---|---|
| 10 g | 2', 3'-dideoxy-3'-fluoro-5'-O-3-[1,3-bis-(L-valyloxy)-2-propyloxycarbonyl propanoyl]guanosine |
| 40 g | lactose |
| 49 g | crystalline cellulose |
| 1 g | magnesium stearate |

A tabletting machine is used to compress the mixture to tablets containing 250 mg of active ingredient.

### FORMULATION EXAMPLE 2

### Enteric coated tablet

The tablets of Formulation Example 1 are spray coated in a tablet coater with a solution comprising

| | |
|---|---|
| 120 g | ethyl cellulose |
| 30 g | propylene glycol |
| 10g | sorbitan monooleate |
| | ad 1 000 ml aq. dist. |

### FORMULATION EXAMPLE 3

| Controlled release formulation | |
|---|---|
| 50 g | 2',3'-dideoxy-3'-fluoro-5'-O-[5-(L-valyloxymethyl)-6-stearoyloxy hexanoyl] guanosine |
| 12 g | hydroxypropylmethylcellulose (Methocell K15) |
| 4.5 g | lactose |

are dry-mixed and granulated with an aqueous paste of povidone. Magnesium stearate (0.5 g) is added and the mixture compressed in a tabletting machine to 13 mm diameter tablets containing 500 mg active agent.

### FORMULATION EXAMPLE 4

| Soft capsules | |
|---|---|
| 250 g | 2',3'-dideoxy-3'-fluoro-5'-O-[5-(L-valyloxymethyl)-6 -stearoyloxyhexanoyl] guanosine |
| 100 g | lecithin |
| 100 g | arachis oil |

The nucleoside compound is dispersed in the lecithin and arachis oil and filled into soft gelatin capsules.

## Claims

1. A compound of formula Ig 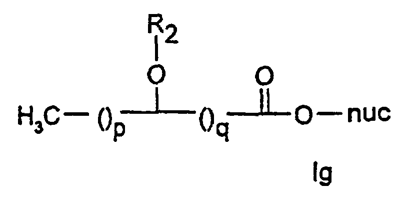 where O-nuc is the residue of a monohydroxyl bearing D- or L- nucleoside analogue; R₂ is the residue of an aliphatic L-amino acid,
p is 0, 1 or 2-20 (optionally including a double bond) and q is 0-5.

2. A compound according to claim 1 of formula IId' 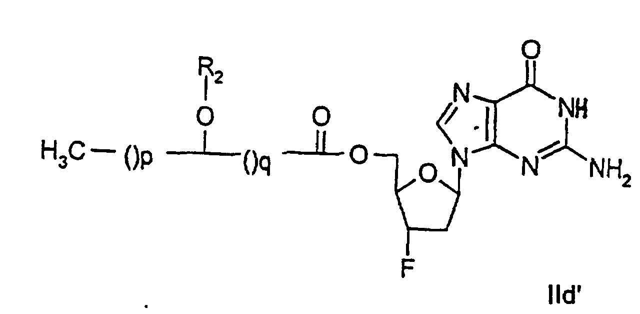 where R₂, p, and q are as defined in claim 1.

3. A compound according to claim 1 or claim 2, wherein q is 0.

4. A compound according to claim 1, 2 or 3, wherein R₂ defines an isoleucine or preferably a valine derivative.

5. A compound according to claim 4, selected from:
2',3'-dideoxy-3'-fluoro-5'-O-[2-(L-valyloxy)-butyryl] guanosine,
2',3'-dideoxy-3'-fluoro-5'-O-[2-(L-valyloxy)-hexanoyl] guanosine,
2',3'-dideoxy-3'-fluoro-5'-O-[2-(L-valyloxy)-octanoyl] guanosine,
2',3'-dideoxy-3'-fluoro-5'-O-[2-(L-valyloxy)-decanoyl] guanosine,
2',3'-dideoxy-3'-fluoro-5'-O-[2-(L-valyloxy)-dodecanoyl] guanosine,
2',3'-dideoxy-3'-fluoro-5'-O-[2-(L-valyloxy)-myristoyl] guanosine,
2',3'-dideoxy-3'-fluoro-5'-O-[2-(L-valyloxy)-palmitoyl] guanosine,
2',3'-dideoxy-3'-fluoro-5'-O-[2-(L-valyloxy)-stearoyl) guanosine,
2',3'-dideoxy-3'-fluoro-5'-O-[2-(L-valyloxy)-docosanoyl] guanosine,
2',3'-dideoxy-3'-fluoro-5'-O-[2-(L-valyloxy)-eicosanoyl] guanosine
2',3'-dideoxy-3'-fluoro-5'-O-[2-(L-isoleucyloxy)-butyryl] guanosine,
2',3'-dideoxy-3'-fluoro-5'-O-[2-(L-isoleucyloxy)-hexanoyl] guanosine,
2',3'-dideoxy-3'-fluoro-5'-O-[2-(L-isoleucyloxy)-octanoyl] guanosine,
2',3'-dideoxy-3'-fluoro-5'-O-[2-(L-isoleucyloxy)-decanoyl] guanosine,
2',3'-dideoxy-3'-fluoro-5'-O-[2-(L-isoleucyloxy)-dodecanoyl] guanosine,
2',3'-dideoxy-3'-fluoro-5'-O-[2-(L-isoleucyloxy)-myristoyl] guanosine,
2',3'-dideoxy-3'-fluoro-5'-O-[2-(L-isoleucyloxy)-palmitoyl] guanosine,
2',3'-dideoxy-3'-fluoro-5'-O-[2-(L-isoleucyloxy)-stearoyl] guanosine;
2',3'-dideoxy-3'-fluoro-5'-O-[2-(L-isoleucyloxy)-docosanoyl] guanosine,
2',3'-dideoxy-3'-fluoro-5'-O-[2-(L-isoleucyloxy)-eicosanoyl] guanosine.

6. A compound according to claims 1,2 or 3 wherein p and q are 0.

7. A compound according to claim 6 denoted:
2',3'-dideoxy-3'-fluoro-5'-O-[2-(L-valyloxy)-propionyl] guanosine; or
2',3'-dideoxy-3'-fluoro-5'-O-[2-(L-isoleucyloxy)-propionyl] guanosine, wherein the propionyl moiety defines an L-lactic acid derivative, and pharmaceutically acceptable salts thereof.

8. The compound according to claim 6 denoted 2',3'-dideoxy-3'-fluoro-5'-O-[2-(L-valyloxy)-propionyl] guanosine, wherein the propionyl moiety defines an L-lactic acid derivative, and pharmaceutically acceptable salts thereof.

9. A compound according to claim 1, 3, 4 or 6, wherein O-Nuc is the residue of an acyclic nucleoside analogue such as acyclovir, or a cyclic nucleoside analogue such as ddl (didanosine), ddC (zalcitabine), d4T (stavudine), FTC, lamivudine (3TC), 1592U89 (4-[2-amino-6-(cyclopropylamino)-9H-purin-9-yl]-2-cyclopentene-1-methanol), AZT (zidovudine), DAPD (D-2,6-diaminopurine dioxolane), F-ddA, or a monohydric L-nucleoside.

10. A compound according to claim 9, selected from the group;
4'-O-[2-(L-valyloxy)-propionyl] acyclovir,
4'-O-[2-(L-isoleucyloxy)-propionyl] acyclovir,
5'-O-[2-(L-valyloxy)-propionyl] ddI,
5'-O-[2-(L-isoleucyloxy)-propionyl] ddI,
5'-O-[2-(L-valyloxy)-propionyl] stavudine,
5'-O-[2-(L-isoleucyloxy)-propionyl] stavudine,
5'-O-[2-(L-valyloxy)-propionyl] lamivudine,
5'-O-[2-(L-isoleucyloxy)-propionyl] lamivudine,
5'-O-[2-(L-valyloxy)-propionyl] DAPD,
5'-O-[2-(L-isoleucyloxy)-propionyl] DAPD;
and the corresponding derivatives of 4-[2-amino-6(cyclopropylamino)-9*H*-purin-9-yl]-2-cyclopentene-1-methanol;
and pharmaceutically acceptable salts thereof.

11. A compound according to any of the preceding claims for use in therapy in human and veterenary medicine.

12. Use of a compound according to any of the preceding claims in the manufacture of a medicament for the prophylaxis or treatment of viral infections.

13. Use according to claim 12, wherein the viral infection is caused by HBV or a retrovirus.

## Patentansprüche

1. Verbindung der Formel Ig 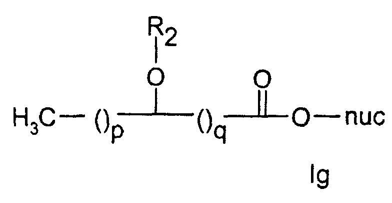 in der O-nuc den Rest eines eine Monohydroxylgruppe aufweisenden D- oder L-Nucleosidanalogen bedeutet;
R₂ den Rest einer aliphatischen L-Aminosäure bedeutet;
p einen Wert von 0, 1 oder von 2-20 (gegebenenfalls unter Einschluss einer Doppelbindung) hat und q einen Wert von 0-5 hat.

2. Verbindung nach Anspruch 1 der Formel IId' 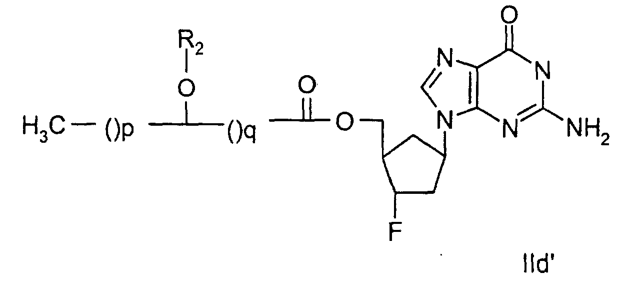 in der R₂, p und q die in Anspruch 1 definierten Bedeutungen haben.

3. Verbindung nach Anspruch 1 oder 2, wobei q den Wert 0 hat.

4. Verbindung nach Anspruch 1, 2 oder 3, wobei R2 ein Isoleucin- oder vorzugsweise ein Valinderivat bedeutet.

5. Verbindung nach Anspruch 4, ausgewählt aus:
2',3'-Didesoxy-3'-fluor-5'-O-[2-(L-valyloxy)-butyryl]-guanosin,
2',3'-Didesoxy-3'-fluor-5'-O-[2-(L-valyloxy)-hexanoyl]-guanosin,
2',3'-Didesoxy-3'-fluor-5'-O-[2-(L-valyloxy)-octanoyl]-guanosin,
2',3'-Didesoxy-3'-fluor-5'-O-[2-(L-valyloxy)-decanoyl]-guanosin,
2',3'-Didesoxy-3'-fluor-5'-O-[2-(L-valyloxy)-dodecanoyl]-guanosin,
2',3'-Didesoxy-3'-fluor-5'-O-[2-(L-valyloxy)-myristoyl]-guanosin,
2',3'-Didesoxy-3'-fluor-5'-O-[2-(L-valyloxy)-palmitoyl]-guanosin,
2',3'-Didesoxy-3'-fluor-5'-O-[2-(L-valyloxy)-stearoyl]-guanosin,
2',3'-Didesoxy-3'-fluor-5'-O-[2-(L-valyloxy)-docosanoyl]-guanosin,
2',3'-Didesoxy-3'-fluor-5'-O-[2-(L-valyloxy)-eicosanoyl]-guanosin,
2',3'-Didesoxy-3'-fluor-5'-O-[2-(L-isoleucyloxy)-butyryl]-guanosin,
2',3'-Didesoxy-3'-fluor-5'-O-[2-(L-isoleucyloxy)-hexanoyl]-guanosin,
2',3'-Didesoxy-3'-fluor-5'-O-[2-(L-isoleucyloxy)-octanoyl]-guanosin,
2',3'-Didesoxy-3'-fluor-5'-O-[2-(L-isoleucyloxy)-decanoyl]-guanosin,
2',3'-Didesoxy-3'-fluor-5'-O-[2-(L-isoleucyloxy)-dodecanoyl]-guanosin,
2',3'-Didesoxy-3'-fluor-5'-O-[2-(L-isoleucyloxy)-myristoyl]-guanosin,
2',3'-Didesoxy-3'-fluor-5'-O-[2-(L-isoleucyloxy)-palmitoyl]-guanosin,
2',3'-Didesoxy-3'-fluor-5'-O-[2-(L-isoleucyloxy)-stearoyl]-guanosin,
2',3'-Didesoxy-3'-fluor-5'-O-[2-(L-isoleucyloxy)-docosanoyl]-guanosin und
2',3'-Didesoxy-3'-fluor-5'-O-[2-(L-isoleucyloxy)-eicosanoyl]-guanosin.

6. Verbindung nach Anspruch 1, 2 oder 3, wobei p und q den Wert 0 haben.

7. Verbindungen nach Anspruch 6, nämlich:
2',3'-Didesoxy-3'-fluor-5'-O-[2-(L-valyloxy)-propionyl]-guanosin; oder
2',3'-Didesoxy-3'-fluor-5'-O-[2-(L-isoleucyloxy)-propionyl]-guanosin, wobei der Propionylrest ein L-Milchsäurederivat definiert, sowie pharmazeutisch verträgliche Salze davon.

8. Verbindung nach Anspruch 6, nämlich 2',3'-Didesoxy-3'-fluor-5'-O-[2-(L-valyloxy)-propionyl]-guanosin, wobei der Propionylrest ein L-Milchsäurederivat definiert, sowie pharmazeutisch verträgliche Salze davon.

9. Verbindung nach Anspruch 1, 3, 4 oder 6, wobei O-Nuc den Rest eines acyclischen Nucleosidanalogen, wie Acyclovir, oder eines cyclischen Nucleosidanalogen, wie ddI (Didanosin), ddC (Zalcitabin), d4T (Stavudin), FTC, Lamivudin (3TC), 1592U89 (4-[2-Amino-6-(cyclopropylamino)-9H-purin-9-yl]-2-cyclopenten-1-methanol), AZT (Zidovudin), DAPD (D-2,6-Diaminopurindioxolan) und F-ddA, oder eines einwertiges L-Nucleosids bedeutet.

10. Verbindung nach Anspruch 9, ausgewählt aus der Gruppe:
4'-O-[2-(L-Valyloxy)-propionyl]-acyclovir,
4'-O-[2-(L-Isoleucyloxy)-propionyl]-acyclovir,
5'-O-[2-(L-Valyloxy)-propionyl]-ddI,
5'-O-[2-(L-Isoleucyloxy)-propionyl]-ddI,
5'-O-[2-(L-Valyloxy)-propionyl]-stavudin,
5'-O-[2-(L-Isoleucyloxy)-propionyl]-stavudin,
5'-O-[2-(L-Valyloxy)-propionyl]-lamivudin,
5'-O-[2-(L-Isoleucyloxy)-propionyl]-lamivudin,
5'-O-[2-(L-Valyloxy)-propionyl]-DAPD,
5'-O-[2-(L-Isoleucyloxy)-propionyl]-DAPD;
und die entsprechenden Derivate von 4-[2-Amino-6-(cyclopropylamino)-9H-purin-9-yl]-2-cyclopenten-1-methanol; sowie die pharmazeutisch verträglichen Salze davon.

11. Verbindung nach einem der vorstehenden Ansprüche zur Verwendung bei der Therapie beim Menschen und in der Veterinärmedizin.

12. Verwendung einer Verbindung nach einem der vorstehenden Ansprüche bei der Herstellung eines Arzneimittels zur Prophylaxe oder Therapie von viralen Infektionen.

13. Verwendung nach Anspruch 12, wobei die virale Infektion durch HBV oder ein Retrovirus verursacht worden ist.

## Revendications

1. Composé de formule Ig 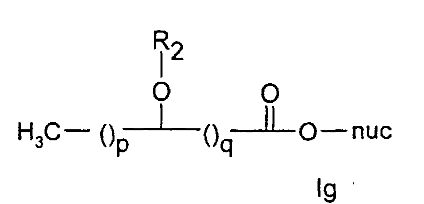 dans laquelle O-nuc est un reste d'analogue D- ou L- de nucléoside portant un groupe monohydroxyle; R₂ est un reste d'acide aminé aliphatique L,
p est 0, 1 ou 2-20 (incluant éventuellement une double liaison) et q est 0-5.

2. Composé selon la revendication 1, de formule IId' 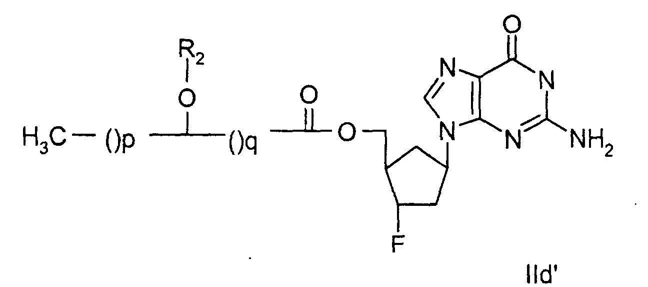 dans laquelle R₂, p, et q sont tels que définis dans la revendication 1.

3. Composé selon la revendication 1 ou 2, dans lequel q est 0.

4. Composé selon la revendication 1, 2 ou 3, dans lequel R₂ désigne un dérivé d'isoleucine ou, préférentiellement, un dérivé de valine.

5. Composé selon la revendication 4, choisi parmi:
2',3'-didéoxy-3'-fluoro-5'-O-2-(L-valyloxy)-butyryl guanosine,
2',3'-didéoxy-3'-fluoro-5'-O-2-(L-valyloxy)-hexanoyl guanosine,
2',3'-didéoxy-3'-fluoro-5'-O-2-(L-valyloxy)-octanoyl guanosine,
2',3'-didéoxy-3'-fluoro-5'-O-2-(L-valyloxy)-décanoyl guanosine,
2',3'-didéoxy-3'-fluoro-5'-O-2-(L-valyloxy)-dodécanoyl guanosine,
2',3'-didéoxy-3'-fluoro-5'-O-2-(L-valyloxy)-myristoyl guanosine,
2',3'-didéoxy-3'-fluoro-5'-O-2-(L-valyloxy)-palmitoyl guanosine,
2',3'-didéoxy-3'-fluoro-5'-O-2-(L-valyloxy)-stéaroyl guanosine,
2',3'-didéoxy-3'-fluoro-5'-O-2-(L-valyloxy)-docosanoyl guanosine,
2',3'-didéoxy-3'-fluoro-5'-O-2-(L-valyloxy)-eicosanoyl guanosine
2',3'-didéoxy-3'-fluoro-5'-O-2-(L-isoleucyloxy)-butyryl guanosine,
2',3'-didéoxy-3'-fluoro-5'-O-2-(L-isoleucyloxy)-hexanoyl guanosine,
2',3'-didéoxy-3'-fluoro-5'-O-2-(L-isoleucyloxy)-octanoyl guanosine,
2',3'-didéoxy-3'-fluoro-5'-O-2-(L-isoleucyloxy)-décanoyl guanosine,
2',3'-didéoxy-3'-fluoro-5'-O-2-(L-isoleucyloxy)-dodécanoyl guanosine,
2',3'-didéoxy-3'-fluoro-5'-O-2-(L-isoleucyloxy)-myristoyl guanosine,
2',3'-didéoxy-3'-fluoro-5'-O-2-(L-isoleucyloxy)-palmitoyl guanosine,
2',3'-didéoxy-3'-fluoro-5'-O-2-(L-isoleucyloxy)-stéaroyl guanosine,
2',3'-didéoxy-3'-fluoro-5'-O-2-(L-isoleucyloxy)-docosanoyl guanosine,
2',3'-didéoxy-3'-fluoro-5'-O-2-(L-isoleucyloxy)-eicosanoyl guanosine.

6. Composé selon la revendication 1, 2 ou 3, dans lequel p et q sont 0.

7. Composé selon la revendication 6, désigné:
2',3'-didéoxy-3'-fluoro-5'-O-2-(L-valyloxy)-propionyl guanosine; ou
2',3'-didéoxy-3'-fluoro-5'-O-2-(L-isoleucyloxy)-propionyl guanosine, dans lequel la partie propionyle définit un dérivé d'acide lactique L, et ses sels pharmaceutiquement acceptables.

8. Composé selon la revendication 6, désigné 2',3'-didéoxy-3'-fluoro-5'-O-2-(L-valyloxy)-propionyl guanosine, dans lequel la partie propionyle définit un dérivé d'acide lactique L, et ses sels pharmaceutiquement acceptables.

9. Composé selon la revendication 1, 3, 4 ou 6, dans lequel O-Nuc est un reste d'analogue de nucléoside acyclique tel que l'acyclovir, ou d'analogue de nucléoside cyclique tel que ddI (didanosine), ddC (zalcitabine), d4T (stavudine), FTC, lamivudine (3TC), 1592U89 (4-2-amino-6-(cyclopropylamino)-9H-purin-9-yl-2-cyclopentène-1-méthanol), AZT (zidovudine), DAPD (D-2,6-diaminopurine dioxolane), F-ddA, ou de nucléoside L monohydrique.

10. Composé selon la revendication 9, choisi parmi le groupe;
4'-O-2-(L-valyloxy)-propionyl acyclovir,
4'-O-2-(L-isoleucyloxy)-propionyl acyclovir,
5'-O-2-(L-valyloxy)-propionyl ddI,
5'-O-2-(L-isoleucyloxy)-propionyl ddI,
5'-O-2-(L-valyloxy)-propionyl stavudine,
5'-O-2-(L-isoleucyioxy)-propionyl stavudine,
5'-O-2-(L-valyloxy)-propionyl lamivudine,
5'-O-2-(L-isoleucyloxy)-propionyl lamivudine,
5'-O-2-(L-valyloxy)-propionyl DAPD,
5'-O-2-(L-isoleucyloxy)-propionyl DAPD;
et les dérivés correspondants du 4-2-amino-6(cyclopropylamino)-9*H*-purin-9-yl-2-cyclopentène-1-méthanol; et leurs sels pharmaceutiquement acceptables.

11. Composé selon l'une quelconque des revendications précédentes, pour une utilisation thérapeutique en médecine humaine et vétérinaire.

12. Utilisation d'un composé selon l'une quelconque des revendications précédentes pour la fabrication d'un médicament destiné à la prévention ou au traitement d'infections virales.

13. Utilisation selon la revendication 12, caractérisée en ce que l'infection virale est causée par un HBV ou un rétrovirus.
